# EUROPEAN PATENT APPLICATION

(11) **EP 1 870 464 A2**
(43) Date of publication of application: **26.12.2007**
(21) Application number: 07005022.4
(22) Date of filing: 30.05.2000
(51) Int. Cl.: C12N 15/12, C07K 14/47, A61K 38/17, G01N 33/53, C12Q 1/68, C12N 15/62, C07K 16/18

(54) **Methods and compositions for inhibiting neoplastic cell growth**

(30) Priority: 22.06.1999 US 140650 P; 23.06.1999 US 141037 P; 20.07.1999 US 144758 P; 01.09.1999 WO PCT/US99/20111; 08.09.1999 WO PCT/US99/20594; 29.10.1999 US 162506 P; 30.11.1999 WO PCT/US99/28313; 01.12.1999 WO PCT/US99/28634; 02.12.1999 WO PCT/US99/28551; 09.12.1999 US 170262 P; 16.12.1999 WO PCT/US99/30095; 20.12.1999 WO PCT/US99/30999; 06.01.2000 WO PCT/US00/00376; 11.02.2000 WO PCT/US00/03565; 18.02.2000 WO PCT/US00/04341; 18.02.2000 WO PCT/US00/04342; 02.03.2000 WO PCT/US00/05841; 03.03.2000 US 187202 P; 10.03.2000 WO PCT/US00/06319; 15.03.2000 WO PCT/US00/06884; 30.03.2000 WO PCT/US00/08439; 17.05.2000 WO PCT/US00/13705
(62) Divisional of application: 00941164.6
(71) Applicant: Genentech, Inc., South San Francisco CA 94080-4990 (US)
(72) Inventor: Baker, Kevin P., Darnestown, MD 20878 (US); Goddard, Audrey, San Francisco, CA 94127 (US); Gurney, Austin L., San Francisco, CA 94114 (US); Herbert, Caroline, Berkeley, CA 94703 (US); Henzel, William, San Mateo, CA 94030 (US); Kabakoff, Rhona C., Pacifica, CA 94044 (US); Shelton, David L., Oakland, CA 94618 (US); Smith, Victoria, Burlingame, CA 94010 (US); Watanabe, Colin K., San Francisco, CA 94110 (US); Wood, William I., Hillsborough, CA 94010 (US)
(74) Representative: Woolley, Lindsey Claire

(57) **Abstract**

The present invention concerns methods and compositions for inhibiting neoplastic cell growth. In particular, the present invention concerns antitumor compositions and methods for the treatment of tumors. The invention further concerns screening methods for identifying growth inhibitory, e.g., antitumor compounds. The present invention is directed to novel polypeptides and to nucleic acid molecules encoding those polypeptides. Also provided herein are vectors and host cells comprising those nucleic acid sequences, chimeric polypeptide molecules comprising the polypeptides of the present invention fused to heterologous polypeptide sequences, antibodies which bind to the polypeptides of the present invention and to methods for producing the polypeptides of the present invention.

## Description

### FIELD OF THE INVENTION

The present invention concerns methods and compositions for inhibiting neoplastic cell growth. In particular, the present invention concerns antitumor compositions and methods for the treatment of tumors. The invention further concerns screening methods for identifying growth inhibitory, *e.g.,* antitumor compounds.

### BACKGROUND OF THE INVENTION

Malignant tumors (cancers) are the second leading cause of death in the United States, after heart disease (Boring et al., CA Cancel J. Clin., 43:7 (1993)).

Cancer is characterized by the increase in the number of abnormal, or neoplastic, cells derived from a normal tissue which proliferate to form a tumor mass, the invasion of adjacent tissues by these neoplastic tumor cells, and the generation of malignant cells which eventually spread via the blood or lymphatic system to regional lymph nodes and to distant sites (metastasis). In a cancerous state a cell proliferates under conditions in which normal cells would not grow. Cancer manifests itself in a wide variety of forms, characterized by different degrees of invasiveness and aggressiveness.

Despite recent advances in cancer therapy, there is a great need for new therapeutic agents capable of inhibiting neoplastic cell growth. Accordingly, it is the objective of the present invention to identify compounds capable of inhibiting the growth of neoplastic cells, such as cancer cells.

### SUMMARY OF THE INVENTION

### A. Embodiments

The present invention relates to methods and compositions for inhibiting neoplastic cell growth. More particularly, the invention concerns methods and compositions for the treatment of tumors, including cancers, such as breast, prostate, colon, lung, ovarian, renal and CNS cancers, leukemia, melanoma, etc., in mammalian patients, preferably humans.

In one aspect, the present invention concerns compositions of matter useful for the inhibition of neoplastic cell growth comprising an effective amount of a PRO polypeptide as herein defined, or an agonist thereof, in admixture with a pharmaceutically acceptable carrier. In a preferred embodiment, the composition of matter comprises a growth inhibitory amount of a PRO polypeptide, or an agonist thereof. In another preferred embodiment, the composition comprises a cytotoxic amount of a PRO polypeptide, or an agonist thereof. Optionally, the compositions of matter may contain one or more additional growth inhibitory and/or cytotoxic and/or other chemotherapeutic agents.

In a further aspect, the present invention concerns compositions of matter useful for the treatment of a tumor in a mammal comprising a therapeutically effective amount of a PRO polypeptide as herein defined, or an agonist thereof. The tumor is preferably a cancer.

In another aspect, the invention concerns a method for inhibiting the growth of a tumor cell comprising exposing the cell to an effective amount of a PRO polypeptide as herein defined, or an agonist thereof. In a particular embodiment, the agonist is an anti-PRO agonist antibody. In another embodiment, the agonist is a small molecule that mimics the biological activity of a PRO polypeptide. The method may be performed *in vitro* or *in vivo*.

In a still further embodiment, the invention concerns an article of manufacture comprising:
(a) a container;
(b) a composition comprising an active agent contained within the container: wherein the composition is effective for inhibiting the neoplastic cell growth, *e.g*.. growth of tumor cells, and the active agent in the composition is a PRO polypeptide as herein defined, or an agonist thereof; and
(c) a label affixed to said container, or a package insert included in said container referring to the use of said PRO polypeptide or agonist thereof, for the inhibition of neoplastic cell growth, wherein the agonist may be an antibody which binds to the PRO polypeptide.
   In a particular embodiment, the agonist is an anti-PRO agonist antibody. In another embodiment, the agonist is a small molecule that mimics the biological activity of a PRO polypeptide. Similar articles of manufacture comprising a PRO polypeptide as herein defined, or an agonist thereof in an amount that is therapeutically effective for the treatment of tumor are also within the scope of the present invention. Also within the scope of the invention are articles of manufacture comprising a PRO polypeptide as herein defined, or an agonist thereof, and a further growth inhibitory agent, cytotoxic agent or chemotherapeutic agent.

### B. Additional Embodiments

In other embodiments of the present invention, the invention provides an isolated nucleic acid molecule comprising a nucleotide sequence that encodes a PRO polypeptide.

In one aspect, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO polypeptide cDNA as disclosed herein, the coding sequence of a PRO polypeptide lacking the signal peptide as disclosed herein, the coding sequence of an extracellular domain of a transmembrane PRO polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs deposited with the ATCC as disclosed herein, or (b) the complement of the DNA molecule of (a).

Another aspect of the invention provides an isolated nucleic acid molecule comprising a nucleotide sequence encoding a PRO polypeptide which is either transmembrane domain-deleted or transmembrane domain-inactivated, or is complementary to such encoding nucleotide sequence, wherein the transmembrane domain(s) of such polypeptides are disclosed herein. Therefore, soluble extracellular domains of the herein described PRO polypeptides are contemplated.

Another embodiment is directed to fragments of a PRO polypeptide coding sequence, or the complement thereof, that may find use as, for example, hybridization probes, for encoding fragments of a PRO polypeptide that may optionally encode a polypeptide comprising a binding site for an anti-PRO antibody or as antisense oligonucleotide probes. Such nucleic acid fragments are usually at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length and alternatively at least about 1000 nucleotides in length, wherein in this context the term "about" means the referenced nucleotide sequence length plus or minus 10% of that referenced length. It is noted that novel fragments of a PRO polypeptide-encoding nucleotide sequence may be determined in a routine manner by aligning the PRO polypeptide-encoding nucleotide sequence with other known nucleotide sequences using any of a number of well known sequence alignment programs and determining which PRO polypeptide-encoding nucleotide sequence fragment(s) are novel. All of such PRO polypeptide-encoding nucleotide sequences are contemplated herein. Also contemplated are the PRO polypeptide fragments encoded by these nucleotide molecule fragments, preferably those PRO polypeptide fragments that comprise a binding site for an anti-PRO antibody.

In another embodiment, the invention provides an isolated PRO polypeptide encoded by any of the isolated nucleic acid sequences hereinabove identified.

In a certain aspect, the invention concerns an isolated PRO polypeptide, comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO polypeptide comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% aminoacid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs deposited with the ATCC as disclosed herein.

In a further aspect, the invention concerns an isolated PRO polypeptide comprising an amino acid sequence scoring at least about 80% positives, alternatively at least about 81% positives, alternatively at least about 82% positives, alternatively at least about 83% positives, alternatively at least about 84% positives, alternatively at least about 85% positives, alternatively at least about 86% positives, alternatively at least about 87% positives, alternatively at least about 88% positives, alternatively at least about 89% positives, alternatively at least about 90% positives, alternatively at least about 91 % positives, alternatively at least about 92% positives, alternatively at least about 93% positives, alternatively at least about 94% positives, alternatively at least about 95% positives, alternatively at least about 96% positives, alternatively at least about 97% positives, alternatively at least about 98% positives and alternatively at least about 99% positives when compared with the amino acid sequence of a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a specific aspect, the invention provides an isolated PRO polypeptide without the N-terminal signal sequence and/or the initiating methionine and is encoded by a nucleotide sequence that encodes such an amino acid sequence as hereinbefore described. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

Another aspect of the invention provides an isolated PRO polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

In yet another embodiment, the invention concerns agonists of a native PRO polypeptide as defined herein. In a particular embodiment, the agonist is an anti-PRO antibody or a small molecule.

In a further embodiment, the invention concerns a method of identifying agonists to a PRO polypeptide which comprise contacting the PRO polypeptide with a candidate molecule and monitoring a biological activity mediated by said PRO polypeptide. Preferably, the PRO polypeptide is a native PRO polypeptide.

In a still further embodiment, the invention concerns a composition of matter comprising a PRO polypeptide, or an agonist of a PRO polypeptide as herein described, or an anti-PRO antibody, in combination with a carrier. Optionally, the carrier is a pharmaceutically acceptable carrier.

Another embodiment of the present invention is directed to the use of a PRO polypeptide, or an agonist thereof as hereinbefore described, or an anti-PRO antibody, for the preparation of a medicament useful in the treatment of a condition which is responsive to the PRO polypeptide, an agonist thereof or an anti-PRO antibody.

In additional embodiments of the present invention, the invention provides vectors comprising DNA encoding any of the herein described polypeptides. Host cells comprising any such vector are also provided. By way of example, the host cells may be CHO cells, *E*. *coli,* yeast, or Baculovirus-infected insect cells. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In yet another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

### BRIEF DESCRIPTION OF THE DRAWINGS

Figure 1 shows a nucleotide sequence (SEQ ID NO:1) of a native sequence PR0240 cDNA, wherein SEQ ID NO:1 is a clone designated herein as "DNA34387-1138".
Figure 2 shows the amino acid sequence (SEQ ID NO:2) derived from the coding sequence of SEQ ID NO:1 shown in Figure 1.
Figure 3 shows a nucleotide sequence (SEQ ID NO:3) of a native sequence PRO381 cDNA. wherein SEQ ID NO:3 is a clone designated herein as "DNA44194-1317".
Figure 4 shows the amino acid sequence (SEQ ID NO:4) derived from the coding sequence of SEQ ID NO:3 shown in Figure 3.
Figure 5 shows a nucleotide sequence (SEQ ID NO:5) of a native sequence PRO534 cDNA, wherein SEQ ID NO:5 is a clone designated herein as "DNA48333-1321".
Figure 6 shows the amino acid sequence (SEQ ID NO:6) derived from the coding sequence of SEQ ID NO:5 shown in Figure 5.
Figure 7 shows a nucleotide sequence (SEQ ID NO:7) of a native sequence PR0540 cDNA, wherein SEQ ID NO:7 is a clone designated herein as "DNA44189-1322".
Figure 8 shows the amino acid sequence (SEQ ID NO:8) derived from the coding sequence of SEQ ID NO:7 shown in Figure 7.
Figure 9 shows a nucleotide sequence (SEQ ID NO:9) of a native sequence PRO698 cDNA, wherein SEQ ID NO:9 is a clone designated herein as "DNA48320-1433".
Figure 10 shows the amino acid sequence (SEQ ID NO:10) derived from the coding sequence of SEQ ID NO:9 shown in Figure 9.
Figure 11 shows a nucleotide sequence (SEQ ID NO: 11) of a native sequence PRO982 cDNA, wherein SEQ ID NO:11 is a clone designated herein as "DNA57700-1408".
Figure 12 shows the amino acid sequence (SEQ ID NO:12) derived from the coding sequence of SEQ ID NO:11 shown in Figure 11.
Figure 13 shows a nucleotide sequence (SEQ ID NO: 13) of a native sequence PRO1005 cDNA. wherein SEQ ID NO:13 is a clone designated herein as "DNA57708-1411" .
Figure 14 shows the amino acid sequence (SEQ ID NO:14) derived from the coding sequence of SEQ ID NO:13 shown in Figure 13.
Figure 15 shows a nucleotide sequence (SEQ ID NO: 15) of a native sequence PRO1007 cDNA, wherein SEQ ID NO: 15 is a clone designated herein as "DNA57690-1374".
Figure 16 shows the amino acid sequence (SEQ ID NO:16) derived from the coding sequence of SEQ ID NO:15 shown in Figure 15.
Figure 17 shows a nucleotide sequence (SEQ ID NO:17) of a native sequence PRO1131 cDNA, wherein SEQ ID NO:17 is a clone designated herein as "DNA59777-1480".
Figure 18 shows the amino acid sequence (SEQ ID NO:18) derived from the coding sequence of SEQ ID NO:17 shown in Figure 17.
Figure 19 shows a nucleotide sequence (SEQ ID NO:19) of a native sequence PRO1157 cDNA, wherein SEQ ID NO:19 is a clone designated herein as "DNA60292-1506".
Figure 20 shows the amino acid sequence (SEQ ID NO:20) derived from the coding sequence of SEQ ID NO:19 shown in Figure 19.
Figure 21 shows a nucleotide sequence (SEQ ID NO:21) of a native sequence PRO1199 cDNA. wherein SEQ ID NO:21 is a clone designated herein as "DNA65351-1366-1".
Figure 22 shows the amino acid sequence (SEQ ID NO:22) derived from the coding sequence of SEQ ID NO:21 shown in Figure 21.
Figure 23 shows a nucleotide sequence (SEQ ID NO:33) of a native sequence PRO1265 cDNA, wherein SEQ ID NO:23 is a clone designated herein as "DNA60764-1533".
Figure 24 shows the amino acid sequence (SEQ ID NO:24) derived from the coding sequence of SEQ ID NO:23 shown in Figure 23.
Figure 25 shows a nucleotide sequence (SEQ ID NO:25) of a native sequence PRO 1286 cDNA, wherein SEQ ID NO:25 is a clone designated herein as "DNA64903-1553".
Figure 26 shows the amino acid sequence (SEQ ID NO:26) derived from the coding sequence of SEQ ID NO:25 shown in Figure 25.
Figure 27 shows a nucleotide sequence (SEQ ID NO:27) of a native sequence PRO 1313 cDNA. wherein SEQ ID NO:27 is a clone designated herein as "DNA64966-1575".
Figure 28 shows the amino acid sequence (SEQ ID NO:28) derived from the coding sequence of SEQ ID NO:27 shown in Figure 27.
Figure 29 shows a nucleotide sequence (SEQ ID NO:29) of a native sequence PRO 1338 cDNA, wherein SEQ ID NO:29 is a clone designated herein as "DNA66667".
Figure 30 shows the amino acid sequence (SEQ ID NO:30) derived from the coding sequence of SEQ ID NO:29 shown in Figure 29.
Figure 31 shows a nucleotide sequence (SEQ ID NO:31) of a native sequence PRO1375 cDNA, wherein SEQ ID NO:31 is a clone designated herein as "DNA67004-1614".
Figure 32 shows the amino acid sequence (SEQ ID NO:32) derived from the coding sequence of SEQ ID NO:31 shown in Figure 31.
Figure 33 shows a nucleotide sequence (SEQ ID NO:33) of a native sequence PRO1410 cDNA, wherein SEQ ID NO:33 is a clone designated herein as "DNA68874-1622".
Figure 34 shows the amino acid sequence (SEQ ID NO:34) derived from the coding sequence of SEQ ID NO:33 shown in Figure 33.
Figure 35 shows a nucleotide sequence (SEQ ID NO:35) of a native sequence PRO 1488 cDNA, wherein SEQ ID NO:35 is a clone designated herein as "DNA73736-1657".
Figure 36 shows the amino acid sequence (SEQ ID NO:36) derived from the coding sequence of SEQ ID NO:35 shown in Figure 35.
Figure 37 shows a nucleotide sequence (SEQ ID NO:37) of a native sequence PRO3438 cDNA, wherein SEQ ID NO:37 is a clone designated herein as "DNAR2364-2538".
Figure 38 shows the amino acid sequence (SEQ ID NO:38) derived from the coding sequence of SEQ ID NO:37 shown in Figure 37.
Figure 39 shows a nucleotide sequence (SEQ ID NO:39) of a native sequence PRO4302 cDNA. wherein SEQ ID NO:39 is a clone designated herein as "DNA92218-2554".
Figure 40 shows the amino acid sequence (SEQ ID NO:40) derived from the coding sequence of SEQ ID NO:39 shown in Figure 39.
Figure 41 shows a nucleotide sequence (SEQ ID NO:41) of a native sequence PRO4400 cDNA, wherein SEQ ID NO:41 is a clone designated herein as "DNA87974-2609".
Figure 42 shows the amino acid sequence (SEQ ID NO:42) derived from the coding sequence of SEQ ID NO:41 shown in Figure 41.
Figure 43 shows a nucleotide sequence (SEQ ID NO:43) of a native sequence PRO5725 cDNA, wherein SEQ ID NO:43 is a clone designated herein as "DNA92265-2669".
Figure 44 shows the amino acid sequence (SEQ ID NO:44) derived from the coding sequence of SEQ ID NO:43 shown in Figure 43.
Figure 45 shows a nucleotide sequence (SEQ ID NO:45) of a native sequence PRO183 cDNA, wherein SEQ ID NO:45 is a clone designated herein as "DNA28498".
Figure 46 shows the amino acid sequence (SEQ ID NO:46) derived from the coding sequence of SEQ ID NO:45 shown in Figure 45.
Figure 47 shows a nucleotide sequence (SEQ ID NO:47) of a native sequence PRO202 cDNA, wherein SEQ ID NO:47 is a clone designated herein as "DNA30869".
Figure 48 shows the amino acid sequence (SEQ ID NO:48) derived from the coding sequence of SEQ ID NO:47 shown in Figure 47.
Figure 49 shows a nucleotide sequence (SEQ ID NO:49) of a native sequence PRO542 cDNA, wherein SEQ ID NO:49 is a clone designated herein as "DNA56505".
Figure 50 shows the amino acid sequence (SEQ ID NO:50) derived from the coding sequence of SEQ ID NO:49 shown in Figure 49.
Figure 51 shows a nucleotide sequence (SEQ ID NO:51) of a native sequence PRO861 cDNA, wherein SEQ ID NO:51 is a clone designated herein as "DNA50798".
Figure 52 shows the amino acid sequence (SEQ ID NO:52) derived from the coding sequence of SEQ ID NO:51 shown in Figure 51.
Figure 53 shows a nucleotide sequence (SEQ ID NO:53) of a native sequence PRO1096 cDNA, wherein SEQ ID NO:53 is a clone designated herein as "DNA61870".
Figure 54 shows the amino acid sequence (SEQ ID NO:54) derived from the coding sequence of SEQ ID NO:53 shown in Figure 53.
Figure 55 shows a nucleotide sequence (SEQ ID NO:55) of a native sequence PRO3562 cDNA, wherein SEQ ID NO:55 is a clone designated herein as "DNA96791".
Figure 56 shows the amino acid sequence (SEQ ID NO:56) derived from the coding sequence of SEQ ID NO:55 shown in Figure 55.

### DETAILED DESCRIPTION OF THE INVENTION

The terms "PRO polypeptide", and "PRO" as used herein and when immediately followed by a numerical designation refer to various polypeptides, wherein the complete designation (*i.e*., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods.

A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (*e.g*., an extracellular domain sequence), naturally-occurring variant forms (*e.g*., alternatively spliced forms) and naturally-occurring allelic variants of the polypeptide. In various embodiments of the invention, the native sequence PRO polypeptides disclosed herein are mature or full-length native sequence polypeptides comprising the full-length amino acid sequences shown in the accompanying figures. Start and stop codons are shown in bold font and underlined in the figures. However, while the PRO polypeptide disclosed in the accompanying figures are shown to begin with methionine residues designated herein as amino acid position I in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD" refers to a form of the PRO polypeptide which is essentially free of the transmembrane and cytoplasmic domains. Ordinarily, a PRO polypeptide ECD will have less than 1% of such transmembrane and/or cytoplasmic domains and preferably, will have less than 0.5% of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are comtemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (*e.g*., Nielsen et al., Prot. Eng., 10:1-6 (1997) and von Heinje et al., Nucl. Acids Res., 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, alternatively at least about 20 amino acids in length, alternatively at least about 30 amino acids in length, alternatively at least about 40 amino acids in length, alternatively at least about 50 amino acids in length, alternatively at least about 60 amino acids in length, alternatively at least about 70 amino acids in length, alternatively at least about 80 amino acids in length, alternatively at least about 90 amino acids in length, alternatively at least about 100 amino acids in length, alternatively at least about 150 amino acids in length, alternatively at least about 200 amino acids in length, alternatively at least about 300 amino acids in length, or more.

"Percent (%) amino acid sequence identity" with respect to the PRO polypeptide sequences identified herein is defined as the percentage of amino acid residues in a candidate sequence that are identical with the amino acid residues in a PRO sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however. % amino acid sequence identity values are obtained as described below by using the sequence comparison computer program ALIGN-2. wherein the complete source code for the ALIGN-2 program is provided in Table I. The ALIGN-2 sequence comparison computer program was authored by Genentech, Inc., and the source code shown in Table I has been filed with user documentation in the U.S. Copyright Office. Washington D.C., 20559, where it is registered under U.S. Copyright Registration No. TXU510087. The ALIGN-2 program is publicly available through Genentech. Inc., South San Francisco. California or may be compiled from the source code provided in Table 1. The ALIGN-2 program should be compiled for use on a UNIX operating system, preferably digital UNIX V4.0D. All sequence comparison parameters are set by the ALIGN-2 program and do not vary.

For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B. and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations. Tables 2-3 demonstrate how to calculate the % amino acid sequence identity of the amino acid sequence designated "Comparison Protein" to the amino acid sequence designated "PRO".

Unless specifically stated otherwise, all % amino acid sequence identity values used herein are obtained as described above using the ALIGN-2 sequence comparison computer program. However, % amino acid sequence identity may also be determined using the sequence comparison program NCBI-BLAST2 (Altschul et al., Nucleic Acids Res., 25:3389-3402 (1997)). The NCBI-BLAST2 sequence comparison program may be downloaded from http://www.ncbi.nlm.nih.gov, or otherwise obtained from the National Institute of Health. Bethesda, MD. NCBI-BLAST2 uses several search parameters, wherein all of those search parameters are set to default values including, for example, unmask = yes, strand = all, expected occurrences = 10, minimum low complexity length =15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

In addition, % amino acid sequence identity may also be determined using the WU-BLAST-2 computer program (Altschul et al., Methods in Enzymology, 266:460-480 (1996)). Most of the WU-BLAST-2 search parameters are set to the default values. Those not set to default values, *i.e*., the adjustable parameters, are set with the following values: overlap span = 1, overlap fraction = 0.125, word threshold (T) = 11, and scoring matrix = BLOSUM62. For purposes herein, a % amino acid sequence identity value is determined by dividing (a) the number of matching identical amino acids residues between the amino acid sequence of the PRO polypeptide of interest having a sequence derived from the native PRO polypeptide and the comparison amino acid sequence of interest (*i.e*., the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

"PRO variant polynucleotide" or "PRO variant nucleic acid sequence" means a nucleic acid molecule which encodes an active PRO polypeptide as defined below and which has at least about 80% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Ordinarily, a PRO variant polynucleotide will have at least about 80% nucleic acid sequence identity, alternatively at least about 81 % nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87%a nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, alternatively at least about 60 nucleotides in length, alternatively at least about 90 nucleotides in length, alternatively at least about 120 nucleotides in length, alternatively at least about 150 nucleotides in length, alternatively at least about 180 nucleotides in length, alternatively at least about 210 nucleotides in length, alternatively at least about 240 nucleotides in length, alternatively at least about 270 nucleotides in length, alternatively at least about 300 nucleotides in length, alternatively at least about 450 nucleotides in length, alternatively at least about 600 nucleotides in length, alternatively at least about 900 nucleotides in length, or more.

"Percent (%) nucleic acid sequence identity" with respect to the PRO polypeptide-encoding nucleic acid sequences identified herein is defined as the percentage of nucleotides in a candidate sequence that are identical with the nucleotides in a PRO polypeptide-encoding nucleic acid sequence, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST. BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % nucleic acid sequence identity values are obtained as described below by using the sequence comparison computer program ALIGN-2, wherein the complete source code for the ALIGN-2 program is provided in Table 1. The ALIGN-2 sequence comparison computer program was authored by Genentech, Inc., and the source code shown in Table 1 has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table I. The ALIGN-2 program should be compiled for use on a UNIX operating system, preferably digital UNIX V4.0D. All sequence comparison parameters are set by the ALIGN-2 program and do not vary.

For purposes herein, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program ALIGN-2 in that program's alignment of C and D. and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 4.5 demonstrate how to calculate the % nucleic acid sequence identity of the nucleic acid sequence designated "Comparison DNA" to the nucleic acid sequence designated "PRO-DNA".

Unless specifically stated otherwise, all % nucleic acid sequence identity values used herein are obtained as described above using the ALIGN-2 sequence comparison computer program. However, % nucleic acid sequence identity may also be determined using the sequence comparison program NCBI-BLAST2 (Altschul et al., Nucleic Acids Res., 25:3389-3402 (1997)). The NCBI-BLAST2 sequence comparison program may be downloaded from http://www.ncbi.nlm.nih.gov, or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In addition, % nucleic acid sequence identity values may also be generated using the WU-BLAST-2 computer program (Altschul et al., Methods in Enzymology, 266:460-480 (1996)). Most of the WU-BLAST-2 search parameters are set to the default values. Those not set to default values, *i.e*., the adjustable parameters, are set with the following values: overlap span = 1, overlap fraction = 0.125, word threshold (T) = 11, and scoring matrix = BLOSUM62. For purposes herein, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (*i.e*., the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

In other embodiments, PRO variant polynucleotides are nucleic acid molecules that encode an active PRO polypeptide and which are capable of hybridizing, preferably under stringent hybridization and wash conditions, to nucleotide sequences encoding the full-length PRO polypeptide shown in the accompanying figures herein. PRO variant polypeptides may be those that are encoded by a PRO variant polynucleotide.

The term "positives", in the context of the amino acid sequence identity comparisons performed as described above, includes amino acid residues in the sequences compared that are not only identical, but also those that have similar properties. Amino acid residues that score a positive value to an amino acid residue of interest are those that are either identical to the amino acid residue of interest or are a preferred substitution (as defined in Table 6 below) of the amino acid residue of interest.

For purposes herein, the % value of positives of a given amino acid sequence A to, with, or against a given amino acid sequence B (which can alternatively be phrased as a given amino acid sequence A that has or comprises a certain % positives to, with, or against a given amino acid sequence B) is calculated as follows:
100 times the fraction X/Y
where X is the number of amino acid residues scoring a positive value by the sequence alignment program ALIGN-2 in that program's alignment of A and B, and where Y is the total number of amino acid residues in B. It will be appreciated that where the length of amino acid sequence A is not equal to the length of amino acid sequence B, the % positives of A to B will not equal the % positives of B to A.

"Isolated", when used to describe the various polypeptides disclosed herein, means a polypeptide that has been identified and separated and/or recovered from a component of its natural environment. Preferably, the isolated polypeptide is free of association with all components with which it is naturally associated. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptides includes polypeptides *in situ* within recombinant cells, since at least one component of the PRO polypeptide natural environment will not be present. Ordinarily, however, isolated polypeptides will be prepared by at least one purification step.

An "isolated" nucleic acid molecule encoding a PRO polypeptide or an "isolated" nucleic acid molecule encoding an anti-PRO antibody is a nucleic acid molecule that is identified and separated from at least one contaminant nucleic acid molecule with which it is ordinarily associated in the natural source of the PRO-encoding nucleic acid or the natural source of the anti-PRO-encoding nucleic acid. Preferably, the isolated nucleic acid is free of association with all components with which it is naturally associated. An isolated PRO-encoding nucleic acid molecule or an isolated anti-PRO-encoding nucleic acid molecule is other than in the form or setting in which it is found in nature. Isolated nucleic acid molecules therefore are distinguished from the PRO-encoding nucleic acid molecule or from the anti-PRO-encoding nucleic acid molecule as it exists in natural cells. However, an isolated nucleic acid molecule encoding a PRO polypeptide or an isolated nucleic acid molecule encoding an anti-PRO antibody includes PRO-nucleic acid molecules or anti-PRO-nucleic acid molecules contained in cells that ordinarily express PRO polypeptides or anti-PRO antibodies where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a PRO polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO monoclonal antibodies (including agonist antibodies), anti-PRO antibody compositions with polyepitopic specificity, single chain anti-PRO antibodies, and fragments of ami-PRO antibodies (see below). The term "monoclonal antibody" as used herein refers to an antibody obtained from a population of substantially homogeneous antibodies, *i.e.,* the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature that can be used. As a result, it follows that higher relative temperatures would tend to make the reaction conditions more stringent, while lower temperatures less so. For additional details and explanation of stringency of hybridization reactions, *see*, Ausubel et al., Current Protocols in Molecular Biology (Wiley Interscience Publishers, 1995).

"Stringent conditions" or "high-stringency conditions", as defined herein, may be identified by those that: (1) employ low ionic strength and high temperature for washing, for example, 0.015 M sodium chloride/0.0015 M sodium citrate/0.1 % sodium dodecyl sulfate at 50°C; (2) employ during hybridization a denaturing agent, such as formamide, for example, 50% (v/v) formamide with 0.1 % bovine serum albumin/0.1 % Ficoll/0.1 % polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1 % sodium pyrophosphate, 5 x Denhardt's solution, sonicated salmon sperm DNA (50 µg/ml), 0.1 % SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately-stringent conditions" may be identified as described by Sambrook et al., Molecular Cloning: A Laboratory Manual (New York: Cold Spring Harbor Press, 1989), and include the use of washing solution and hybridization conditions (*e.g*., temperature, ionic strength, and % SDS) less stringent than those described above. An example of moderately stringent conditions is overnight incubation at 37°C in a solution comprising: 20% formamide, 5 x SSC (150 mM NaCl. 15 mM trisodium citrate). 50 mM sodium phosphate (pH 7.6). 5 x Denhardt's solution, 10% dextran sulfate, and 20 mg/ml denatured sheared salmon sperm DNA. followed by washing the filters in I x SSC at about 37°-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with otherepitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i*.*e*., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

"Active" or "activity" for the purposes herein refers to form(s) of PRO polypeptides which retain a biological and/or an immunological activity of native or naturally-occurring PRO polypeptides, wherein "biological" activity refers to a biological function (either inhibitory or stimulatory) caused by a native or naturally-occurring PRO polypeptide other than the ability to induce the production of an antibody against an antigenic epitope possessed by a native or naturally-occurring PRO polypeptide and an "immunological" activity refers to the ability to induce the production of an antibody against an antigenic epitope possessed by a native or naturally-occurring PRO polypeptide.

"Biological activity" in the context of an antibody or another agonist that can be identified by the screening assays disclosed herein (*e.g*., an organic or inorganic small molecule, peptide, etc.) is used to refer to the ability of such molecules to invoke one or more of the effects listed herein in connection with the definition of a "therapeutically effective amount." In a specific embodiment, "biological activity" is the ability to inhibit neoplastic cell growth or proliferation. A preferred biological activity is inhibition, including slowing or complete stopping, of the growth of a target tumor (*e.g*., cancer) cell. Another preferred biological activity is cytotoxic activity resulting in the death of the target tumor *(e.g.,* cancer) cell. Yet another preferred biological activity is the induction of apoptosis of a target tumor (*e.g*., cancer) cell.

The phrase "immunological activity" means immunological cross-reactivity with at least one epitope of a PRO polypeptide.

"Immunological cross-reactivity" as used herein means that the candidate polypeptide is capable of competitively inhibiting the qualitative biological activity of a PRO polypeptide having this activity with polyclonal antisera raised against the known active PRO polypeptide. Such antisera are prepared in conventional fashion by injecting goats or rabbits, for example, subcutaneously with the known active analogue in complete Freund's adjuvant, followed by booster intraperitoneal or subcutaneous injection in incomplete Freunds. The immunological cross-reactivity preferably is "specific", which means that the binding affinity of the immunologically cross-reactive molecule (*e.g*., antibody) identified, to the corresponding PRO polypeptide is significantly higher (preferably at least about 2-times, more preferably at least about 4-times, even more preferably at least about 6-times, most preferably at least about 8-times higher) than the binding affinity of that molecule to any other known native polypeptide.

"Tumor", as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include breast cancer, prostate cancer, colon cancer, squamous cell cancer, small-cell lung cancer, non-small cell lung cancer, ovarian cancer, cervical cancer, gastrointestinal cancer, pancreatic cancer, glioblastoma, liver cancer, bladder cancer, hepatoma, colorectal cancer, endometrial carcinoma, salivary gland carcinoma, kidney cancer, vul val cancer, thyroid cancer, hepatic carcinoma and various types of head and neck cancer.

"Treatment" is an intervention performed with the intention of preventing the development or altering the pathology of a disorder. Accordingly, "treatment" refers to both therapeutic treatment and prophylactic or preventative measures. Those in need of treatment include those already with the disorder as well as those in which the disorder is to be prevented. In tumor *(e.g.,* cancer) treatment, a therapeutic agent may directly decrease the pathology of tumor cells, or render the tumor cells more susceptible to treatment by other therapeutic agents, *e.g*., radiation and/or chemotherapy.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, etc.

An "effective amount" of a polypeptide disclosed herein or an agonist thereof, in reference to inhibition of neoplastic cell growth, is an amount capable of inhibiting, to some extent, the growth of target cells. The term includes an amount capable of invoking a growth inhibitory, cytostatic and/or cytotoxic effect and/or apoptosis of the target cells. An "effective amount" of a PRO polypeptide or an agonist thereof for purposes of inhibiting neoplastic cell growth may be determined empirically and in a routine manner.

A "therapeutically effective amount", in reference to the treatment of tumor, refers to an amount capable of invoking one or more of the following effects: (I) inhibition, to some extent, of tumor growth, including, slowing down and complete growth arrest; (2) reduction in the number of tumor cells; (3) reduction in tumor size; (4) inhibition (*i.e*., reduction, slowing down or complete stopping) of tumor cell infiltration into peripheral organs; (5) inhibition (*i.e*., reduction, slowing down or complete stopping) of metastasis; (6) enhancement of anti-tumor immune response, which may, but does not have to, result in the regression or rejection of the tumor; and/or (7) relief, to some extent, of one or more symptoms associated with the disorder. A "therapeutically effective amount" of a PRO polypeptide or an agonist thereof for purposes of treatment of tumor may be determined empirically and in a routine manner.

A "growth inhibitory amount" of a PRO polypeptide or an agonist thereof is an amount capable of inhibiting the growth of a cell, especially tumor, *e.g*., cancer cell, either *in vitro* or *in vivo.* A "growth inhibitory amount" of a PRO polypeptide or an agonist thereof for purposes of inhibiting neoplastic cell growth may be determined empirically and in a routine manner.

A "cytotoxic amount" of a PRO polypeptide or an agonist thereof is an amount capable of causing the destruction of a cell, especially tumor, *e.g.,* cancer cell, either *in vitro* or *in vivo.* A "cytotoxic amount" of a PRO polypeptide or an agonist thereof for purposes of inhibiting neoplastic cell growth may be determined empirically and in a routine manner.

The term "cytotoxic agent" as used herein refers to a substance that inhibits or prevents the function of cells and/or causes destruction of cells. The term is intended to include radioactive isotopes (*e.g*., I¹³¹,I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of tumor, *e.g*., cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, *e.g.,* paclitaxel (Taxol, Bristol-Myers Squibb Oncology, Princeton, NJ), and doxetaxel (Taxotere, Rhône-Poulenc Rorer, Antony, Rnace), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins *(see,* U.S. Patent No. 4,675,187), melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially tumor, *e.g.,* cancer cell, either *in vitro* or *in vivo.* Thus, the growth inhibitory agent is one which significantly reduces the percentage of the target cells in S phase. Examples of growth inhibitory agents include agents that block cell cycle progression (at a place other than S phase), such as agents that induce G I arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G I also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al., (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β: insulin-like growth factor-I and -II: erythropoietin (EPO); osteoinductive factors; interferons such as interferon-α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1. IL-1α, IL-2, IL-3. IL-4, IL-5, IL-6, IL-7, IL-8, IL-9, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The term "prodrug" as used in this application refers to a precursor or derivative form of a pharmaceutically active substance that is less cytotoxic to tumor cells compared to the parent drug and is capable of being enzymatically activated or converted into the more active parent form. *See, e.g.,* Wilman, "Prodrugs in Cancer Chemotherapy". Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, glycosylated prodrugs oroptionally substituted phenylacetamide-containing prodrugs. 5-fluorocytosine and other 5-fluorouridine prodrugs which can be derivatized into prodrug form for use in this invention include, but are not limited to, those chemotherapeutic agents described above.

The term "agonist" is used in the broadest sense and includes any molecule that mimics a biological activity of a native PRO polypeptide disclosed herein. Suitable agonist molecules specifically include agonist antibodies or antibody fragments, fragments or amino acid sequence variants of native PRO polypeptides, peptides, small organic molecules, etc. Methods for identifying agonists of a PRO polypeptide may comprise contacting a tumor cell with a candidate agonist molecule and measuring the inhibition of tumor cell growth.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework regions (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (*see*, Kabat et al., NIH Publ. No.91-3242, Vol. I, pages 647-669 (1991)). The constant domains are not involved directly in binding an antibody to an antigen, but exhibit various effector functions, such as participation of the antibody in antibody-dependent cellular toxicity.

The term "hypervariable region" when used herein refers to the amino acid residues of an antibody which are responsible for antigen-binding. The hypervariable region comprises amino acid residues from a "complementarity determining region" or "CDR" *(i.e.,* residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institute of Health, Bethesda, MD. [1991]) and/or those residues from a "hypervariable loop" *(i.e.,* residues 26-32 (L1), 50-52 (L2) and 91-96 (L3) in the light chain variable domain and 26-32 (HI), 53-55 (H2) and 96-101 (H3) in the heavy chain variable domain; Clothia and Lesk, J. Mol. Biol., 196:901-917 [1987]). "Framework" or "FR" residues are those variable domain residues other than the hypervariable region residues as herein defined.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (Zapata et al., Protein Eng., 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')₂ fragment that has two antigen-combining sites and is still capable of cross-linking antigen.

"Fv" is the minimum antibody fragment which contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH I domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to differentclasses. There are five major classes of immunoglobulins: IgA,IgD, IgE, IgG, and IgM, and several of these may be further divided into subclasses (isotypes), *e.g*., IgG, IgG2, IgG3, IgG4, IgA, and IgA2.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al.; Nature, 256:495 [1975], or may be made by recombinant DNA methods (*see*, *e.g.,* U.S. Patent No. 4,816,567). The "monoclonal antibodies" may also be isolated from phage antibody libraries using the techniques described in Clackson et al., Nature, 352:624-628 [1991] and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 [1984]).

"Humanized" forms of non-human (*e.g.*, murine) antibodies are chimeric immunoglobulins. immunoglobulin chains or fragments thereof (such as Fv. Fab, Fab'. F(ab')₂ or other antigen-binding subsequences of antibodies) which contain minimal sequence derived from non-human immunoglobulin. For the most part, humanized antibodies are human immunoglobulins (recipient antibody) in which residues from a CDR of the recipient are replaced by residues from a CDR of a non-human species (donor antibody) such as mouse, rat or rabbit having the desired specificity, affinity, and capacity. In some instances, Fv FR residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, *see*, Jones et al., Nature, 321:522-525 (1986); Reichmann et al., Nature, 332:323-329 [1988]; and Presta, Curr. Op. Struct. Biol., 2:593-596 (1992). The humanized antibody includes a PRIMATIZED™ antibody wherein the antigen-binding region of the antibody is derived from an antibody produced by immunizing macaque monkeys with the antigen of interest.

"Single-chain Fv" or "sFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the sFv to form the desired structure for antigen binding. For a review of sFv, *see*, Pluckthun in The Pharmacology of Monoclonal Antibodies, Vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The word "label" when used herein refers to a detectable compound or composition which is conjugated directly or indirectly to the antibody so as to generate a "labeled" antibody. The label may be detectable by itself (*e.g*., radioisotope labels or fluorescent labels) or, in the case of an enzymatic label, may catalyze chemical alteration of a substrate compound or composition which is detectable. The label may also be a non-detectable entity such as a toxin.

By "solid phase" is meant a non-aqueous matrix to which the antibody of the present invention can adhere. Examples of solid phases encompassed herein include those formed partially or entirely of glass (*e.g.,* controlled pore glass), polysaccharides (*e.g.,* agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

As shown below, Table I provides the complete source code for the ALIGN-2 sequence comparison computer program. This source code may be routinely compiled for use on a UNIX operating system to provide the ALIGN-2 sequence comparison computer program.

In addition, Tables 2-5 show hypothetical exemplifications for using the below described method to determine % amino acid sequence identity (Tables 2-3) and % nucleic acid sequence identity (Tables 4-5) using the ALIGN-2 sequence comparison computer program, wherein "PRO" represents the amino acid sequence of a hypothetical PRO polypeptide of interest, "Comparison Protein" represents the amino acid sequence of a polypeptide against which the "PRO" polypeptide of interest is being compared, "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, "X", "Y", and "Z" each represent different hypothetical amino acid residues and "N", "L" and "V" each represent different hypothetical nucleotides.

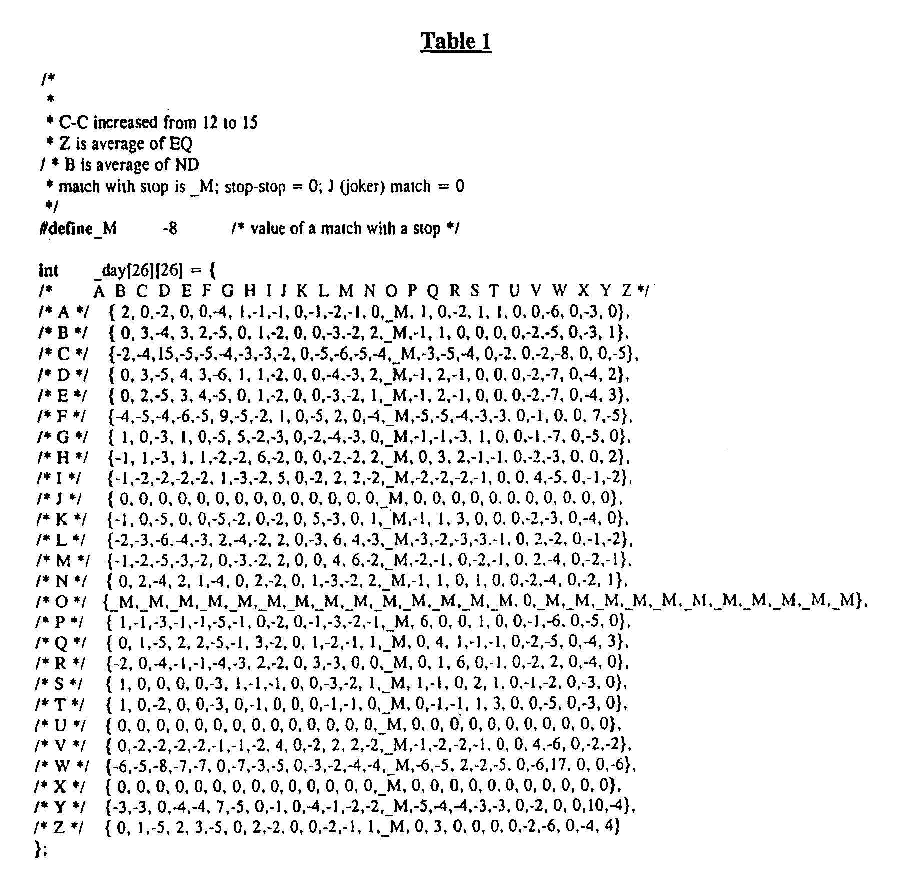

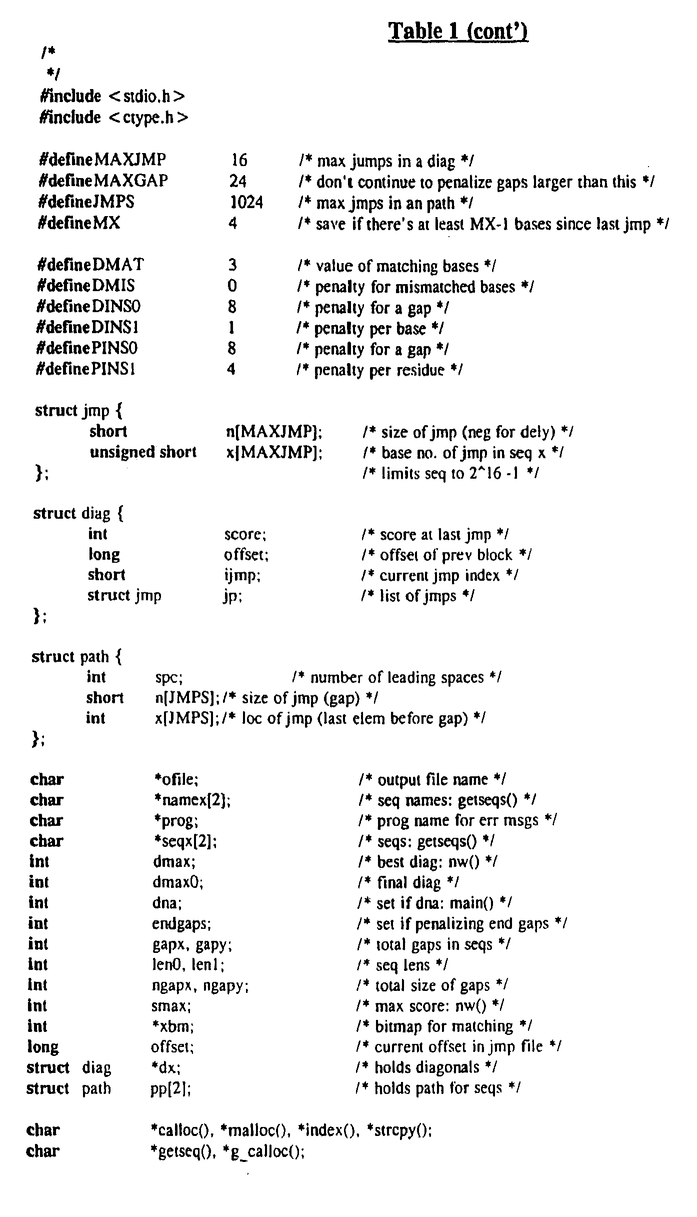

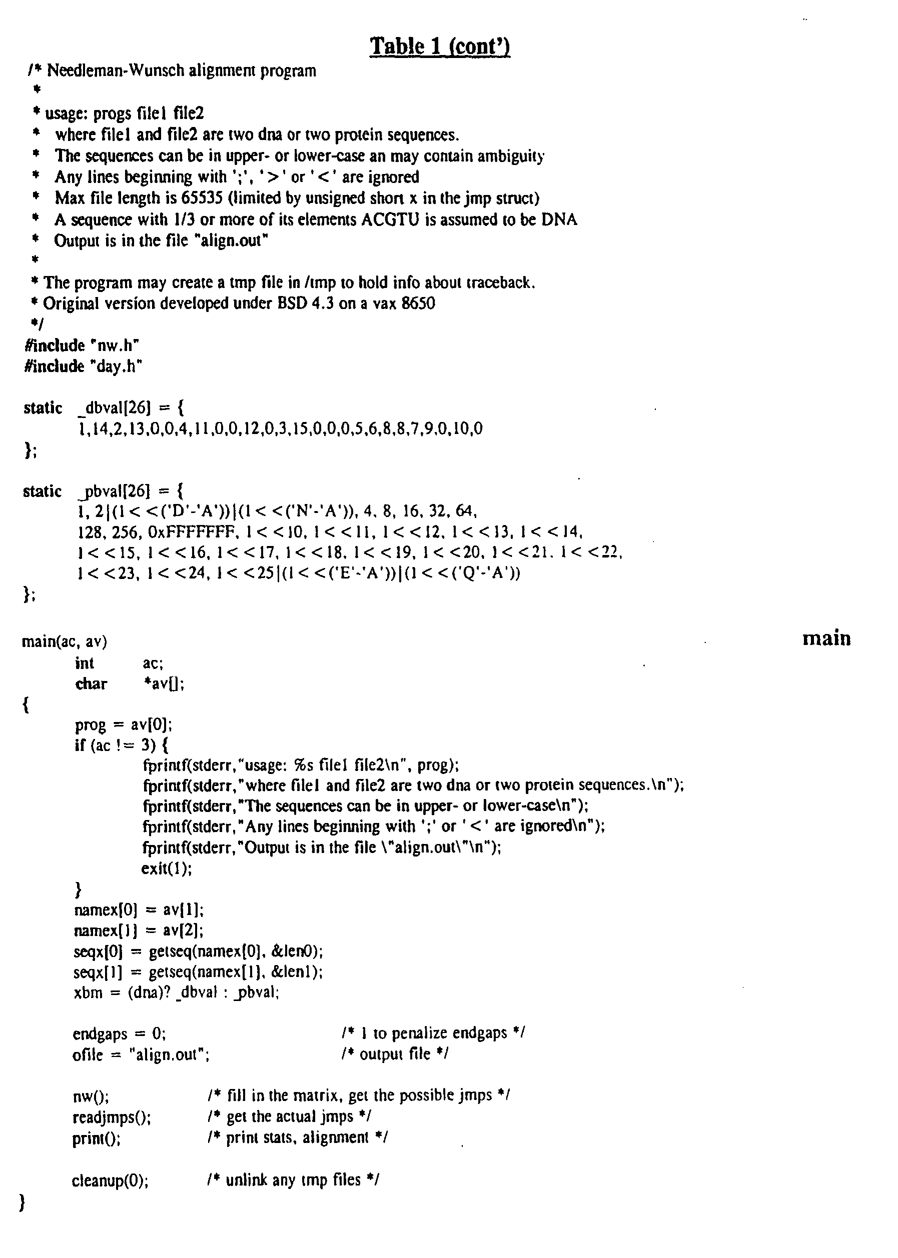

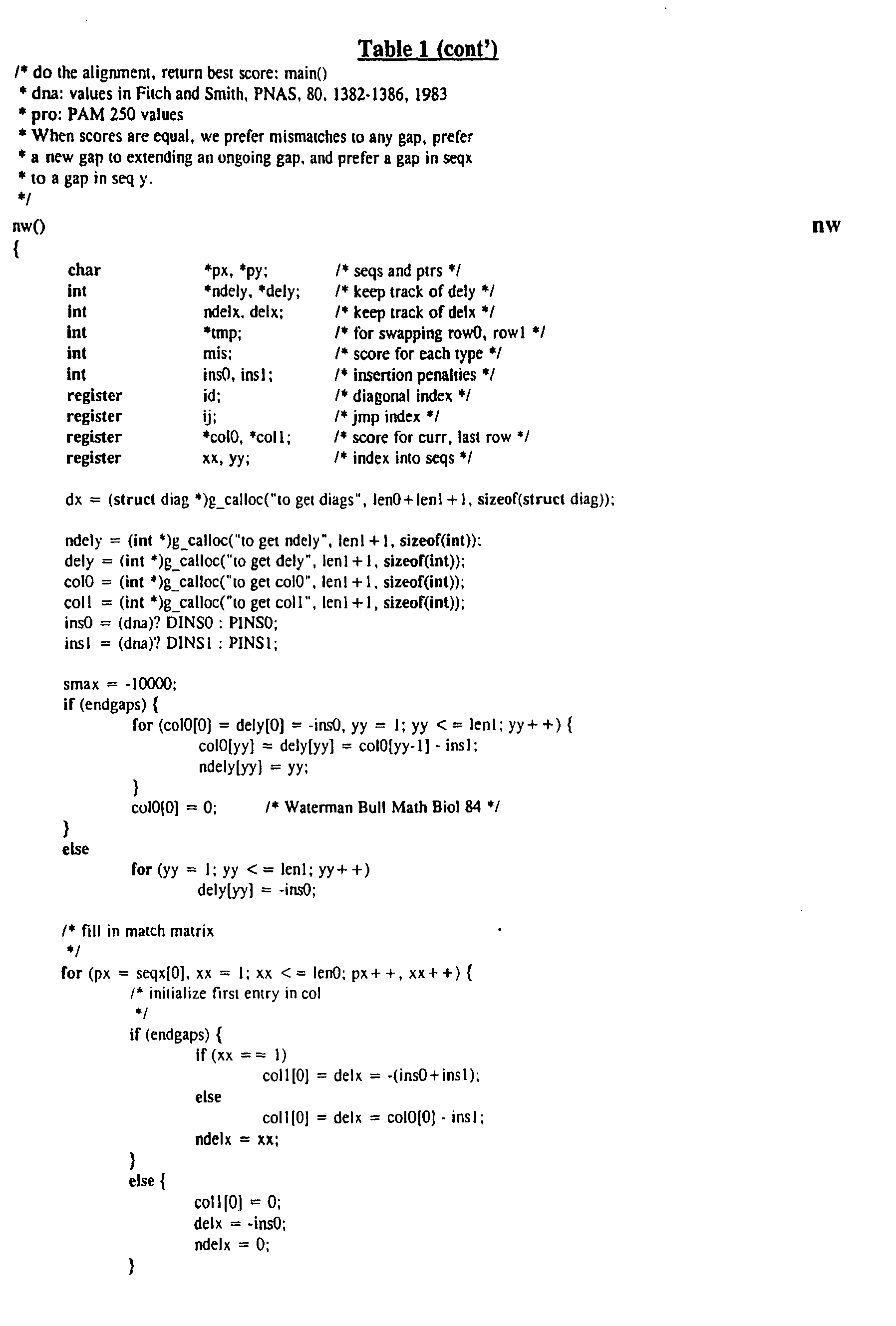

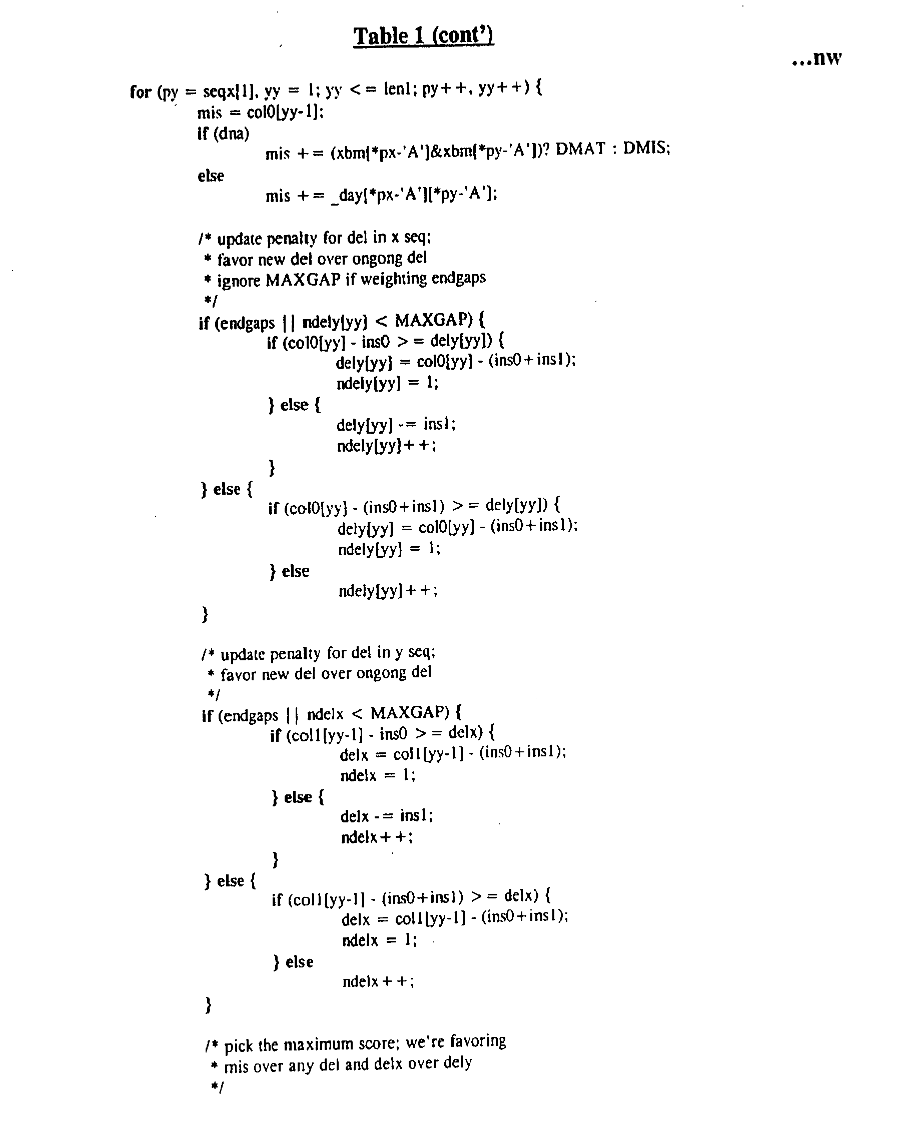

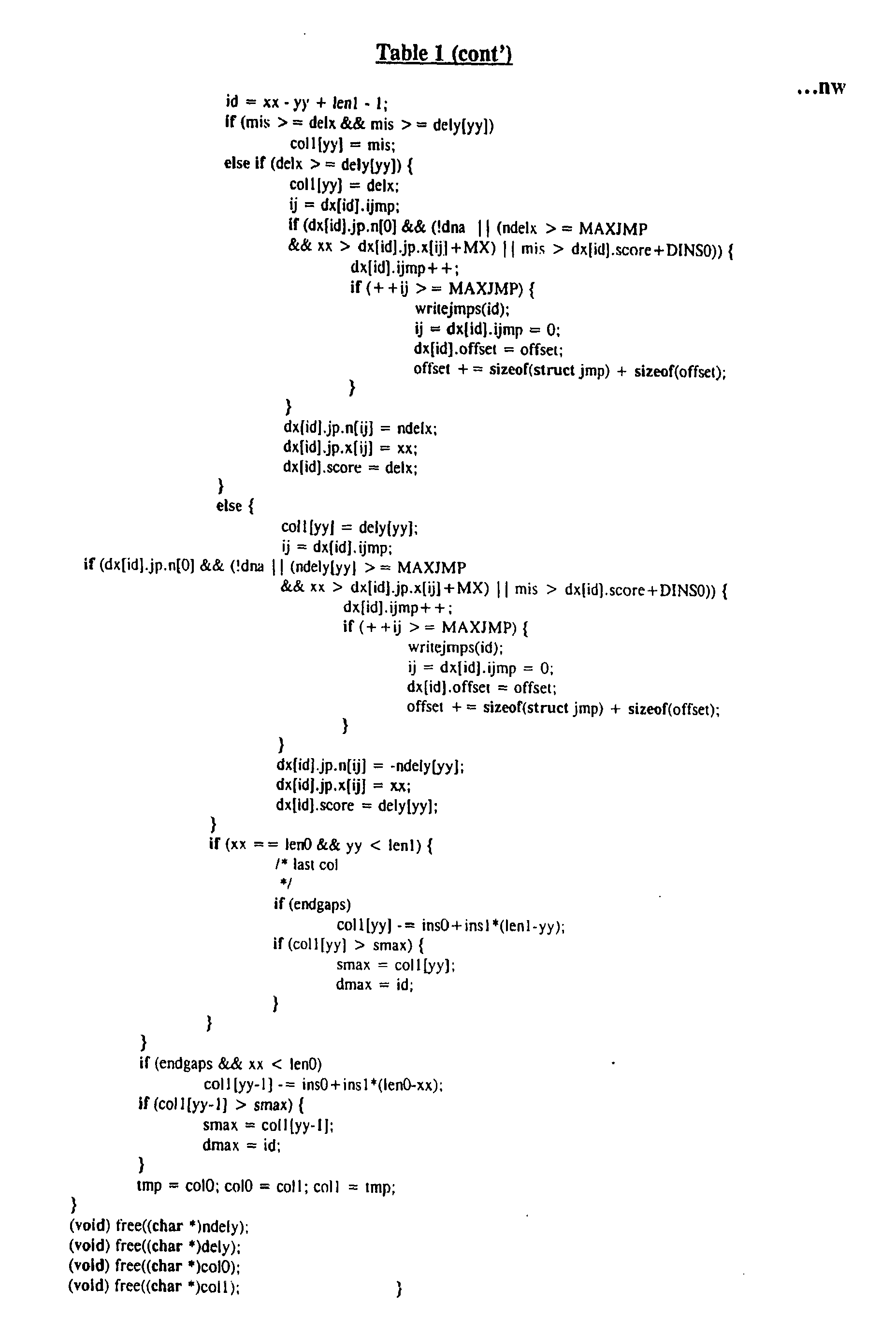

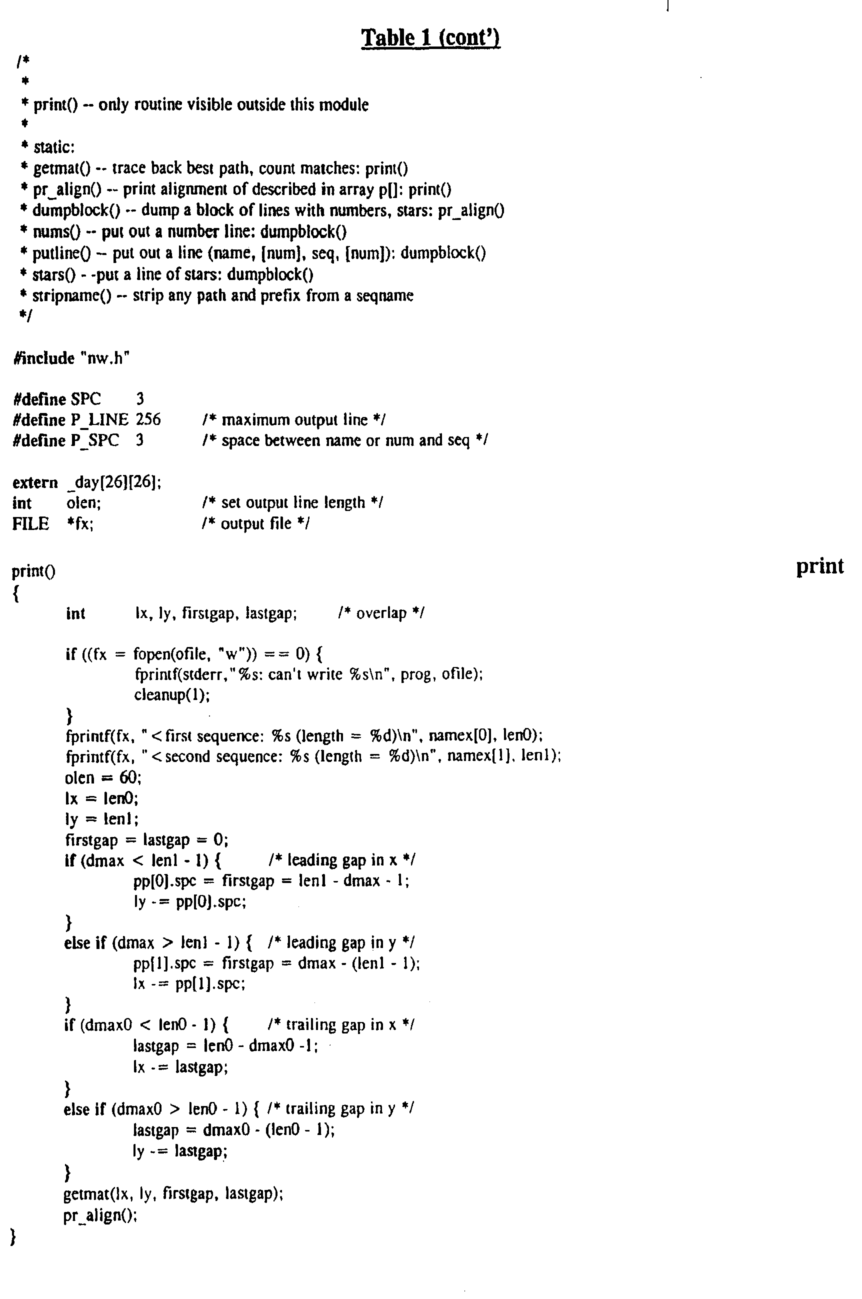

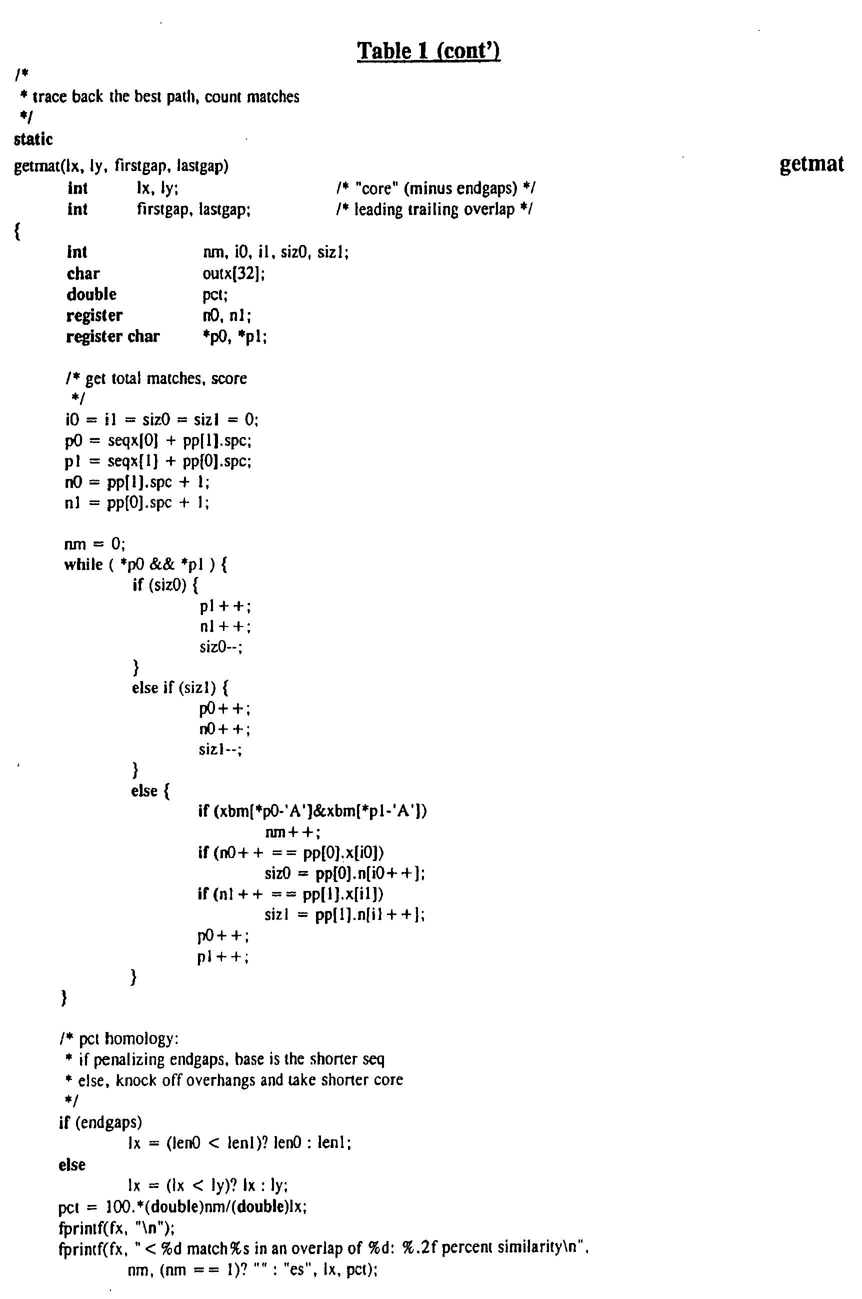

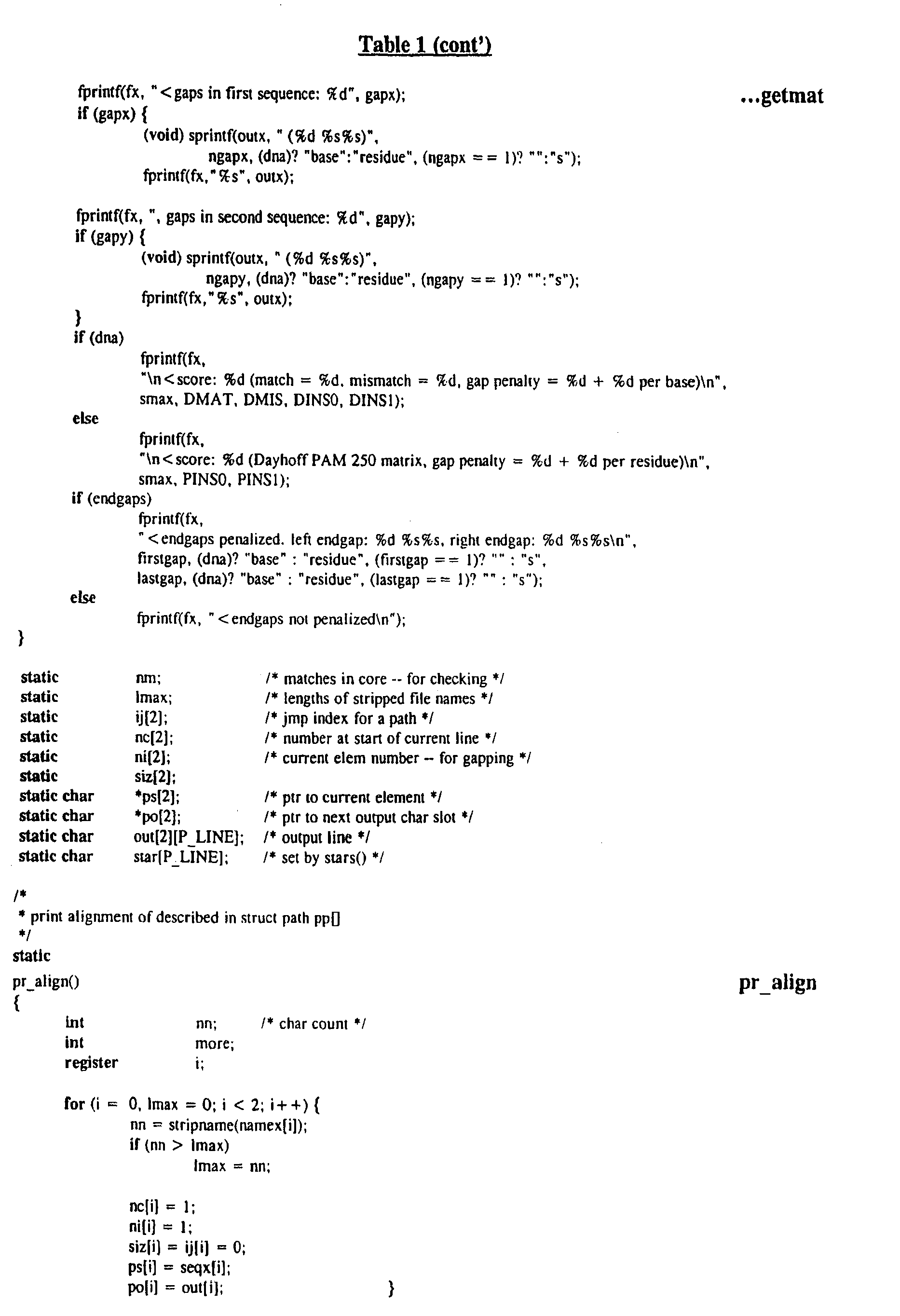

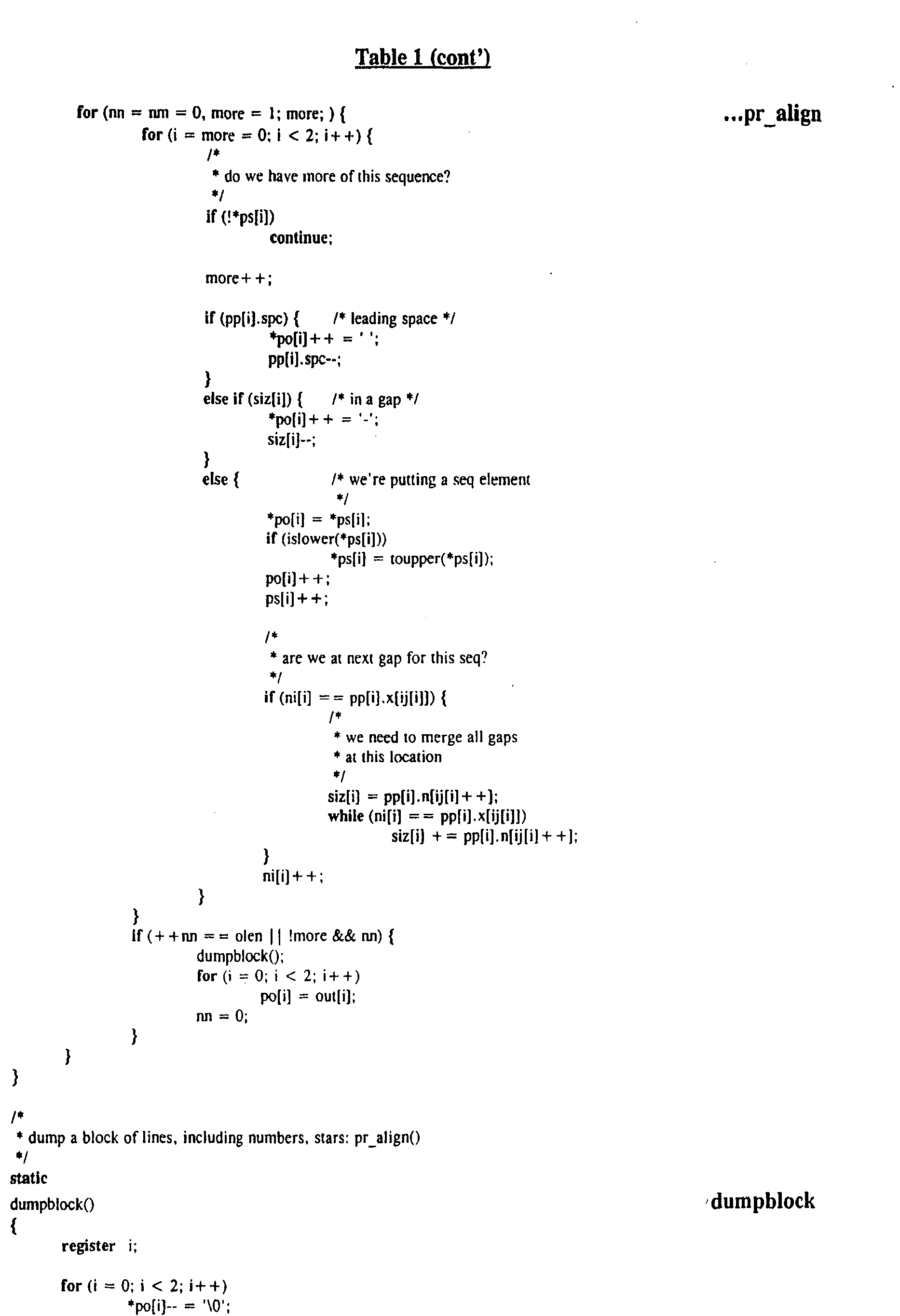

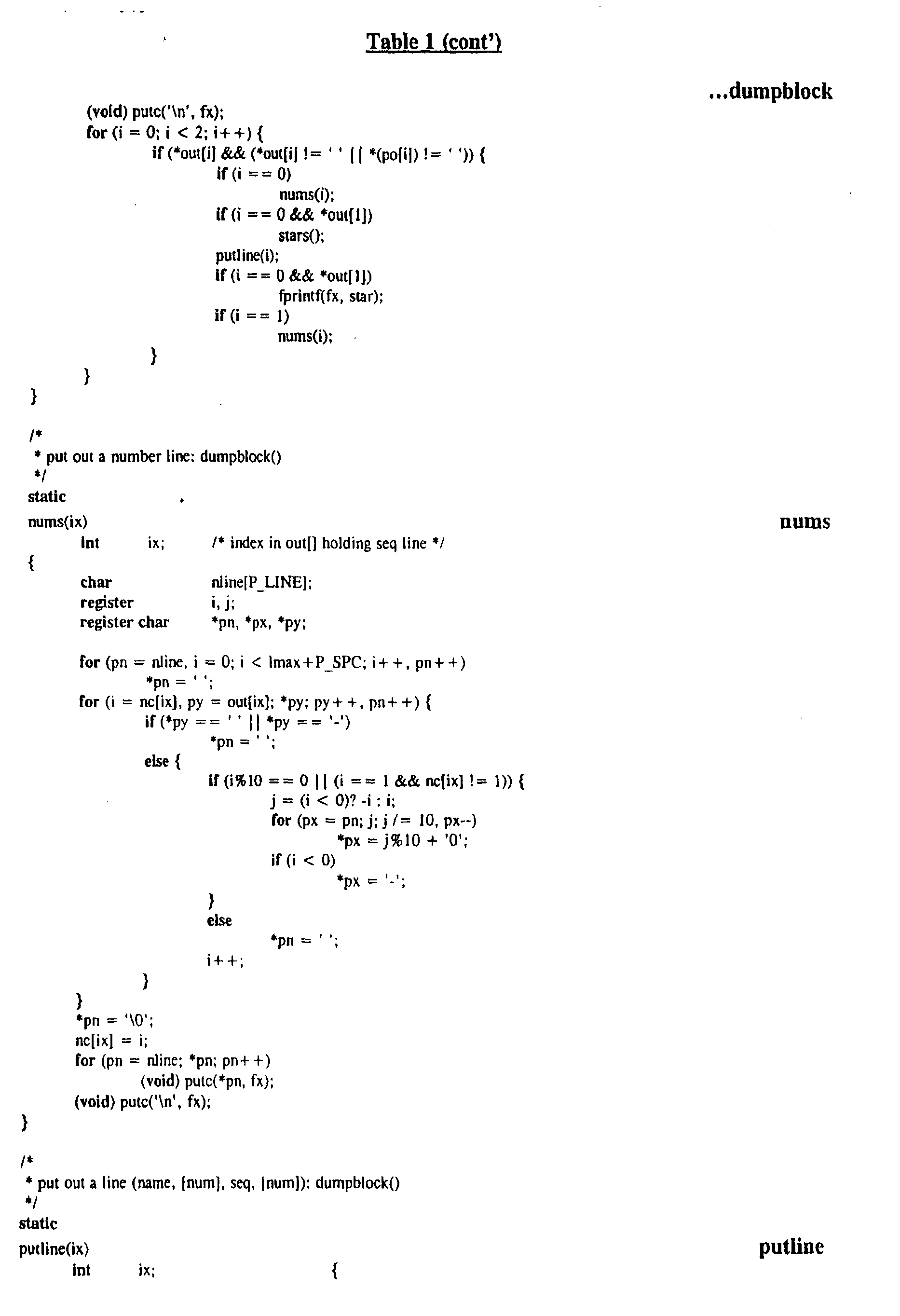

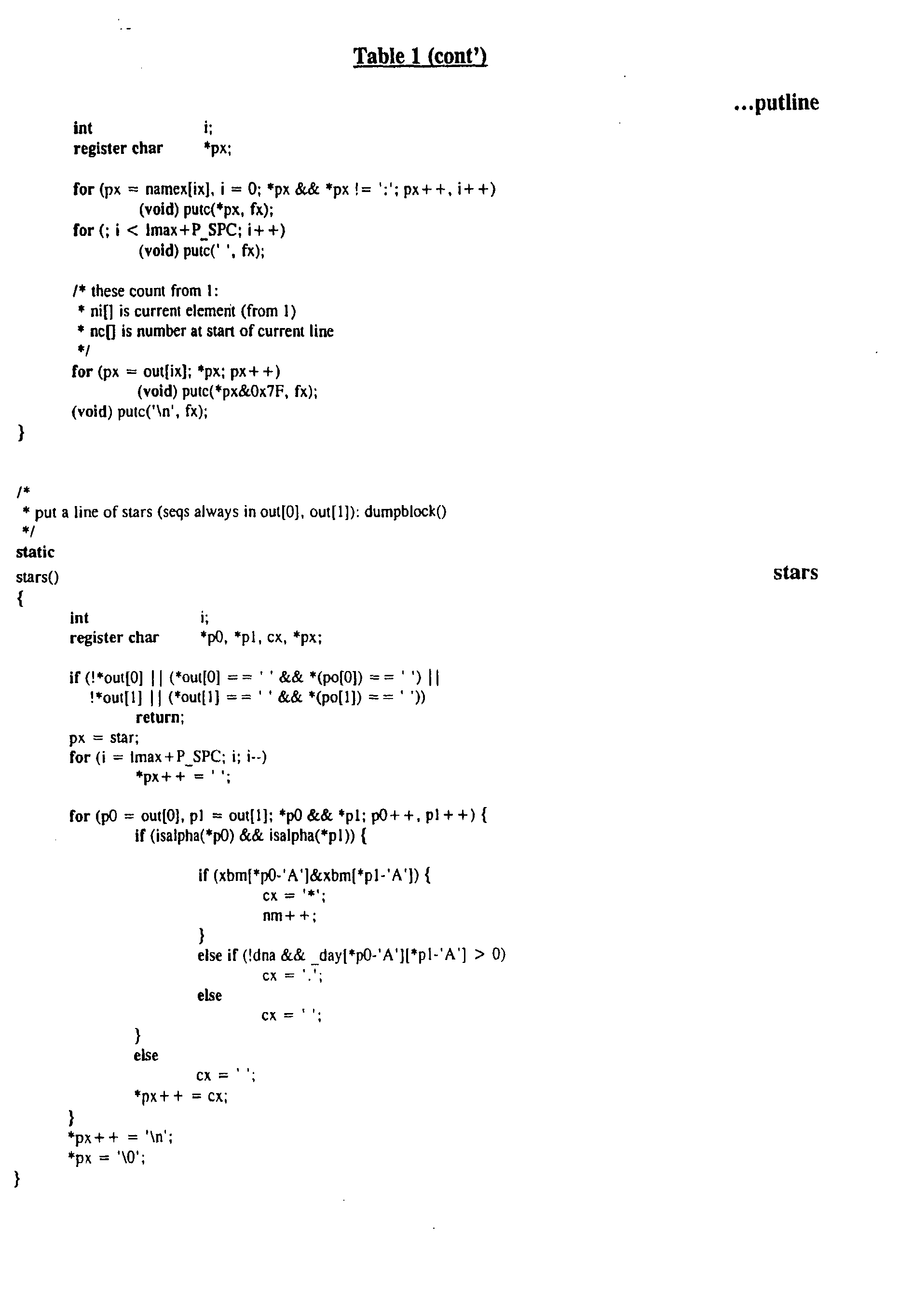

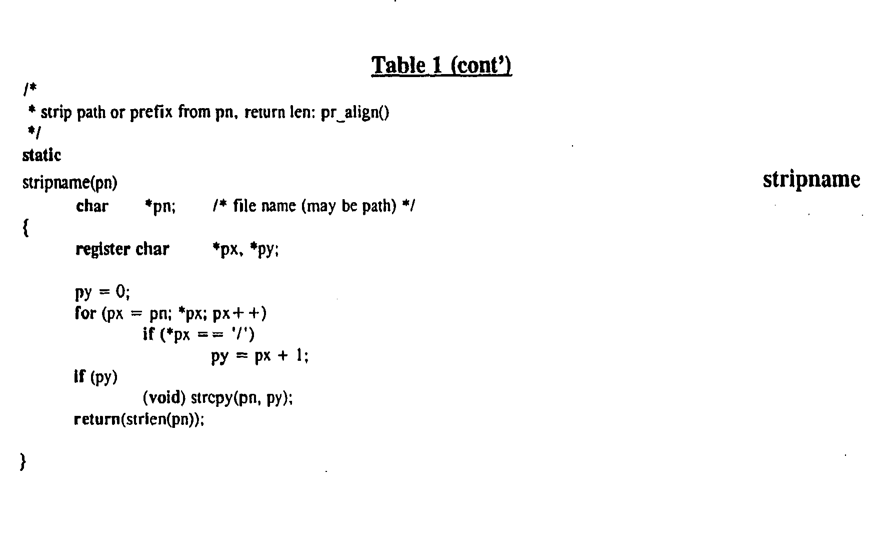

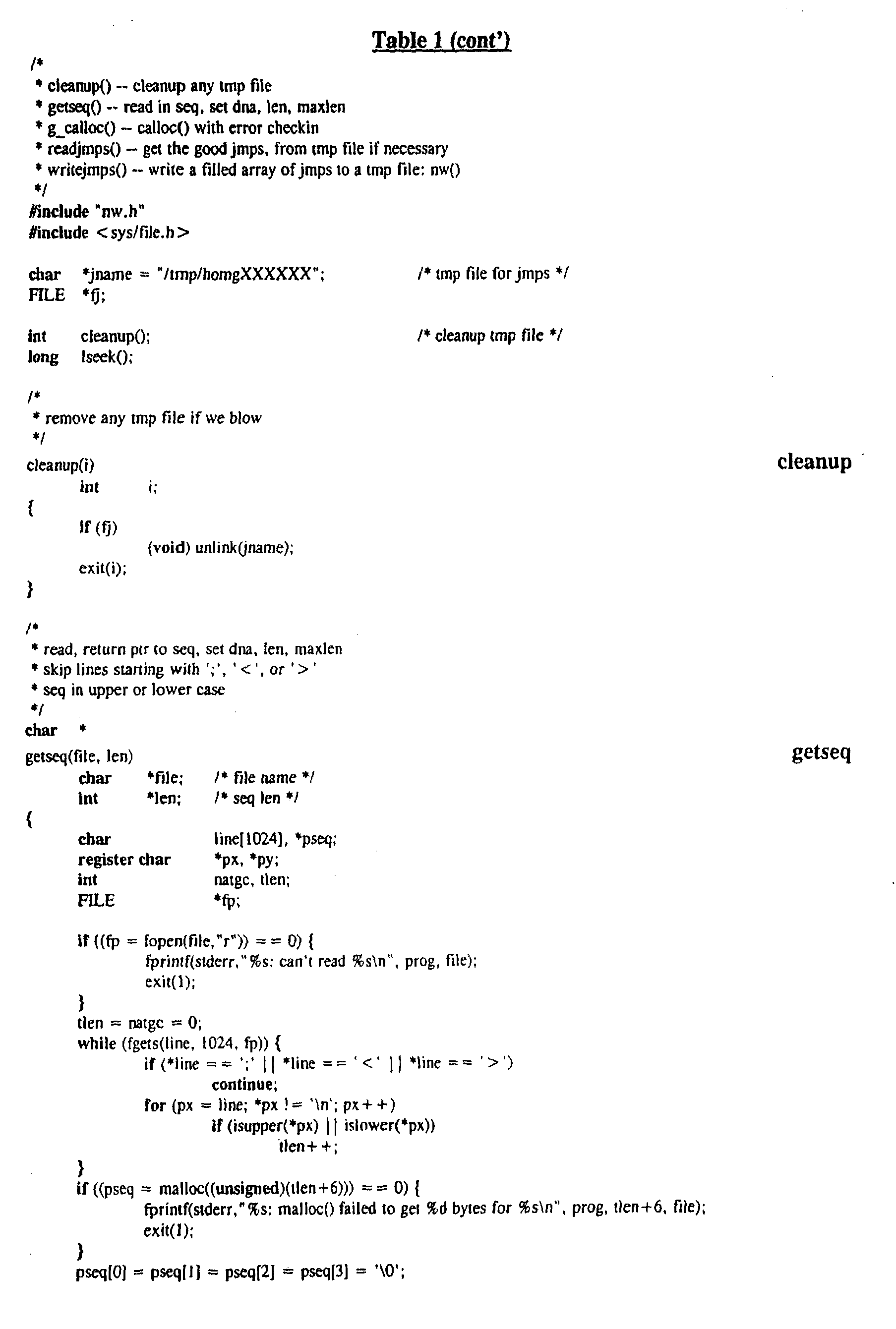

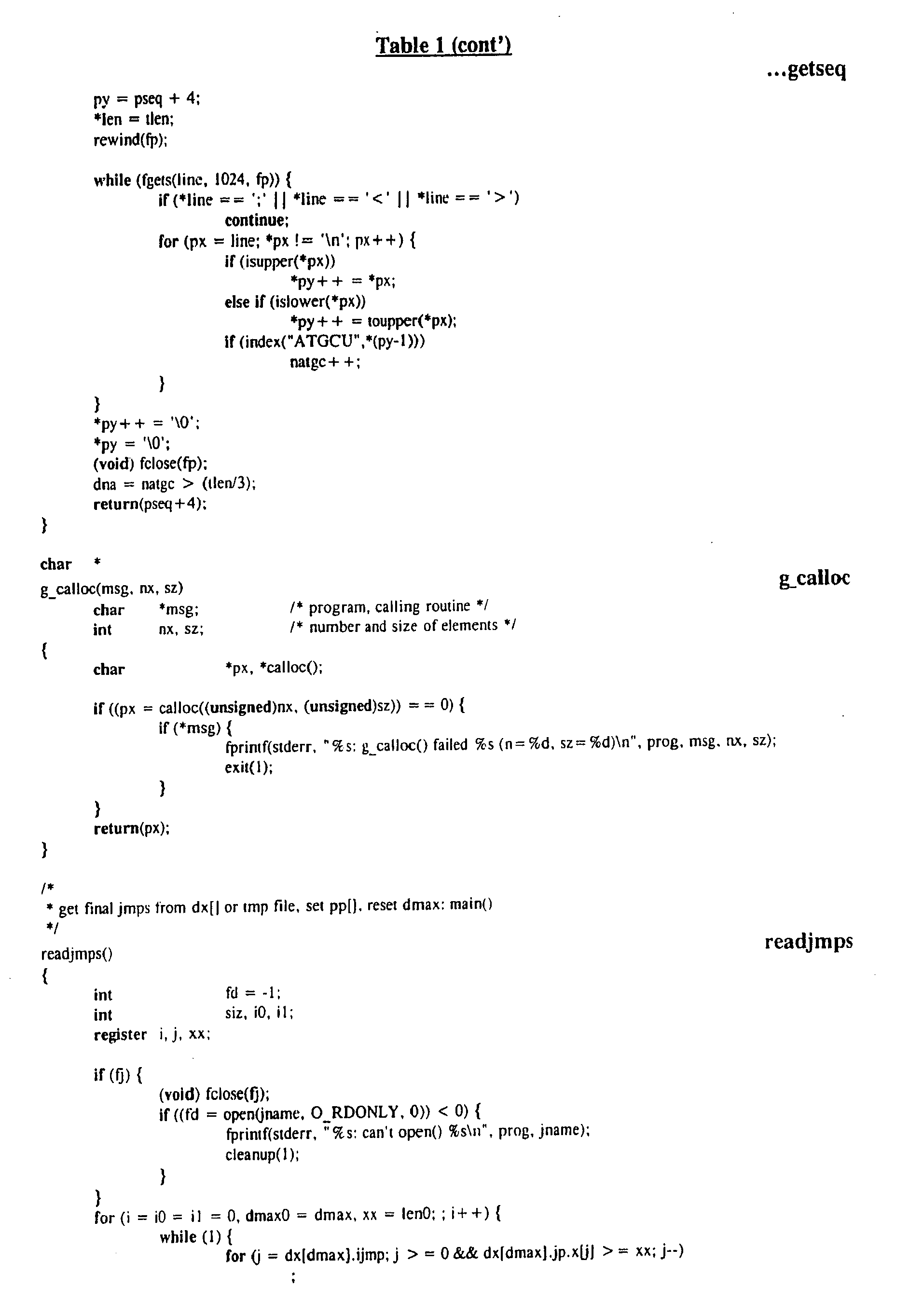

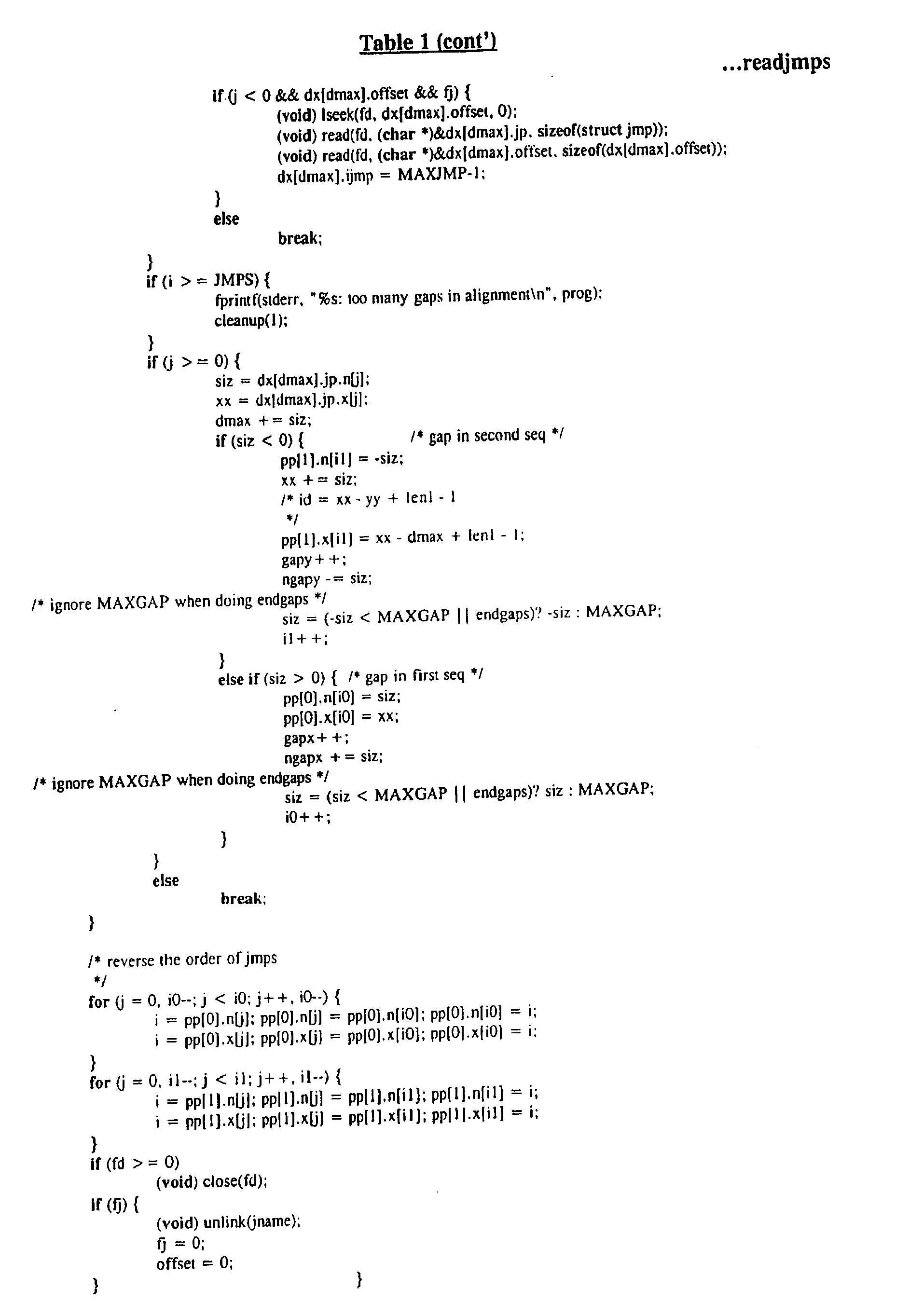

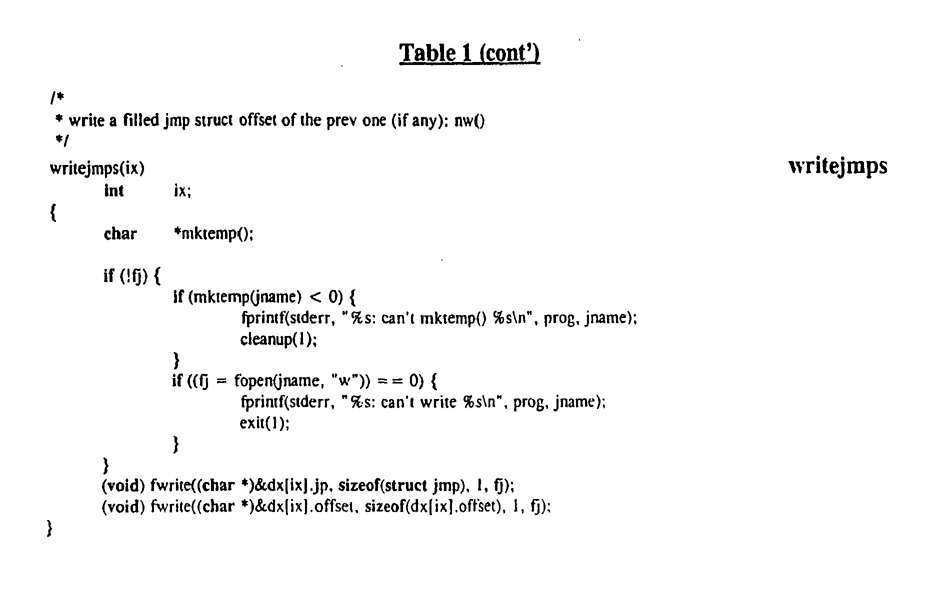

**Table 2**

| | | |
|---|---|---|
| PRO | XXXXXXXXXXXXXXX | (Length = 15 amino acids) |
| Comparison Protein | XXXXXYYYYYYY | (Length = 12 amino acids) |
| % amino acid sequence identity = | | |
| (the number of identically matching amino acid residues between the two polypeptide sequences as determined by ALIGN-2) divided by (the total number of amino acid residues of the PRO polypeptide) = | | |
| 5 divided by 15 =33.3% | | |

**Table 3**

| | | |
|---|---|---|
| PRO | XXXXXXXXXX | (Length = 10 amino acids) |
| Comparison Protein | XXXXXYYYYYYZZYZ | (Length = 15 amino acids) |
| % amino acid sequence identity = | | |
| (the number of identically matching amino acid residues between the two polypeptide sequences as determined by ALIGN-2) divided by (the total number of amino acid residues of the PRO polypeptide) = | | |
| 5 divided by 10 = 50% | | |

**Table 4**

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |
| % nucleic acid sequence identity = | | |
| (the number of identically matching nucleotides between the two nucleic acid sequences as determined by ALIGN-2) divided by (the total number of nucleotides of the PRO-DNA nucleic acid sequence) = | | |
| 6 divided by 14 = 42.9% | | |

**Table 5**

| | | |
|---|---|---|
| PRO-DNA | NNNNNNNNNNNN | (Length = 12 nucleotides) |
| Comparison DNA | NNNNLLLVV | (Length = 9 nucleotides) |
| % nucleic acid sequence identity = | | |
| (the number of identically matching nucleotides between the two nucleic acid sequences as determined by ALIGN-2) divided by (the total number of nucleotides of the PRO-DNA nucleic acid sequence) = | | |
| 4 divided by 12 = 33.3% | | |

### II. Compositions and Methods of the Invention

### A. Full-length PRO Polypeptides

The present invention provides newly identified and isolated nucleotide sequences encoding polypeptides referred to in the present application as PRO polypeptides. In particular, cDNAs encoding the PRO polypeptide has been identified and isolated, as disclosed in further detail in the Examples below.

As disclosed in the Examples below, cDNA clones encoding PRO polypeptides have been deposited with the ATCC. The actual nucleotide sequences of the clones can readily be determined by the skilled artisan by sequencing of the deposited clones using routine methods in the art. The predicted amino acid sequences can be determined from the nucleotide sequences using routine skill. For the PRO polypeptides and encoding nucleic acids described herein, Applicants have identified what is believed to be the reading frame best identifiable with the sequence information available at the time.

### B. PRO Variants

In addition to the full-length native sequence PRO polypeptides described herein, it is contemplated that PRO variants can be prepared. PRO variants can be prepared by introducing appropriate nucleotide changes into the PRO DNA, and/or by synthesis of the desired PRO polypeptide. Those skilled in the art will appreciate that amino acid changes may alter post-translational processes of the PRO polypeptide, such as changing the number or position of glycosylation sites or altering the membrane anchoring characteristics.

Variations in the native full-length sequence PRO polypeptide or in various domains of the PRO polypeptide described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO polypeptide that results in a change in the amino acid sequence of the PRO polypeptide as compared with the native sequence PRO polypeptide. Optionally the variation is by substitution of at least one amino acid with any other amino acid in one or more of the domains of the PRO polypeptide. Guidance in determining which amino acid residue may be inserted, substituted or deleted without adversely affecting the desired activity may be found by comparing the sequence of the PRO polypeptide with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, *i.e.,* conservative amino acid replacements. Insertions or deletions may optionally be in the range of about I to 5 amino acids. The variation allowed may be determined by systematically making insertions, deletions or substitutions of amino acids in the sequence and testing the resulting variants for activity exhibited by the full-length or mature native sequence.

PRO polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO polypeptide,

PRO fragments may be prepared by any of a number of conventional techniques. Desired peptide fragments may be chemically synthesized. An alternative approach involves generating PRO fragments by enzymatic digestion, *e.g.,* by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO polypeptide fragments share at least one biological and/or immunological activity with the native PRO polypeptide shown in the accompanying figures.

In particular embodiments, conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are introduced and the products screened.

**Table 6**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; | |
| | ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)|, cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

### C. Modifications of PRO Polypeptides

Covalent modifications of PRO polypeptides are included within the scope of this invention. One type of covalent modification includes reacting targeted amino acid residues of a PRO polypeptide with an organic derivatizing agent that is capable of reacting with selected side chains or the N- or C- terminal residues of the PRO polypeptide. Derivatization with bifunctional agents is useful, for instance, for crosslinking PRO polypeptides to a water-insoluble support matrix or surface for use in the method for purifying anti-PRO antibodies, and vice-versa. Commonly used crosslinking agents include, *e.g*., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate,

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO polypeptide included within the scope of this invention comprises altering the native glycosylation pattern of the polypeptide. "Altering the native glycosylation pattern" is intended for purposes herein to mean deleting one or more carbohydrate moieties found in native sequence PRO polypeptides (either by removing the underlying glycosylation site or by deleting the glycosylation by chemical and/or enzymatic means), and/or adding one or more glycosylation sites that are not present in the native sequence PRO polypeptide. In addition, the phrase includes qualitative changes in the glycosylation of the native proteins, involving a change in the nature and proportions of the various carbohydrate moieties present.

Addition of glycosylation sites to the PRO polypeptide may be accomplished by altering the amino acid sequence. The alteration may be made, for example, by the addition of, or substitution by, one or more serine or threonine residues to the native sequence PRO polypeptide (for O-linked glycosylation sites). The PRO polypeptide amino acid sequence may optionally be altered through changes at the DNA level, particularly by mutating the DNA encoding the PRO polypeptide at preselected bases such that codons are generated that will translate into the desired amino acids.

Another means of increasing the number of carbohydrate moieties on the PRO polypeptide is by chemical or enzymatic coupling of glycosides to the polypeptide. Such methods are described in the art, *e.g*., in WO 87/05330 published 11 September 1987, and in Aplin and Wriston. CRC Crit. Rev. Biochem., pp.259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO polypeptides comprises linking the PRO polypeptide to one of a variety of nonproteinaceous polymers, *e.g*., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO polypeptide of the present invention may also be modified in a way to form a chimeric molecule comprising a PRO polypeptide fused to another, heterologous polypeptide or amino acid sequence.

In one embodiment, such a chimeric molecule comprises a fusion of the PRO polypeptide with a tag polypeptide which provides an epitope to which an anti-tag antibody can selectively bind. The epitope tag is generally placed at the amino- or carboxyl- terminus of the PRO polypeptide. The presence of such epitope-tagged forms of the PRO polypeptide can be detected using an antibody against the tag polypeptide. Also, provision of the epitope tag enables the PRO polypeptide to be readily purified by affinity purification using an anti-tag antibody or another type of affinity matrix that binds to the epitope tag. Various tag polypeptides and their respective antibodies are well known in the art. Examples include poly-histidine (poly-His) or poly-histidine-glycine (poly-His-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto |Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [ Hopp et al., BioTechnology, 6:1204-1210(1988)] the KT3 epitope peptide [Martin et al., Science, 255:192-194 (1992)]; an α-tubulin epitope peptide [Skinner et al., J. Biol. Chem., 266: 15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO polypeptide with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO polypeptide in place of at least one variable region within an Ig molecule. In a particularly preferred embodiment, the immunoglobulin fusion includes the hinge, CH2 and CH3, or the hinge, CH1, CH2 and CH3 regions of an IgG I molecule. For the production of immunoglobulin fusions *see* also, U.S. Patent No. 5,428.130 issued June 27,1995.

### D. Preparation of PRO Polypeptides

The description below relates primarily to production of PRO polypeptides by culturing cells transformed or transfected with a vector containing PRO polypeptide nucleic acid. It is of course, contemplated that alternative methods, which are well known in the art, may be employed to prepare PROpolypeptides. For instance, the PRO polypeptide sequence, or portions thereof, may be produced by direct peptide synthesis using solid-phase techniques [*see, e.g.,* Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO polypeptide may be chemically synthesized separately and combined using chemical or enzymatic methods to produce the full-length PRO polypeptide.

### I. Isolation of DNA Encoding PRO Polypeptides

DNA encoding PRO polypeptides may be obtained from a cDNA library prepared from tissue believed to possess the PRO mRNA and to express it at a detectable level. Accordingly, human PRO DNA can be conveniently obtained from a cDNA library prepared from human tissue, such as described in the Examples. The PRO-encoding gene may also be obtained from a genomic library or by known synthetic procedures (*e.g*., automated nucleic acid synthesis).

Libraries can be screened with probes (such as antibodies to the PRO polypeptide or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding the PRO polypeptide is to use PCR methodology [Sambrook *et al., supra;* Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook *et al., supra.*

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook *et al., supra,* to detect precursors and processing intermediates of mRNA that may not have been reverse-transcribed into cDNA.

### 2. Selection and Transformation of Host Cells

Host cells are transfected or transformed with expression or cloning vectors described herein for PRO polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook *et al., supra*.

Methods of eukaryotic cell transfection and prokaryotic cell transformation are known to the ordinarily skilled artisan, for example, CaCl₂, CaPO₄, liposome-mediated and electroporation, Depending on the host cell used, transformation is performed using standard techniques appropriate to such cells. The calcium treatment employing calcium chloride, as described in Sambrook *et al., supra,* or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J, Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, *e.g.,* polybrene, polyornithine, may also be used. For various techniques for transforming mammalian cells, *see,* Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E*. *coli* X1776 (ATCC 31,537); *E*. *coli* strain W3110 (ATCC 27,325) and K5 772 (ATCC 53,635). Other suitable prokaryotic host cells include Enterobacteriaceae such as *Escherichia, e.g., E. coli, Enterobacter, Ervinia, Klebsiella, Proteus, Salmonella, e.g., Salmonella typhimurium*, *Serratia, e.g., Serratia marcescans,* and *Shigella,* as well as *Bacilli* such as *B. subtilis* and *B. licheniformis (e.g., B. licheniformis* 41P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA; E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169degP ompT kan'; E. coli* W3110 strain 37D6, which has the complete genotype *tonA ptr3 phoA E15 (argF-lac)169 degP ompT rbs7 ilvG kan'; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, *e.g.,* PCR or other nucleic acid polymerase reactions, are suitable.

In addition to prokaryotes, eukaryotic microbes such as filamentous fungi or yeast are suitable cloning or expression hosts for PRO-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943.529; Fleer et al., Bio/Technology, 9:968-975 (1991)) such as, *e.g., K. lactis* (MW98-8C, CBS683, CBS4574; Louvencourt et al., J. Bacteriol., 737 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28 :265-278 [1988]); *Candida: Trichoderma reesia* (EP 244,234); *Neurospora crassa* (Case et al., Proc. Natl. Acad. Sci. USA, 76:5259-5263 [1979]): *Schwanniomyces* such as *Schwanniomyces occidentalis* (EP 394,538 published 31 October 1990); and filamentous fungi such as, *e.g., Neurospora. Penicillium, Tolypocladium* (WO 91/00357 published 10 January 1991 and *Aspergillus* hosts such as *A. nidulans* (Ballance et al., Biochem. Biophys, Res. Commun., 112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia. Saccharomyces. Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PRO polypeptides are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV 1 line transformed by SV40 (COS-7, ATCC CRL 1651); human embryonic kidney line (293 or 293 cells subcloned for growth in suspension culture, Graham et al., J. Gen. Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod, 23:243-251 (1980)); human lung cells (W138, ATCC CCL 75); human liver cells (Hep G2, HB 8065); and mouse mammary tumor (MMT060562, ATCC CCL51). The selection of the appropriate host cell is deemed to be within the skill in the art.

### 3. Selection and Use of a Replicable Vector

The nucleic acid *(e.g.,* cDNA or genomic DNA) encoding PRO polypeptides may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO polypeptide may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO-encoding DNA that is inserted into the vector. The signal sequence may be a prokaryotic signal sequence selected, for example, from the group of the alkaline phosphatase, penicillinase, Ipp, or heat-stable enterotoxin II leaders. For yeast secretion the signal sequence may be, *e.g.,* the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2*µ* plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, *e.g*., ampicillin, neomycin, methotrexate, or tetracycline, (b) complement auxotrophic deficiencies, or (c) supply critical nutrients not available from complex media, *e.g.,* the gene encoding D-alanine racemase for *Bacilli.*

An example of suitable selectable markers for mammalian cells are those that enable the identification of cells competent to take up the PRO-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10: 157 (1980)]. The *trp* 1 gene provides a selection marker for a mutant strain of yeast lacking the ability to grow in tryptophan, for example, ATCC No. 44076 or PEP4-1 [Jones, Genetics, 85: 12 (1977)].

Expression and cloning vectors usually contain a promoter operably linked to the PRO-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding the PRO polypeptide.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg.,7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, *e.g*., the actin promoter or an immunoglobulin promoter, and from heat-shock promoters, provided such promoters are compatible with the host cell systems.

Transcription of a DNA encoding the PRO polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO polypeptide coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding the PRO polypeptide.

Still other methods, vectors, and host cells suitable for adaptation to the synthesis of PRO polypeptides in recombinant vertebrate cell culture are described in Gething et al., Nature, 293:620-625 (1981); Mantei et al., Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO polypeptide or against a synthetic peptide based on the DNA sequences provided herein or against exogenous sequence fused to PRO DNA and encoding a specific antibody epitope.

### 5. Purification of PRO Polypeptides

Forms of PRO polypeptides may be recovered from culture medium or from host cell lysates. If membrane-bound, it can be released from the membrane using a suitable detergent solution (*e.g*., Triton-X 100) or by enzymatic cleavage. Cells employed in expression of PRO polypeptides can be disrupted by various physical or chemical means, such as freeze-thaw cycling, sonication, mechanical disruption, or cell lysing agents.

It may be desired to purify PRO polypeptides from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO polypeptide. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO polypeptide produced.

### E. Antibodies

Some drug candidates for use in the compositions and methods of the present invention are antibodies and antibody fragments which mimic the biological activity of a PRO polypeptide.

### 1. Polyclonal Antibodies

Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### 2. Monoclonal Antibodies

The antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells,

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center. San Diego, California and the American Type Culture Collection. Manassas. Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against the PRO polypeptide. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (EUSA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, *supra*]*.* Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (*e.g*., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison *et al., supra*] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

### 3. Human and Humanized Antibodies

The antibodies of the invention may further comprise humanized antibodies or human antibodies. Humanized forms of non-human (*e.g.,* murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole *et al.,* and Boerner *et al.,* are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by the introducing of human immunoglobulin loci into transgenic animals, *e.g*., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5.569.825; 5,625,126; 5,633,425; 5,661,016, and in the following scientific publications: Marks et al., Bio/Technology, 10: 779-783 (1992); Lonberg et al., Nature, 368: 856-859 (1994); Morrison, Nature, 368: 812-13 (1994); Fishwild et al., Nature Biotechnology, 14:845-51 (1996): Neuberger, Nature Biotechnology, 14: 826 (1996): Lonberg and Huszar, Intern. Rev. Immunol., 13 :65-93 (1995).

### 4. Bispecific Antibodies

Bispecific antibodies are monoclonal, preferably human or humanized, antibodies that have binding specificities for at least two different antigens. In the present case, one of the binding specificities is for the PRO polypeptide, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies *see,* for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (*e.g*., tyrosine or tryptophan). Compensatory "cavities" of identical or similar size to the large side chain(s) are created on the interface of the second antibody molecule by replacing large amino acid side chains with smaller ones (*e.g*., alanine or threonine). This provides a mechanism for increasing the yield of the heterodimer over other unwanted end-products such as homodimers.

Bispecific antibodies can be prepared as full length antibodies or antibody fragments (*e.g*., F(ab')₂ bispecific antibodies). Techniques for generating bispecific antibodies from antibody fragments have been described in the literature. For example, bispecific antibodies can be prepared using chemical linkage. Brennan et al., Science, 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby et al., J. Exp. Med., 175:217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol., 148(5): 1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. *See,* Gruber et al., J. Immunol., 152:5368 (1994).

Antibodies with more than two valencies are contemplated. For example, trispecific antibodies can be prepared. Tutt et al., J. Immunol., 147:60 (1991).

Exemplary bispecific antibodies may bind to two different epitopes on a given PRO polypeptide herein. Alternatively, an anti-PRO polypeptide arm may be combined with an arm which binds to a triggering molecule on a leukocyte such as a T-cell receptor molecule (*e.g*., CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular PRO polypeptide. Bispecific antibodies may also be used to localize cytotoxic agents to cells which express a particular PRO polypeptide. These antibodies possess a PRO-binding arm and an arm which binds a cytotoxic agent or a radionuclide chelator, such as EOTUBE, DPTA, DOTA, or TETA. Another bispecific antibody of interest binds the PRO polypeptide and further binds tissue factor (TF).

### 5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676.980.

### 6. Effector Function Engineering

It may be desirable to modify the antibody of the invention with respect to effector function, so as to enhance, *e.g.,* the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). *See,* Caron et al., J. Exp. Med., 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff et al., Cancer Research, 53: 2560-2565 (1993). Alternatively, an antibody can be engineered that has dual Fc regions and may thereby have enhanced complement lysis and ADCC capabilities. *See*, Stevenson et al., Anti-Cancer Drug Design, 3: 219-230 (1989).

### 7. Immunoconjugates

The invention also pertains to immunoconjugates comprising an antibody conjugated to a cytotoxic agent such as a chemotherapeutic agent, toxin (*e.g*., an enzymatically active toxin of bacterial, fungal, plant, or animal origin, or fragments thereof), or a radioactive isotope (i.e., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa),* ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³⁵In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazoniumderivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098(1987). Carbon-14-labeled 1-isothiocyanatobenzyl-3-methyldiethylene triaminepentaacetic acid (MX-DTPA) is an exemplary chelating agent for conjugation of radionucleotide to the antibody. *See,* WO94/11026.

In another embodiment, the antibody may be conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (*e.g.*, avidin) that is conjugated to a cytotoxic agent (*e.g*.. a radionucleotide).

### 8. Immunoliposomes

The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl. Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem., 257: 286-288 (1982) via a disulfide-interchange reaction. A chemotherapeutic agent (such as Doxorubicin) is optionally contained within the liposome. *See*, Gabizon et al., J. National Cancer Inst., 81(19): 1484 (1989).

### F. Identification of Proteins Capable of Inhibiting Neoplastic Cell Growth or Proliferation

The proteins disclosed in the present application have been assayed in a panel of 60 tumor cell lines currently used in the investigational, disease-oriented, *in vitro* drug-discovery screen of the National Cancer Institute (NCI). The purpose of this screen is to identify molecules that have cytotoxic and/or cytostatic activity against different types of tumors. NCI screens more than 10,000 new molecules per year (Monks et al., J. Natl. Cancer Inst., 83:757-766 (1991); Boyd, Cancer: Princ. Pract. Oncol. Update, 3(10):1-12 ([1989]). The tumor cell lines employed in this study have been described in Monks *et al., supra.* The cell lines the growth of which has been significantly inhibited by the proteins of the present application are specified in the Examples.

The results have shown that the proteins tested show cytostatic and, in some instances and concentrations, cytotoxic activities in a variety of cancer cell lines, and therefore are useful candidates for tumor therapy.

Other cell-based assays and animal models for tumors (*e.g.*, cancers) can also be used to verify the findings of the NCI cancer screen, and to further understand the relationship between the protein identified herein and the development and pathogenesis of neoplastic cell growth. For example, primary cultures derived from tumors in transgenic animals (as described below) can be used in the cell-based assays herein, although stable cell lines are preferred. Techniques to derive continuous cell lines from transgenic animals are well known in the art *(see, e.g.,* Small et al., Mol. Cell, Biol., 5:642-648 [1985]).

### G. Animal Models

A variety of well known animal models can be used to further understand the role of the molecules identified herein in the development and pathogenesis of tumors, and to test the efficacy of candidate therapeutic agents, including antibodies, and other agonists of the native polypeptides, including small molecule agonists. The *in vivo* nature of such models makes them particularly predictive of responses in human patients. Animal models of tumors and cancers (*e.g*., breast cancer, colon cancer, prostate cancer, lung cancer, etc.) include both non-recombinant and recombinant (transgenic) animals. Non-recombinant animal models include, for example, rodent. *e.g*., murine models. Such models can be generated by introducing tumor cells into syngeneic mice using standard techniques, *e.g*., subcutaneous injection, tail vein injection, spleen implantation, intraperitoneal implantation, implantation under the renal capsule, or orthopin implantation, *e.g*., colon cancer cells implanted in colonic tissue. *(See, e.g.,* PCT publication No. WO 97/33551, published September 18, 1997).

Probably the most often used animal species in oncological studies are immunodeficient mice and, in particular, nude mice. The observation that the nude mouse with hypo/aplasia could successfully act as a host for human tumor xenografts has lead to its widespread use for this purpose. The autosomal recessive *nu* gene has been introduced into a very large number of distinct congenic strains of nude mouse, including, for example, ASW, A/He, AKR, BALB/c. B I O.LP, 07. C3H, C57BL, C57, CBA, DBA, DDD, list, NC. NFR, NFS. NFS/N, NZB. NZC, NZW, P, RIII and SJL. In addition, a wide variety of other animals with inherited immunological defects other than the nude mouse have been bred and used as recipients of tumor xenografts. For further details *see, e.g*., The Nude Mouse in Oncology Research, E. Boven and B. Winograd, eds., CRC Press, Inc., 1991.

The cells introduced into such animals can be derived from known tumor/cancer cell lines, such as, any of the above-listed tumor cell lines, and, for example, the B104-1-1 cell line (stable NIH-3T3 cell line transfected with the *neu* protooncogene); ras-transfected NIH-3T3 cells; Caco-2 (ATCC HTB-37); a moderately well-differentiated grade II human colon adenocarcinoma cell line, HT-29 (ATCC HTB-38), or from tumors and cancers. Samples of tumor or cancer cells can be obtained from patients undergoing surgery, using standard conditions, involving freezing and storing in liquid nitrogen (Karmali et al., Br. J. Cancer, 48:689-696 [1983]).

Tumor cells can be introduced into animals, such as nude mice, by a variety of procedures. The subcutaneous (s.c.) space in mice is very suitable for tumor implantation. Tumors can be transplanted s.c. as solid blocks, as needle biopsies by use of a trochar, or as cell suspensions. For solid block or trochar implantation, tumor tissue fragments of suitable size are introduced into the s.c. space. Cell suspensions are freshly prepared from primary tumors or stable tumor cell lines, and injected subcutaneously. Tumor cells can also be injected as subdermal implants. In this location, the inoculum is deposited between the lower part of the dermal connective tissue and the s.c. tissue. Boven and Winograd (1991), *supra.* Animal models of breast cancer can be generated, for example, by implanting rat neuroblastoma cells (from which the *neu* oncogen was initially isolated), or *neu-*transformed NIH-3T3 cells into nude mice, essentially as described by Drebin et al., Proc. Natl. Acad. Sci. USA, 83:9129-9133 (1986).

Similarly, animal models of colon cancer can be generated by passaging colon cancer cells in animals, *e.g.,* nude mice, leading to the appearance of tumors in these animals. An orthotopic transplant model of human colon cancer in nude mice has been described, for example, by Wang et al., Cancer Research, 54:4726-4728 (1994) and Too et al., Cancer Research, 55:681-684 (1995). This model is based on the so-called "METAMOUSE™" sold by AntiCancer, Inc., (San Diego, California).

Tumors that arise in animals can be removed and cultured *in vitro.* Cells from the *in vitro* cultures can then be passaged to animals. Such tumors can serve as targets for further testing or drug screening. Alternatively, the tumors resulting from the passage can be isolated and RNA from pre-passage cells and cells isolated after one or more rounds of passage analyzed for differential expression of genes of interest. Such passaging techniques can be performed with any known tumor or cancer cell lines.

For example, Meth A. CMS4, CMS5. CMS21, and WEHI-164 are chemically induced fibrosarcomas of BALB/c female mice (DeLen et al., J. Exp. Med., 146:720 [1977]), which provide a highly controllable model system for studying the anti-tumor activities of various agents (Palladino et al., J. Immunol., 138:4023-4032 [1987]). Briefly, tumor cells are propagated *in vitro* in cell culture. Prior to injection into the animals, the cell lines are washed and suspended in buffer, at a cell density of about 10x10⁶ to 10x10⁷ cells/ml. The animals are then infected subcutaneously with 10 to 100 µl of the cell suspension, allowing one to three weeks for a tumor to appear.

In addition, the Lewis lung (3LL) carcinoma of mice, which is one of the most thoroughly studied experimental tumors, can be used as an investigational tumor model. Efficacy in this tumor model has been correlated with beneficial effects in the treatment of human patients diagnosed with small cell carcinoma of the lung (SCCL). This tumor can be introduced in normal mice upon injection of tumor fragments from an affected mouse or of cells maintained in culture (Zupi et al., Br. J. Cancer, 41, suppl. 4:309 [1980]), and evidence indicates that tumors can be started from injection of even a single cell and that a very high proportion of infected tumor cells survive. For further information about this tumor model *see,* Zacharski, Haemostasis, 16:300-320 (1986).

One way of evaluating the efficacy of a test compound in an animal model on an implanted tumor is to measure the size of the tumor before and after treatment. Traditionally, the size of implanted tumors has been measured with a slide caliper in two or three dimensions. The measure limited to two dimensions does not accurately reflect the size of the tumor, therefore, it is usually converted into the corresponding volume by using a mathematical formula. However, the measurement of tumor size is very inaccurate. The therapeutic effects of a drug candidate can be better described as treatment-induced growth delay and specific growth delay. Another important variable in the description of tumor growth is the tumor volume doubling time. Computer programs for the calculation and description of tumor growth are also available, such as the program reported by Rygaard and Spang-Thomsen, Proc. 6th Int. Workshop on Immune-Deficient Animals. Wu and Sheng eds., Basel, 1989, 301. It is noted, however, that necrosis and inflammatory responses following treatment may actually result in an increase in tumor size, at least initially. Therefore, these changes need to be carefully monitored, by a combination of a morphometric method and flow cytometric analysis.

Recombinant (transgenic) animal models can be engineered by introducing the coding portion of the genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, *e.g*., baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (*e.g.,* Van der Putten et al., Proc. Natl. Acad. Sci. USA, 82:6148-615 [1985]); gene targeting in embryonic stem cells (Thompson et al., Cell, 56:313-321 [1989]); electroporation of embryos (Lo, Mol. Cell. Biol., 3:1803-1814 [1983]); sperm-mediated gene transfer (Lavitrano et al., Cell, 57:717-73 [1989]). For review, *see*, for example, U.S. Patent No. 4,736,866.

For the purpose of the present invention, transgenic animals include those that carry the transgene only in part of their cells ("mosaic animals"). The transgene can be integrated either as a single transgene, or in concatamers, *e.g.*, head-to-head or head-to-tail tandems. Selective introduction of a transgene into a particular cell type is also possible by following, for example, the technique of Lasko et al., Proc. Natl. Acad. Sci. USA, 89:6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry. The animals are further examined for signs of tumor or cancer development.

The efficacyof antibodies specifically binding the polypeptides identified herein and other drug candidates, can be tested also in the treatment of spontaneous animal tumors. A suitable target for such studies is the feline oral squamous cell carcinoma (SCC). Feline oral SCC is a highly invasive, malignant tumor that is the most common oral malignancy of cats, accounting for over 60% of the oral tumors reported in this species. It rarely metastasizes to distant sites, although this low incidence of metastasis may merely be a reflection of the short survival times for cats with this tumor. These tumors are usually not amenable to surgery, primarily because of the anatomy of the feline oral cavity. At present, there is no effective treatment for this tumor. Prior to entry into the study, each cat undergoes complete clinical examination, biopsy, and is scanned by computed tomography (CT). Cats diagnosed with sublingual oral squamous cell tumors are excluded from the study. The tongue can become paralyzed as a result of such tumor, and even if the treatment kills the tumor, the animals may not be able to feed themselves. Each cat is treated repeatedly, over a longer period of time. Photographs of the tumors will be taken daily during the treatment period, and at each subsequent recheck. After treatment, each cat undergoes another CT scan. CT scans and thoracic radiograms are evaluated every 8 weeks thereafter. The data are evaluated for differences in survival, response and toxicity as compared to control groups. Positive response may require evidence of tumor regression, preferably with improvement of quality of life and/or increased life span.

In addition, other spontaneous animal tumors, such as fibrosarcoma, adenocarcinoma, lymphoma, chrondroma, leiomyosarcoma of dogs, cats, and baboons can also be tested. Of these mammary adenocarcinoma in dogs and cats is a preferred model as its appearance and behavior are very similar to those in humans. However, the use of this model is limited by the rare occurrence of this type of tumor in animals.

### H. Screening Assays for Drug Candidates

Screening assays for drug candidates are designed to identify compounds that competitively bind or complex with the receptor(s) of the polypeptides identified herein, or otherwise signal through such receptor(s). Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art.

In binding assays, the interaction is binding and the complex formed can be isolated or detected in the reaction mixture. In a particular embodiment, a receptor of a polypeptide encoded by the gene identified herein or the drug candidate is immobilized on a solid phase, *e.g.,* on a microtiter plate, by covalent or non-covalent attachments. Non-covalent attachment generally is accomplished by coating the solid surface with a solution of the polypeptide and drying. Alternatively, an immobilized antibody, *e.g.,* a monoclonal antibody, specific for the polypeptide to be immobilized can be used to anchor it to a solid surface. The assay is performed by adding the non-immobilized component, which may be labeled by a detectable label, to the immobilized component, *e.g*., the coated surface containing the anchored component. When the reaction is complete, the non-reacted components are removed, *e.g*., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular receptor, its interaction with that polypeptide can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers [Fields and Song, Nature (London), 340:245-246 (1989); Chien et al., Proc. Natl. Acad. Sci. USA, 88:9578-9582 (1991)] as disclosed by Chevray and Nathans [Proc. Natl. Acad. Sci. USA, 89:5789-5793 (1991)]. Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GALA, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

### I. Pharmaceutical Compositions

The polypeptides of the present invention, agonist antibodies specifically binding proteins identified herein, as well as other molecules identified by the screening assays disclosed herein, can be administered for the treatment of tumors, including cancers, in the form of pharmaceutical compositions.

Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology *(see. e.g.,* Marasco et al., Proc. Natl. Acad. Sci. USA, 90:7889-7893 [1993]).

The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

Therapeutic formulations of the polypeptides identified herein, or agonists thereof are prepared for storage by mixing the active ingredient having the desired degree of purity with optional pharmaceutically acceptable carriers, excipients or stabilizers *(*Remington's Pharmaceutical Sciences. 16th edition, Osol, A. ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine, preservatives (such as octadecyldimethylbenzyl ammonium chloride; hexamethonium chloride: benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.,* Zn-protein complexes); and/or nonionic surfactants such as TWEEN™, PLURONICS™ or polyethylene glycol (PEG).

The formulation herein may also contain more than one active compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. ed. (1980).

The formulations to be used for *in vivo* administration must be sterile. This is readily accomplished by filtration through sterile filtration membranes, prior to or following lyophilization and reconstitution.

Therapeutic compositions herein generally are placed into a container having a sterile access port, for example, an intravenous solution bag or vial having a stopper pierceable by a hypodermic injection needle.

Sustained-release preparations may be prepared. Suitable examples of sustained-release preparations include semipermeable matrices of solid hydrophobic polymers containing the antibody, which matrices are in the form of shaped articles, *e.g.,* films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### J. Methods of Treatment

It is contemplated that the polypeptides of the present invention and their agonists, including antibodies, peptides, and small molecule agonists, may be used to treat various tumors, *e.g*., cancers. Exemplary conditions or disorders to be treated include benign or malignant tumors (*e.g*.. renal, liver, kidney, bladder, breast, gastric, ovarian, colorectal, prostate, pancreatic, lung, vulval, thyroid, hepatic carcinomas; sarcomas; glioblastomas; and various head and neck tumors); leukemias and lymphoid malignancies; other disorders such as neuronal, glial, astrocytal, hypothalamic and other glandular, macrophagal, epithelial, stromal and blastocoelic disorders; and inflammatory, angiogenic and immunologic disorders. The anti-tumor agents of the present invention (including the polypeptides disclosed herein and agonists which mimic their activity, *e.g*., antibodies, peptides and small organic molecules), are administered to a mammal, preferably a human, in accord with known methods, such as intravenous administration as a bolus or by continuous infusion over a period of time, or by intramuscular, intraperitoneal, intracerobrospinal, intraocular, intraarterial, intralesional, subcutaneous, intraarticular, intrasynovial, intrathecal, oral, topical, or inhalation routes.

Other therapeutic regimens may be combined with the administration of the anti-cancer agents of the instant invention. For example, the patient to be treated with such anti-cancer agents may also receive radiation therapy. Alternatively, or in addition, a chemotherapeutic agent may be administered to the patient. Preparation and dosing schedules for such chemotherapeutic agents may be used according to manufacturers' instructions or as determined empirically by the skilled practitioner. Preparation and dosing schedules for such chemotherapy are also described in Chemotherapy Service, ed., M.C. Perry, Williams & Wilkins, Baltimore, MD (1992). The chemotherapeutic agent may precede, or follow administration of the anti-tumor agent of the present invention, or may be given simultaneously therewith. The anti-cancer agents of the present invention may be combined with an anti-oestrogen compound such as tamoxifen or an anti-progesterone such as onapristone *(see,* EP 616812) in dosages known for such molecules.

It may be desirable to also administer antibodies against tumor associated antigens, such as antibodies which bind to the ErbB2, EGFR, ErbB3, ErbB4, or vascular endothelial factor (VEGF). Alternatively, or in addition, two or more antibodies binding the same or two or more different cancer-associated antigens may be co-administered to the patient. Sometimes, it may be beneficial to also administer one or more cytokines to the patient. In a preferred embodiment, the anti-cancer agents herein are co-administered with a growth inhibitory agent. For example, the growth inhibitory agent may be administered first, followed by the administration of an anti-cancer agent of the present invention. However, simultaneous administration or administration of the anti-cancer agent of the present invention first is also contemplated. Suitable dosages for the growth inhibitory agent are those presently used and may be lowered due to the combined action (synergy) of the growth inhibitory agent and the antibody herein.

For the prevention or treatment of disease, the appropriate dosage of an anti-tumor agent herein will depend on the type of disease to be treated, as defined above, the severity and course of the disease, whether the agent is administered for preventive or therapeutic purposes, previous therapy, the patient's clinical history and response to the agent, and the discretion of the attending physician. The agent is suitably administered to the patient at one time or over a series of treatments. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" in Toxicokinetics and New Drug Development, Yacobi et al., eds., Pergamon Press, New York 1989, pp. 42-96.

For example, depending on the type and severity of the disease, about 1 µg/kg to 15 mg/kg (e.g., 0.1-20 mg/kg) of an antitumor agent is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays. Guidance as to particular dosages and methods of delivery is provided in the literature; *see*, for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

### K. Articles of Manufacture

In another embodiment of the invention, an article of manufacture containing materials useful for the diagnosis or treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is an anti-tumor agent of the present invention. The label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

The following examples are offered for illustrative purposes only, and are not intended to limit the scope of the present invention in any way.

All patent and literature references cited in the present specification are hereby incorporated by reference in their entirety.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE 1

### Extracellular Domain Homology Screening to Identify Novel Polypeptides and cDNA Encoding Therefor

The extracellular domain (ECD) sequences (including the secretion signal sequence, if any) from about 950 known secreted proteins from the Swiss-Prot public database were used to search EST databases. The EST databases included public databases (*e.g*., Dayhoff, GenBank), and proprietary databases (*e.g*. LIFESEQ^{®}, Incyte Pharmaceuticals, Palo Alto, CA). The search was performed using the computer program BLAST or BLAST-2 (Altschul et al., Methods in Enzymology, 266:460-480 (1996)) as a comparison of the ECD protein sequences to a 6 frame translation of the EST sequences. Those comparisons with a BLAST score of 70 (or in some cases, 90) or greater that did not encode known proteins were clustered and assembled into consensus DNA sequences with the program "phrap" (Phil Green, University of Washington, Seattle, WA).

Using this extracellular domain homology screen, consensus DNA sequences were assembled relative to the other identified EST sequences using phrap. In addition, the consensus DNA sequences obtained were often (but not always) extended using repeated cycles of BLAST or BLAST-2 and phrap to extend the consensus sequence as far as possible using the sources of EST sequences discussed above.

Based upon the consensus sequences obtained as described above, oligonucleotides were then synthesized and used to identify by PCR a cDNA library that contained the sequence of interest and for use as probes to isolate a clone of the full-length coding sequence for a PRO polypeptide. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5 kbp. In order to screen several libraries for a full-length clone, DNA from the libraries was screened by PCR amplification, as per Ausubel et al., Current Protocols in Molecular Biology, with the PCR primer pair. A positive library was then used to isolate clones encoding the gene of interest using the probe oligonucleotide and one of the primer pairs.

The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253:1278-1280 (1991)) in the unique XhoI and NotI sites.

### EXAMPLE 2

### Isolation of cDNA Clones by Amylase Screening

### 1. Preparation of oligo dT primed cDNA library

mRNA was isolated from a human tissue of interest using reagents and protocols from Invitrogen, San Diego, CA (Fast Track 2). This RNA was used to generate an oligo dT primed cDNA library in the vector pRK5D using reagents and protocols from Life Technologies, Gaithersburg, MD (Super Script Plasmid System). In this procedure, the double stranded cDNA was sized to greater than 1000 bp and the SalI/NotI linkered cDNA was cloned into XhoI/NotI cleaved vector. pRK5D is a cloning vector that has an sp6 transcription initiation site followed by an SfiI restriction enzyme site preceding the XhoI/NotI cDNA cloning sites.

### 2. Preparation of random primed cDNA library

A secondary cDNA library was generated in order to preferentially represent the 5' ends of the primary cDNA clones. Sp6 RNA was generated from the primary library (described above), and this RNA was used to generate a random primed cDNA library in the vector pSST-AMY.0 using reagents and protocols from Life Technologies (Super Script Plasmid System, referenced above). In this procedure the double stranded cDNA was sized to 500-1000 bp, linkered with blunt to NotI adaptors, cleaved with SfiI, and cloned into SfiI/NotI cleaved vector. pSST-AMY.0 is a cloning vector that has a yeast alcohol dehydrogenase promoter preceding the cDNA cloning sites and the mouse amylase sequence (the mature sequence without the secretion signal) followed by the yeast alcohol dehydrogenase terminator, after the cloning sites. Thus, cDNAs cloned into this vector that are fused in frame with amylase sequence will lead to the secretion of amylase from appropriately transfected yeast colonies.

### 3. Transformation and Detection

DNA from the library described in paragraph 2 above was chilled on ice to which was added electrocompetent DH10B bacteria (Life Technologies, 20 ml). The bacteria and vector mixture was then electroporated as recommended by the manufacturer. Subsequently, SOC media (Life Technologies, 1 ml) was added and the mixture was incubated at 37°C for 30 minutes. The transformants were then plated onto 20 standard 150 mm LB plates containing ampicillin and incubated for 16 hours (37°C). Positive colonies were scraped off the plates and the DNA was isolated from the bacterial pellet using standard protocols, *e.g*., CsCl-gradient. The purified DNA was then carried on to the yeast protocols below.

The yeast methods were divided into three categories: (1) Transformation of yeast with the plasmid/cDNA combined vector; (2) Detection and isolation of yeast clones secreting amylase; and (3) PCR amplification of the insert directly from the yeast colony and purification of the DNA for sequencing and further analysis.

The yeast strain used was HD56-5A (ATCC-90785). This strain has the following genotype: MAT alpha, ura3-52, leu2-3, leu2-112, his3-11. his3-15, MAL⁺, SUC⁺, GAL⁺. Preferably, yeast mutants can be employed that have deficient post-translational pathways. Such mutants may have translocation deficient alleles in *sec*71, *sec* 72*, sec*62, with truncated *sec*-71 being most preferred. Alternatively, antagonists (including antisense nucleotides and/or ligands) which interfere with the normal operation of these genes, other proteins implicated in this post translation pathway (*e.g*., SEC61p, SEC72p, SEC62p, SEC63p, TDJ1p or SSA1p-4p) or the complex formation of these proteins may also be preferably employed in combination with the amylase-expressing yeast.

Transformation was performed based on the protocol outlined by Gietz et al., Nucl. Acid. Res., 20:1425 (1992). Transformed cells were then inoculated from agar into YEPD complex media broth (100 ml) and grown overnight at 30°C. The YEPD broth was prepared as described in Kaiser et al., Methods in Yeast Genetics, Cold Spring Harbor Press, Cold Spring Harbor, NY, p. 207 (1994). The overnight culture was then diluted to about 2 x 10⁶ cells/ml (approx. OD₆₀₀=0.1) into fresh YEPD broth (500 ml) and regrown to 1 x 10⁷ cells/ml (approx. OD₆₀₀=0.4-0.5).

The cells were then harvested and prepared for transformation by transfer into GS3 rotor bottles in a Sorval GS3 rotor at 5,000 rpm for 5 minutes, the supernatant discarded, and then resuspended into sterile water, and centrifuged again in 50 ml falcon tubes at 3,500 rpm in a Beckman GS-6KR centrifuge. The supernatant was discarded and the cells were subsequently washed with LiAc/TE (10 ml, 10 mM Tris-HCl, 1 mM EDTA pH 7.5, 100 mM Li₂OOCCH₃), and resuspended into LiAc/TE (2.5 ml).

Transformation took place by mixing the prepared cells (100 µl) with freshly denatured single stranded salmon testes DNA (Lofstrand Labs, Gaithersburg, MD) and transforming DNA (1 µg, vol. < 10 µl) in microfuge tubes. The mixture was mixed briefly by vortexing, then 40% PEG/TE (600 µl, 40% polyethylene glycol-4000, 10 mM Tris-HCl. 1 mM EDTA, 100 mM Li₂OOCCH_{3,} pH 7.5) was added. This mixture was gently mixed and incubated at 30°C while agitating for 30 minutes. The cells were then heat shocked at 42°C for 15 minutes, and the reaction vessel centrifuged in a microfuge at 12,000 rpm for 5-10 seconds, decanted and resuspended into TE (500 µl, 10 mM Tris-HCl, I mM EDTA pH 7.5) followed by recentrifugation. The cells were then diluted into TE (1 ml) and aliquots (200 µl) were spread onto the selective media previously prepared in 150 mm growth plates (VWR).

Alternatively, instead of multiple small reactions, the transformation was performed using a single, large scale reaction, wherein reagent amounts were scaled up accordingly.

The selective media used was a synthetic complete dextrose agar lacking uracil (SCD-Ura) prepared as described in Kaiser et al., Methods in Yeast Genetics, Cold Spring Harbor Press. Cold Spring Harbor, NY, p. 208-210 (1994). Transformants were grown at 30°C for 2-3 days.

The detection of colonies secreting amylase was performed by including red starch in the selective growth media. Starch was coupled to the red dye (Reactive Red-120, Sigma) as per the procedure described by Biely et al., Anal. Biochem., 172:176-179 (1988). The coupled starch was incorporated into the SCD-Ura agar plates at a final concentration of 0.15% (w/v), and was buffered with potassium phosphate to a pH of 7.0 (50-100 mM final concentration).

The positive colonies were picked and streaked across fresh selective media (onto 150 mm plates) in order to obtain well isolated and identifiable single colonies. Well isolated single colonies positive for amylase secretion were detected by direct incorporation of red starch into buffered SCD-Ura agar. Positive colonies were determined by their ability to break down starch resulting in a clear halo around the positive colony visualized directly.

### 4. Isolation of DNA by PCR Amplification

When a positive colony was isolated, a portion of it was picked by a toothpick and diluted into sterile water (30 µl) in a 96 well plate. At this time, the positive colonies were either frozen and stored for subsequent analysis or immediately amplified. An aliquot of cells (5 µl) was used as a template for the PCR reaction in a 25 µl volume containing: 0.5 µl Klentaq (Clontech, Palo Alto, CA); 4.0 µl 10 mM dNTP's (Perkin Elmer-Cetus); 2.5 µl Kentaq buffer (Clontech); 0.25 µl forward oligo 1; 0.25 µl reverse oligo 2; 12.5 µl distilled water. The sequence of the forward oligonucleotide 1 was:
5'-TGTAAAACGACGGCCAGTTAAATAGACCTGCAATTATTAATCT-3' (SEQ ID NO:57)
The sequence of reverse oligonucleotide 2 was:
5'-CAGGAAACAGCTATGACCACCTGCACACCTGCAAATCCATT-3' (SEQ ID NO:58)

PCR was then performed as follows:

| | | | |
|---|---|---|---|
| a. | | Denature | 92°C, 5 minutes |
| b. | 3 cycles of: | Denature | 92°C, 30 seconds |
| | | Anneal | 59°C, 30 seconds |
| | | Extend | 72°C, 60 seconds |
| c. | 3 cycles of: | Denature | 92°C, 30 seconds |
| | | Anneal | 57°C, 30 seconds |
| | | Extend | 72°C, 60 seconds |
| d. | 25 cycles of: | Denature | 92°C, 30 seconds |
| | | Anneal | 55°C, 30 seconds |
| | | Extend | 72°C, 60 seconds |
| e. | | Hold | 4°C |

The underlined regions of the oligonucleotides annealed to the ADH promoter region and the amylase region, respectively, and amplified a 307 bp region from vector pSST-AMY.0 when no insert was present. Typically, the first 18 nucleotides of the 5' end of these oligonucleotides contained annealing sites for the sequencing primers. Thus, the total product of the PCR reaction from an empty vector was 343 bp. However, signal sequence-fused cDNA resulted in considerably longer nucleotide sequences.

Following the PCR, an aliquot of the reaction (5 µl ) was examined by agarose gel electrophoresis in a 1% agarose gel using a Tris-Borate-EDTA (TBE) buffering system as described by Sambrook *et al., supra.* Clones resulting in a single strong PCR product larger than 400 bp were further analyzed by DNA sequencing after purification with a 96 Qiaquick PCR clean-up column (Qiagen Inc., Chats worth, CA).

### EXAMPLE 3

### Isolation of cDNA Clones Using Signal Algorithm Analysis

Various polypeptide-encoding nucleic acid sequences were identified by applying a proprietary signal sequence finding algorithm developed by Genentech, Inc., (South San Francisco, CA) upon ESTs as well as clustered and assembled EST fragments from public (*e.g*., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals. Use of this algorithm resulted in the identification of numerous polypeptide-encoding nucleic acid sequences.

### EXAMPLE 4

### Isolation of cDNA Clones Encoding Human PRO240

A consensus DNA sequence was assembled relative to other EST sequences using phrap as described in Example 1 above. This consensus sequence is designated herein as DNA30873. Based on the DNA30873 consensus sequence, oligonucleotides were synthesized: 1) to identify by PCR a cDNA library that contained the sequence of interest, and 2) for use as probes to isolate a clone of the full-length coding sequence for PR0240.

PCR primers (forward and reverse) were synthesized:
forward PCR primer:
5'-T'CAGCTCCAGACTCTGATACTGCC-3' (SEQ ID NO:59)
reverse PCR primer:
5'-TGCCTTTCTAGGAGGCAGAGCTCC-3' (SEQ ID NO:60)
Additionally, a synthetic oligonucleotide hybridization probe was constructed from the consensus DNA30873 sequence which had the following nucleotide sequence:
hybridization probe:
5'-GGACCCAGAAATGTGTCCTGAGAATGGATCTTGTGTACCTGATGGTCCAG-3' (SEQ ID NO:61)

In order to screen several libraries for a source of a full-length clone, DNA from the libraries was screened by PCR amplification with the PCR primer pair identified above. A positive library was then used to isolate clones encoding the PRO240 gene using the probe oligonucleotide and one of the PCR primers.

RNA for construction of the cDNA libraries was isolated from human fetal liver tissue. The cDNA libraries used to isolate the cDNA clones were constructed by standard methods using commercially available reagents such as those from Invitrogen, San Diego, CA. The cDNA was primed with oligo dT containing a NotI site, linked with blunt to Sall hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253: 1278-1280 (1991)) in the unique XhoI and Notl sites.

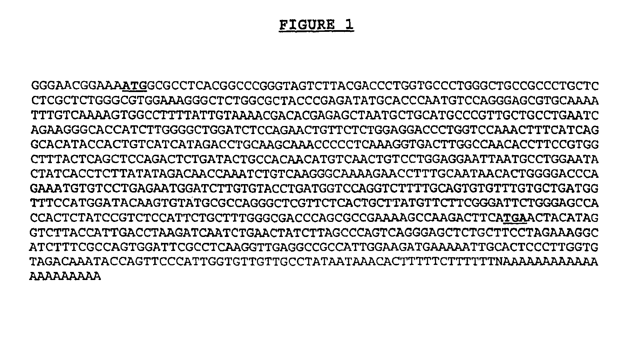

DNA sequencing of the clones isolated as described above gave the full-length DNA sequence for a full-length PRO240 polypeptide (designated herein as DNA34387-1138 [Figure 1, SEQ ID NO:1]) and the derived protein sequence for that PRO240 polypeptide.

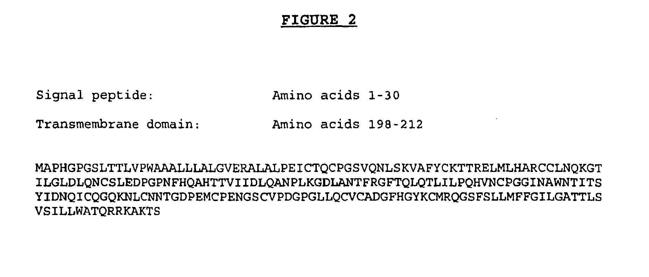

The full length clone identified above contained a single open reading frame with an apparent translational initiation site at nucleotide positions 12-14 and a stop signal at nucleotide positions 699-701 (Figure 1, SEQ ID NO:1). The predicted polypeptide precursor is 229 amino acids long and is shown in Figure 2 (SEQ ID NO:2). Analysis of the full-length PRO240 sequence shown in Figure 2 (SEQ ID NO:2) evidences the presence of a variety of important polypeptide domai ns as shown in Figure 2, wherein the locations given for those important polypeptide domains are approximate as described above. Analysis of the full-length PRO240 sequence evidences the presence of the following: a signal peptide from about amino acid I to about amino acid 30 and a transmembrane domain from about amino acid 198 to about amino acid 212. Clone DNA34387-1138 has been deposited with ATCC on September 16, 1997 and is assigned ATCC deposit no. 209260.

An analysis of the Dayhoff database (version 35.45 SwissProt 35), using the ALIGN-2 sequence alignment analysis of the full-length sequence shown in Figure 2 (SEQ ID NO:2), evidenced sequence identity between the PRO240 amino acid sequence and the serrate precursor protein from *Drosophilia melanogaster* and the C-serrate-1 protein from *Gallus gallus* (30% and 35%, respectively).

### EXAMPLE 5

### Isolation of cDNA Clones Encoding Human PRO381

A consensus DNA sequence was assembled relative to other EST sequences using phrap as described in Example 1 above. This consensus sequence is designated herein as DNA39651. Based on the DNA39651 consensus sequence, oligonucleotides were synthesized: 1) to identify by PCR a cDNA library that contained the sequence of interest, and 2) for use as probes to isolate a clone of the full-length coding sequence for PRO381.

A pair of PCR primers (forward and reverse) were synthesized:
forward PCR primer (39651.f1):
5'-CTTTCCTTGCTTCAGCAACATGAGGC-3' (SEQ ID NO:62)
reverse PCR primer (39651 .r1):
5'-GCCCAGAGCAGGAGGAATGATGAGC-3' (SEQ ID NO:63)
Additionally, a synthetic oligonucleotide hybridization probe was constructed from the consensus DNA39651 sequence which had the following nucleotide sequence:
hybridization probe (39651 p1):
5'-GTGGAACGCGGTCTTGACTCTGTTCGTCACTTCTTTGATTGGGGCTITG-3' (SEQ ID NO:64)

In order to screen several libraries for a source of a full-length clone, DNA from the libraries was screened by PCR amplification with the PCR primer pair identified above. A positive library was then used to isolate clones encoding the PRO381 gene using the probe oligonucleotide and one of the PCR primers. RNA for construction of the cDNA libraries was isolated from human fetal kidney tissue (LIB227).

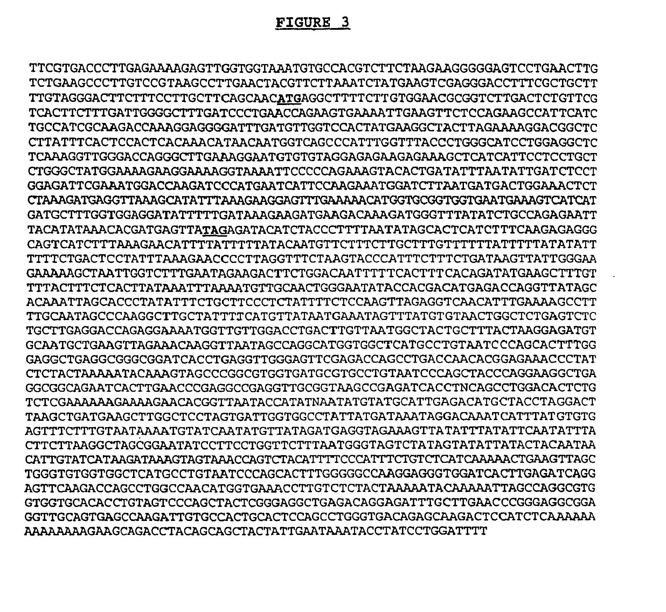

DNA sequencing of the isolated clones isolated as described above gave the full-length DNA sequence for DNA44194-1317 [Figure 3, SEQ ID NO:3]; and the derived protein sequence for PR0381.

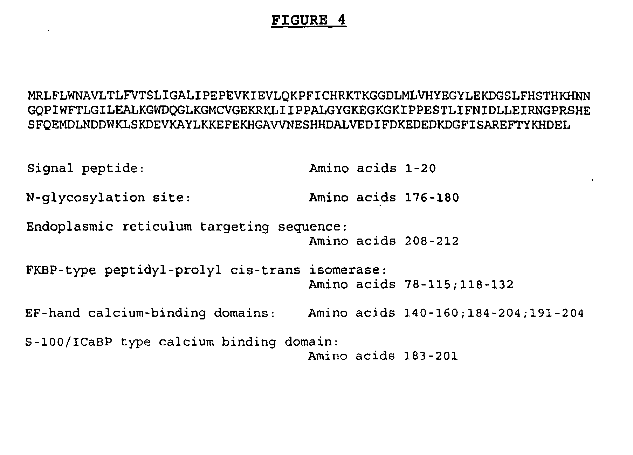

The entire coding sequence of DNA44194-1317 is included in Figure 3 (SEQ ID NO:3). Clone DNA44194-1317 contains a single open reading frame with an apparent translational initiation site at nucleotide positions 174-176, and an apparent stop codon at nucleotide positions 807-809. The predicted polypeptide precursor is 211 amino acids long. Analysis of the full-length PRO381 sequence shown in Figure 4 (SEQ ID NO:4) evidences the presence of a variety of important polypeptide domains, wherein the locations given for those important polypeptide domains are approximate as described above. Analysis of the full-length PRO381 polypeptide shown in Figure 4 evidences the presence of the following: a signal peptide from about amino acid I to about amino acid 20; a potential N-glycosylation site from about amino acid 176 to about amino acid 180; an endoplasmic reticulum targeting sequence from about amino acid 208 to about amino acid 212; FKBP-type peptidyl-prolyl cis-trans isomerase sites from about amino acid 78 to about amino acid 115, and from about amino acid 118 to about amino acid 132; EF-hand calcium binding domains from about amino acid 140 to about amino acid 160, from about amino acid 184 to about amino acid 204, and from about amino acid 191 to about amino acid 204; and an S-100/ICaBP type calcium binding domain from about amino acid 183 to about amino acid 201. Clone DNA44194-1317 has been deposited with the ATCC on April 28, 1998 and is assigned ATCC deposit no. 209808. The full-length PRO381 protein shown in Figure 4 has an estimated molecular weight of about 24,172 daltons and a pI of about 5.99.

Analysis of the amino acid sequence of the full-length PRO381 polypeptide suggests that it possesses sugnificant sequence similarity to FKBP immunophilin proteins, thereby indicating that PRO381 may be a novel FKBP immunophilin homolog. More specifically, an analysis of the Dayhoff database (version 35.45 SwissProt 35), using a WU-BLAST2 sequence alignment analysis of the full-length sequence shown in Figure 4 (SEQ ID NO:4), revealed sequence identity between the PRO381 aminoacid sequence and the following Dayhoff sequences: AF040252_1, 149669, P_R93551, S71238, CELC05C8_1, CEU27353_1, MIP_TRYCR, CEZC455_3, FKB4_HUMAN and 140718.

### EXAMPLE 6

### Isolation of cDNA Clones Encoding Human PRO534

A consensus sequence was obtained relative to a variety of EST sequences as described in Example I above, wherein the consensus sequence obtained is herein designated DNA43038. Based on the 43048 consensus sequence, oligonucleotides were synthesized: 1) to identify by PCR a cDNA library that contained the sequence of interest, and 2) for use as probes to isolate a clone of the full-length coding sequence for PRO534.

A pair of PCR primers (forward and reverse) were synthesized:
forward PCR primer:
5'-CACAGAGCCAGAAGTGGCGGAATC-3' (SEQ ID NO:65)
reverse PCR primer:
5'-CCACATGTTCCTGCTCTTGTCCTGG-3' (SEQ ID NO:66)
Additionally, a synthetic oligonucleotide hybridization probe was constructed from the consensus DNA43038 sequence which had the following nucleotide sequence:
hybridization probe:
5'-CGGTAGTGACTGTACTCTAGTCCTGTTITACACCCCGTGGTGCCG-3' (SEQ ID NO:67).

In order to screen several libraries for a source of a full-length clone, DNA from the libraries was screened by PCR amplification with the PCR primer pair identified above. A positive library was then used to isolate clones encoding the PRO534 gene using the probe oligonucleotide and one of the PCR primers. RNA for construction of the cDNA libraries was isolated from human fetal lung tissue (LIB26).

DNA sequencing of the clones isolated as described above gave the full-length DNA sequence for PRO534 [herein designated as DNA48333-1321] (SEQ ID NO:5) and the derived protein sequence for PRO534.

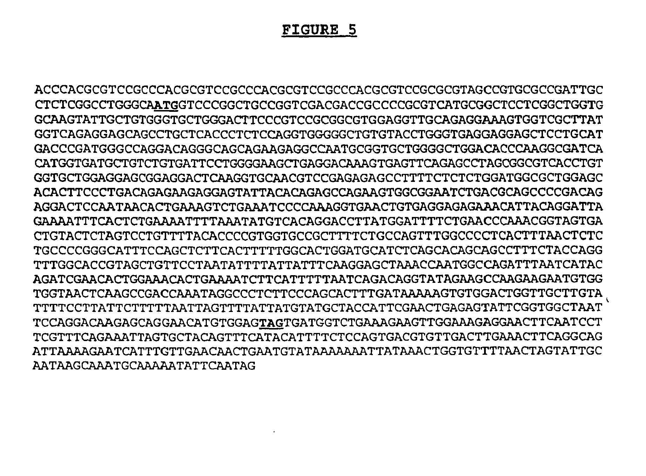

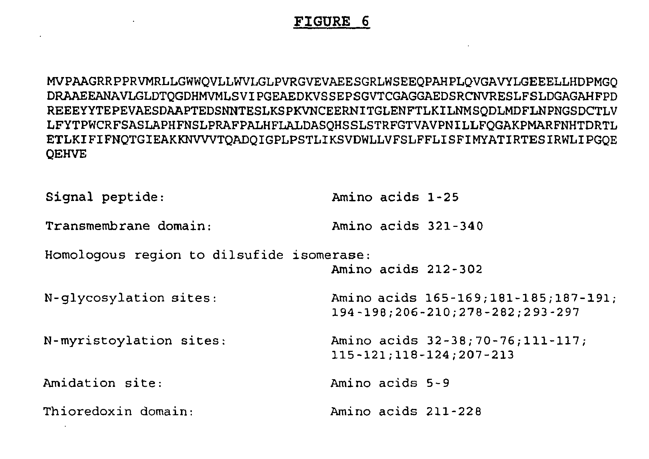

The entire nucleotide sequence of DNA48333-1321 is shown in Figure 5 (SEQ ID NO:5). Clone DNA48333-1321 contains a single open reading frame with an apparent translational initiation site at nucleotide positions 87-89 and ending at the stop codon at nucleotide positions 1167-1169 (Figure 5). The predicted polypeptide precursor is 360 amino acids long (Figure 6). The full-length PRO534 protein shown in Figure 6 has an estimated molecular weight of about 39,885 daltons and a pl of about 4.79. Clone DNA48333-1321 has been deposited with ATCC on March 26, 1998 and is assigned ATCC deposit no. 209701. It is understood that the deposited clone contains the actual sequence, and that the sequences provided herein are representative based on current sequencing techniques.

Analysis of the amino acid sequence of the full-length PRO534 polypeptide suggests that portions of it possess significant sequence identity with the protein disulfide isomerase, thereby indicating that PRO534 may be a novel disulfide isomerase.

Still analyzing the amino acid sequence of PRO534, the signal peptide is at about amino acids 1-25 of SEQ ID NO:6. The transmembrane domain is at about amino acids 321-340 of SEQ ID NO:6. The disulfide isomerase corresponding region is at about amino acids 212-302 of SEQ ID NO:6. The thioredoxin domain is at about amino acids 211-228 of SEQ ID NO:6. N-glycosylation sites are at about amino acids: 165-169, 181-185, 187-191, 194-198, 206-210, 278-282, and 293-297 of SEQ ID NO:6. N-myristoylation sites are at about amino acids: 32-38, 70-76, 111-117, 115-121, 118-124, and 207-213 of SEQ ID NO:6. An amidation site is at about amino acids 5-9 of SEQ ID NO:6. The corresponding nucleotides can routinely be determined from the sequences provided herein. PRO534 has a transmembrane domain rather than an ER retention peptide like other protein disulfide isomerases. Additionally, PRO534 may have an intron at the 5 prime end.

### EXAMPLE 7

### Isolation of cDNA Clones Encoding Human PRO540

A consensus DNA sequence was assembled relative to other EST sequences using phrap as described in Example 1 above. This consensus sequence is designated herein as DNA39631. Based on the DNA39631 consensus sequence, oligonucleotides were synthesized: 1) to identify by PCR a cDNA library that contained the sequence of interest, and 2) for use as probes to isolate a clone of the full-length coding sequence for PRO540.

A pair of PCR primers (forward and reverse) were synthesized:
forward PCR primer (39631.fl):
5'-CTGGGGCTACACACGGGGTGAGG-3' (SEQ ID NO:68)
reverse PCR primer (39631.rl):
5'-GGTGCCGCTGCAGAAAGTAGAGCG-3' (SEQ ID NO:69)
hybridization probe (39631.pl):
5'-GCCCCAAATGAAAACGGGCCCTACTTCCTGGCCCTCCGCGAGATG-3' (SEQ ID NO:70)

In order to screen several libraries for a source of a full-length clone, DNA from the libraries was screened by PCR amplification with one of the PCR primer pairs identified above. A positive library was then used to isolate clones encoding the PRO540 gene using the probe oligonucleotide and one of the PCR primers.

RNA for construction of the cDNA libraries was isolated from human fetal kidney tissue (LIB227). The cDNA libraries used to isolate the cDNA clones were constructed by standard methods using commercially available reagents such as those from Invitrogen, San Diego, CA. The cDNA was primed was oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253: 1278-1280 (1991)) in the unique XhoI and NotI sites.

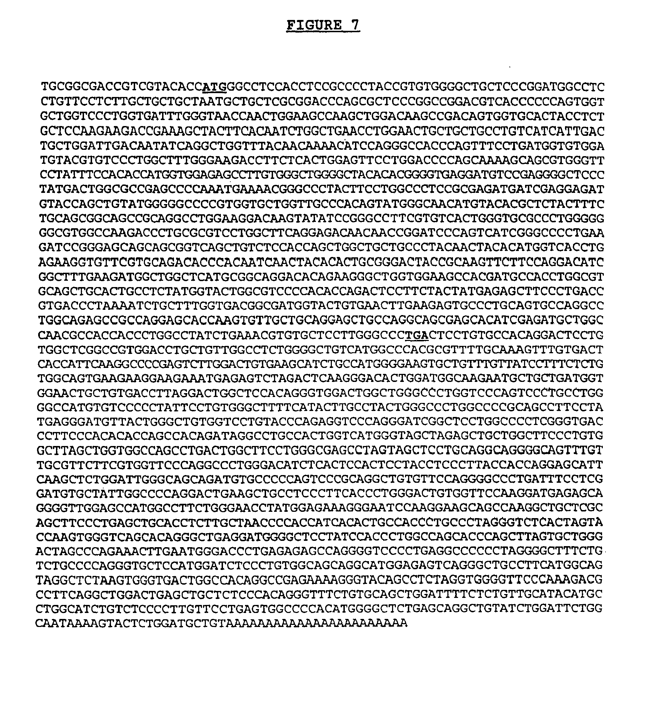

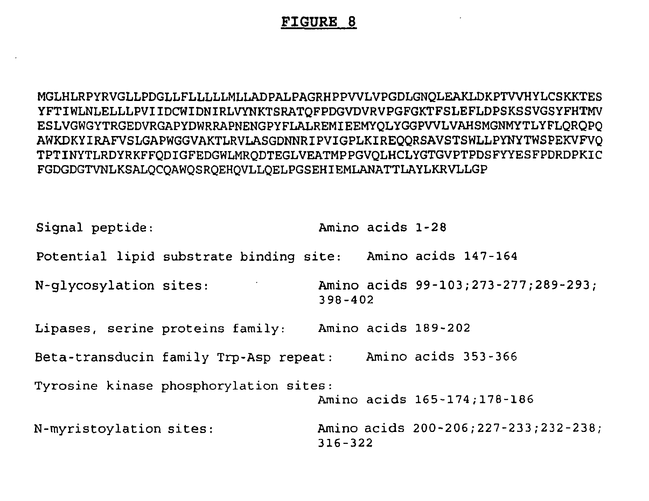

DNA sequencing of the clones isolated as described above gave the full-length DNA sequence for PRO540 herein designated as DNA44189-1322 (SEQ ID NO: 7). Clone DNA44189-1322 contains a single open reading frame with an apparent translational initiation site at nucleotide positions 21-23 and ending at the stop codon at nucleotide positions 1257-1259 (Figure 7). The predicted encoded polypeptide precursor is 412 amino acids long (Figure 8; SEQ ID NO: 8). The full-length PRO540 protein shown in Figure 8 has an estimated molecular weight of about 46,658 daltons and a pI of about 6.65. Important regions of the amino acid sequence of PRO540 (including approximate locations) include the signal peptide (residues 1-28), potential N-glycosylation sites (residues 99-103, 273-277, 289-293, 398-402), a potential lipid substrate binding site (residues 147-164), a sequence typical of lipases and serine proteins (residues 189-202), tyrosine kinase phosphorylation sites (residues 165-174 and 178-186), a beta-transducin family Trp-Asp repeat (residues 353-366) and N-myristoylation sites (residues 200-206, 227-233, 232-238 and 316-322). Clone DNA44189-1322 was deposited with the ATCC on March 26, 1998 and is assigned ATCC deposit no. 209699.

### EXAMPLE 8

### Isolation of cDNA Clones Encoding Human PRO698

A yeast screening assay was employed to identify cDNA clones that encoded potential secreted proteins. Use of this yeast screening assay allowed identification of a single cDNA clone herein designated as DNA39906. Based on the DNA39906 sequence, oligonucleotides were synthesized: 1) to identify by PCR a cDNA library that contained the sequence of interest, and 2) for use as probes to isolate a clone of the full-length coding sequence for PRO698. In order to screen several libraries for a full-length clone, DNA from the libraries was screened by PCR amplification, as per Ausubel et al., Current Protocols in Molecular Biology, with the PCR primer pair. A positive library was then used to isolate clones encoding the gene of interest using the probe oligonucleotide and one of the primer pairs.

PCR primers (forward and reverse) were synthesized:
forward PCR primer:
5'-AGCTGTGGTCATGGTGGTGTGGTG-3' (SEQ ID NO:71)
reverse PCR primer:
5'-CTACCTTGGCCATAGGTGATCCGC-3' (SEQ ID NO:72)
Additionally, a synthetic oligonucleotide hybridization probe was constructed from the consensus DNA39906 sequence which had the following nucleotide sequence:
hybridization probe:
5'-CATCAGCAAACCGTCTGTGGTTCAGCTCAACTGGAGAGGGTT-3' (SEQ ID NO:73)

In order to screen several libraries for a source of a full-length clone, DNA from the libraries was screened by PCR amplification with the PCR primer pair identified above. A positive library was then used to isolate clones encoding the PRO698 gene using the probe oligonucleotide and one of the PCR primers. RNA for construction of the cDNA libraries was isolated from human bone marrow tissue (LIB255). The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see*, Holmes et al., Science, 253:1278-1280 (1991)) in the unique XhoI and NotI sites.

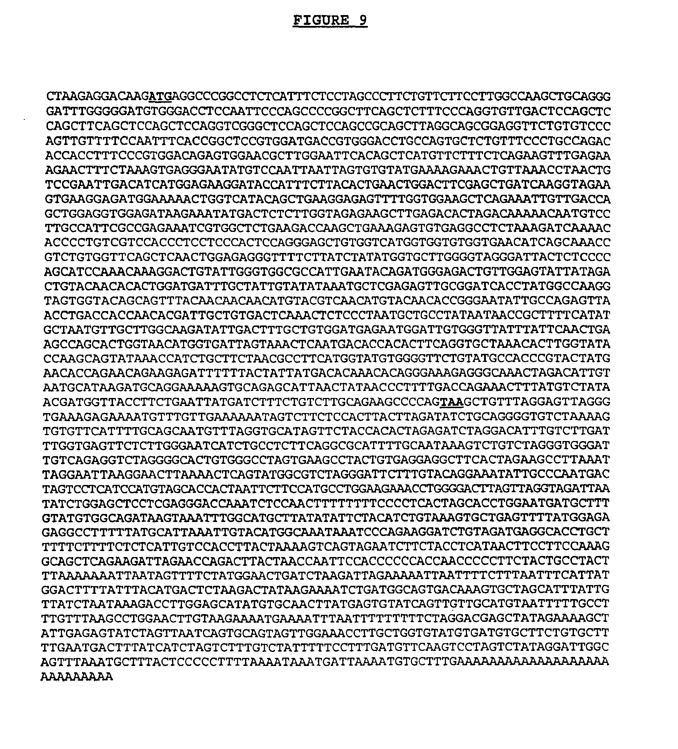

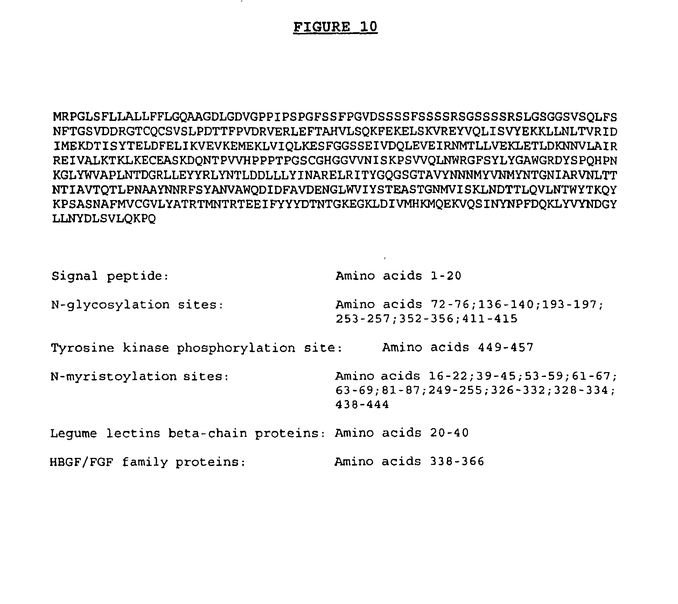

A full length clone was identified (herein designated DNA48320-1433 [SEQ ID NO:9]) that contained a single open reading frame with an apparent translational initiation site at nucleotide positions 14-16 and ending at the stop codon found at nucleotide positions 1544-1546 (Figure 9, SEQ ID NO:9). The predicted polypeptide precursor is 510 amino acids long, and has a calculated molecular weight of approximately 57,280 daltons and an estimated pI of approximately 5.61. Analysis of the full-length PRO698 sequence shown in Figure 10 (SEQ ID NO: 10) evidences the presence of the following: a signal peptide from about amino acid 1 to about amino acid 20, potential N-glycosylation sites from about amino acid 72 to about amino acid 76, from about amino acid 136 to about amino acid 140, from about amino acid 193 to about amino acid 197, from about amino acid 253 to about amino acid 257, from about amino acid 352 to about amino acid 356, and from about amino acid 411 to about amino acid 415; a tyrosine kinase phosphorylation site from about amino acid 449 to about amino acid 457; an amino acid block having homology to legume lectin beta-chain proteins from about amino acid 20 to about amino acid 40; N-myristoylation sites from about amino acid 16 to about amino acid 22, from about amino acid 39 to about amino acid 45, from about amino acid 53 to about amino acid 59, from about amino acid 61 to about amino acid 67, from about amino acid 63 to about amino acid 69, from about amino acid 81 to about amino acid 87, from about amino acid 249 to about amino acid 255, from about amino acid 326 to about amino acid 332, from about amino acid 328 to about amino acid 334, and from about amino acid 438 to about amino acid 444; and an amino acid block having homology to the HBGF/FGF family of proteins from about amino acid 338 to about amino acid 366. Clone DNA48320-1433 has been deposited with ATCC on May 27, 1998 and is assigned ATCC deposit no. 209904.

Analysis of the amino acid sequence of the full-length PRO698 polypeptide suggests that it possesses significant sequence similarity to the olfactomedin protein, thereby indicating that PRO698 may be a novel olfactomedin homolog. More specifically, an analysis of the Dayhoff database (version 35.45 SwissProt 35) evidenced significant homology between the PRO698 amino acid sequence and the following Dayhoff sequences, OLFM_RANCA, 173637, AB006686S3_1 RNU78105_1, RNU72487_1, P_R98225, CELC48E7_4, CEF11C3_3, XLU85970_1 and S42257.

### EXAMPLE 9

### Isolation of cDNA Clones Encoding Human PRO982

DNA57700-1408 was identified by applying the proprietary signal sequence finding algorithm described in Example 3 above. Use of the above described signal sequence algorithm allowed identification of an EST cluster sequence from the Incyte database, designated herein as Incyte cluster sequence no. 43715. This EST cluster sequence was then compared to a variety of expressed sequence tag (EST) databases which included public EST databases *(e.g.,* GenBank) and a proprietary EST DNA database (LIFESEQ^{®}, Incyte Pharmaceuticals, Palo Alto, CA) to identify existing homologies. The homology search was performed using the computer program BLAST or BLAST2 (Altshul et al., Methods in Enzymolgy, 266:460-480 (1996)). Those comparisons resulting in a BLAST score of 70 (or in some cases, 90) or greater that did not encode known proteins were clustered and assembled into a consensus DNA sequence with the program "phrap" (Phil Green, University of Washington, Seattle, Washington). The consensus sequence obtained therefrom is herein designated DNA56095.

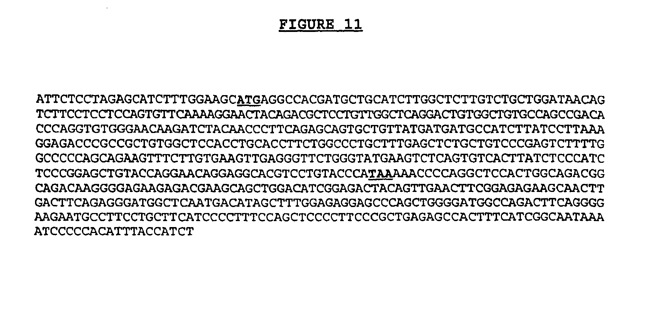

In light of an observed sequence homology between the DNA56095 consensus sequence and Merck EST no. AA024389, from the Merck database, the Merck EST no. AA024389 was purchased and the cDNA insert was obtained and sequenced. It was found herein that the cDNA insert encoded a full-length protein. The sequence of this cDNA insert is shown in Figure 11 (SEQ ID NO:11) and is herein designated as DNA57700-1408.

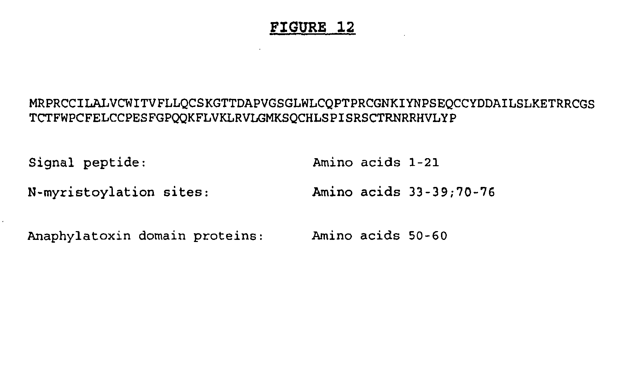

Clone DNA57700-1408 (Figure 11; SEQ ID NO:11) contains a single open reading frame with an apparent translational initiation site at nucleotide positions 26-28 and ending at the stop codon at nucleotide positions 401-403 (Figure 11; SEQ ID NO:11). The predicted polypeptide precursor is 125 amino acids long (Figure 12) and has a calculated molecular weight of approximately 14,198 daltons and an estimated pI of approximately 9.01 (Figure 12). Further analysis of the PRO982 (SEQ ID NO:12) polypeptide of Figure 12 reveals a signal peptide from about amino acid residues 1 to about amino acid 21; N-myristoylation sites from about amino acid 33 to about amino acid 39 and from about amino acid 70 to about amino acid 76; and a potential anaphylatoxin domain from about amino acid residue 50 to about amino acid 60. A cDNA clone containing DNA57700-1408 was deposited with the ATCC on January 12, 1999 and is assigned ATCC deposit No. 203583.

### EXAMPLE 10

### Isolation of cDNA Clones Encoding Human PRO1005

DNA57708-1411 was identified by applying the proprietary signal sequence finding algorithm described in Example 3 above. Use of the above described signal sequence algorithm allowed identification of an EST cluster sequence from the Incyte database, designated herein as Incyte cluster sequence no. 49243. This EST cluster sequence was then compared to a variety of expressed sequence tag (EST) databases which included public EST databases *(e.g.,* GenBank) and a proprietary EST DNA database (LIFESEQ^{®}, Incyte Pharmaceuticals, Palo Alto, CA) to identify existing homologies. The homology search was performed using the computer program BLAST or BLAST2 (Altshul et al., Methods in Enzymolgy, 266:460-480 (1996)). Those comparisons resulting in a BLAST score of 70 (or in some cases, 90) or greater that did not encode known proteins were clustered and assembled into aconsensus DNA sequence with the program "phrap" (Phil Green. University of Washington. Seattle, Washington). The consensus sequence obtained therefrom is herein designated DNA56380.

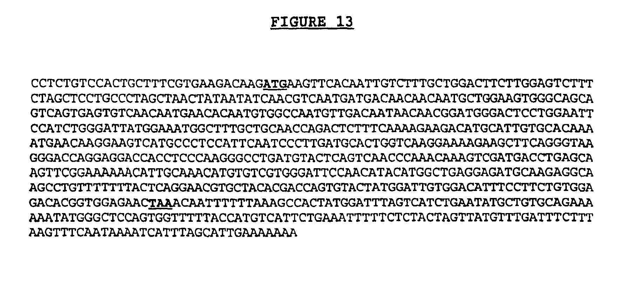

In light of an observed sequence homology between the DNA56380 consensus sequence and Merck EST no. AA256657, from the Merck database, the Merck EST no. AA256657 was purchased and the cDNA insert was obtained and sequenced. It was found herein that the cDNA insert encoded a full-length protein. The sequence of this cDNA insert is shown in Figure 13 (SEQ ID NO:13) and is herein designated as DNA57708-1411.

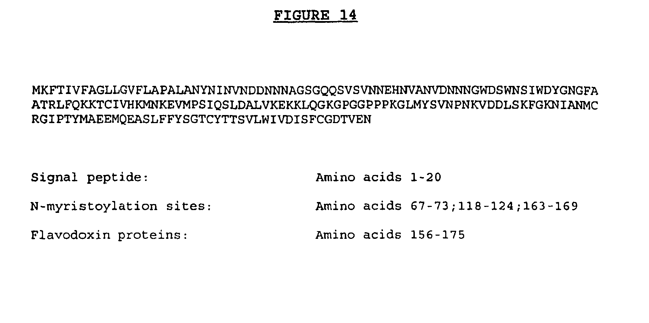

Clone DNA57708-1411 (Figure 13; SEQ ID NO:13) contains a single open reading frame with an apparent translational initiation site at nucleotide positions 30-32 and ending at the stop codon at nucleotide positions 585-587 (Figure 13; SEQ ID NO:13). The predicted polypeptide precursor is 185 amino acids long (Figure 14). The full-length PRO1005 protein shown in Figure 14 (SEQ ID NO:14) has an estimated molecular weight of about 20,331 daltons and a pl of about 5.85. Analysis of the full-length PRO1005 sequence shown in Figure 14 (SEQ ID NO:14) evidences the presence of important polypeptide domains as shown in Figure 14, wherein the locations given for those important polypeptide domains are approximate as described above. Analysis of the full-length PRO1005 sequence shown in Figure 14 evidences the following: a signal peptide from about amino acid 1 to about amino acid 20; N-myristoylation sites from about amino acid 67 to about amino acid 73, from about amino acid 118 to about amino acid 124, and from about amino acid 163 to about amino acid 169: and a flavodoxin protein homology from about amino acid 156 to about amino acid 175. Clone DNA57708-1411 has been deposited with ATCC on June 23, 1998 and is assigned ATCC deposit no. 203021.

An analysis of the Dayhoff database (version 35.45 SwissProt 35 using the ALIGN-2 sequence alignment analysis of the full-length sequence shown in Figure 14 (SEQ ID NO: 14), evidenced sequence identity between the PRO1005 amino acid sequence and the following Dayhoff sequences: DDU07187_1, DDU87912_1, CELD1007_14, A42239. DDU42597_1, CYAG_DICDI, S50452, MRKC_KLEPN, P_R41998, and XYNA_RUMFL.

### EXAMPLE 11

### Isolation of cDNA Clones Encoding Human PRO1007

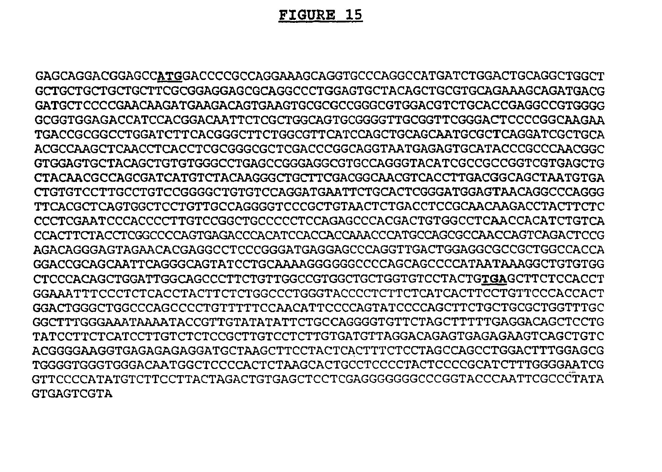

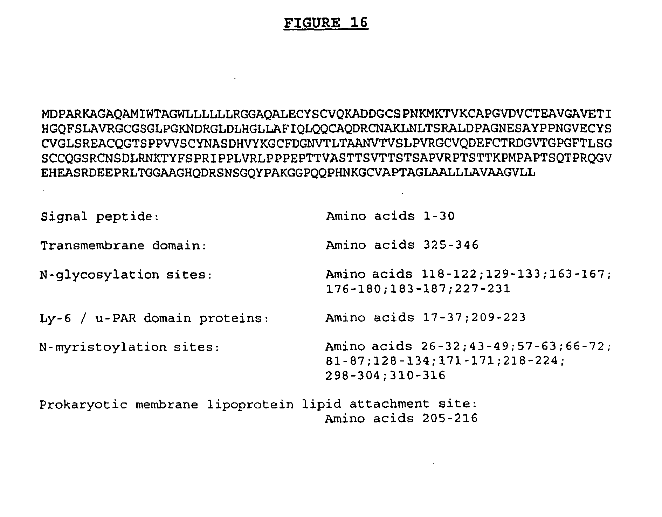

A consensus DNA sequence was assembled relative to other EST sequences using phrap as described in Example 1 above. Use of the ECD homology procedure described above resulted in the identification of an EST sequence designated Merck EST T70513, which was derived from human liver tissue (clone 83012 from library 341). Merck EST T70513 was obtained and further examined and sequenced, resulting in the isolation of the full-length DNA sequence herein designated DNA57690-1374 (Figure 15, SEQ ID NO:15) and the derived PRO1007 native sequence polypeptide (Figure 16, SEQ ID NO:16).

Clone DNA57690-1374 (SEQ ID NO:15) contains a single open reading frame with an apparent translation initiation site at nucleotide positions 16-18 and ending at the stop codon (TGA) at nucleotide positions 1054-1056 (Figure 15), as indicated by bolded underline. The predicted PRO1007 polypeptide precursor (SEQ ID NO:16) is 346 amino acids long (Figure 16), and has a calculated molecular weight of 35.971 daltons and a pl of 8.17. A cDNA clone containing DNA57690-1374 has been deposited with the ATCC on 9 June 1998, and has been assigned deposit number 209950.

Analysis of the amino acid sequence of PRO1007 (SEQ ID NO:16) reveals the putative signal peptide at about amino acid residues 1-30; a transmembrane domain at about amino acid residues 325-346: N-glycosylation sites at about amino acid residues 118-122, 129-133, 163-167, 176-180, 183-187 and 227-231; a Ly-6/u-Par domain protein at about amino acid residues 17-37 and 209-223; N-myristoylation sites at about amino acid residues 26-32, 43-49, 57-63, 66-72, 81-87, 128-134, 171-171, 218-224, 298-304 and 310-316; and a prokaryotic membrane lipoprotein lipid attachment site at about amino acid residues 205-216. The corresponding nucleotides of the amino acids presented herein can be routinely determined given the sequences provided herein.

### EXAMPLE 12

### Isolation of cDNA Clones Encoding Human PRO 1131

A cDNA sequence isolated in the amylase screen described in Example 2 above is herein designated DNA43546. The DNA43546 sequence was then compared to a variety of expressed sequence tag (EST) databases which included public EST databases (e.g., GenBank) and a proprietary EST DNA database (LIFESEQ™, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases, 90) or greater that did not encode known proteins were clustered and assembled into consensus DNA sequences with the program "phrap" (Phil Green, University of Washington, Seattle, Washington). The consensus sequence obtained therefrom is herein designated DNA45627.

Based on the DNA45627 sequence, oligonucleotide probes were generated and used to screen a human library prepared as described in paragraph 1 of Example 2 above. The cloning vector was pRK5B (pRK5B is a precursor of pRK5D that does not contain the Sfil site; *see,* Holmes et al., Science, 253:1278-1280 (1991)), and the cDNA size cut was less than 2800 bp.

PCR primers (forward and 2 reverse) were synthesized:
forward PCR primer:
5'-ATGCAGGCCAAGTACAGCAGCAC-3' (SEQ ID NO:74)
reverse PCR primer 1:
5'-CATGCTGACGACTTCCTGCAAGC-3' (SEQ ID NO:75)
reverse PCR primer 2:
5'-CCACACAGTCTCTGCTTCTTGGG-3' (SEQ ID NO:76)

Additionally, a synthetic oligonucleotide hybridization probe was constructed from the DNA45627 sequence which had the following nucleotide sequence:
hybridization probe:
5'-ATGCTGGATGATGATGGGGACACCACCATGAGCCTGCATT-3' (SEQ ID NO:77)

In order to screen several libraries for a source of a full-length clone, DNA from the libraries was screened by PCR amplification with the PCR primer pair identified above. A positive library was then used to isolate clones encoding the PRO1131 gene using the probe oligonucleotide and one of the PCR primers.

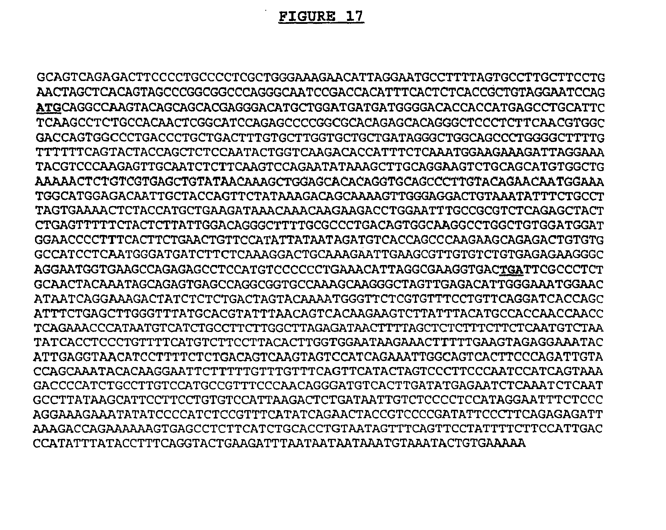

A full length clone was identified that contained a single open reading frame with an apparent translational initiation site at nucleotide positions 144-146, and a stop signal at nucleotide positions 984-986 (Figure 17; SEQ ID NO:17). The predicted polypeptide precursor is 280 amino acids long, and has a calculated molecular weight of approximately 31,966 daltons and an estimated pl of approximately 6.26. The transmembrane domain sequence is at about amino acid residues 49-74 of SEQ ID NO:18; N-glycosylation sites are at about amino acid residues 95-98 and 169-172 of SEQ ID NO:18; tyrosine kinase phosphorylation sites are at about amino acid residues 142-150 and 156-164 of SEQ ID NO: 18; N-myristoylation sites are at about amino acid residues 130-136. 214-220 and 242-248 of SEQ ID NO:18; and the region having sequence identity with LDL receptors is about amino acid residues 50-265 of SEQ ID NO:18. Clone DNA59777-1480 has been deposited with the ATCC on August 11, 1998 and is assigned ATCC deposit no. 203111.

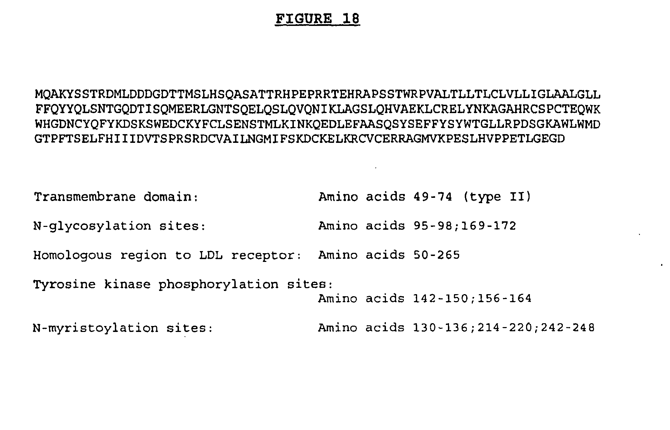

An analysis of the Dayhoff database (version 35.45 SwissProt 35), using a WU-BLAST2 sequence alignment analysis of the full-length sequence shown in Figure 18 (SEQ ID NO:18), evidenced some sequence identity between the PRO1131 amino acid sequence and the following Dayhoff sequences: AB010710_1, 149053, 149115, RNU56863_1, LY4A_MOUSE, 155686, MMU56404_1,149361, AF030313_1 and MMU09739_1.

### EXAMPLE 13

### Isolation of cDNA Clones Encoding Human PRO1 157

DNA60292-1506 was identified by applying the proprietary signal sequence finding algorithm described in Example 3 above. Use of the above described signal sequence algorithm allowed identification of an EST cluster sequence from the LIFESEQ^{®} database, designated Incyte EST cluster no. 65816. This EST cluster sequence was then compared to a variety of expressed sequence tag (EST) databases which included public EST databases (*e.g*., GenBank) and a proprietary EST DNA database (LIFESEQ®, Incyte Pharmaceuticals, Palo Alto, CA) to identify existing homologies. One or more of the ESTs was derived from a human mast cell line from normal human prostatic epithelial cells. The homology search was performed using the computer program BLAST or BLAST2 (Altshul et al., Methods in Enzymology, 266:460-480 (1996)). Those comparisons resulting in a BLAST score of 70 (or in some cases, 90) or greater that did not encode known proteins were clustered and assembled into a consensus DNA sequence with the program "phrap" (Phil Green, University of Washington, Seattle, Washington). The consensus sequence obtained therefrom is herein designated as DNA56058.

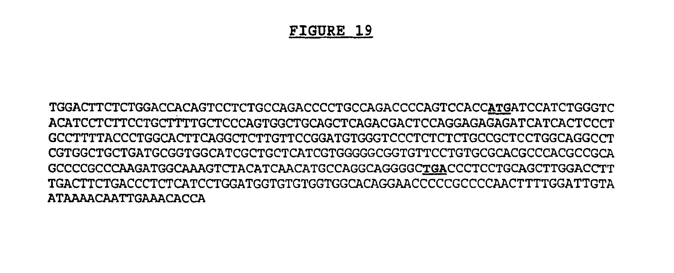

In light of the sequence homology between the DNA56058 consensus sequence and the Merck EST no. AA516481, Merck EST no. AA516481 was purchased and the cDNA insert was obtained and sequenced. The sequence of this cDNA insert is shown in Figure 19 (SEQ ID NO:19) and is herein designated as DNA60292-1506.

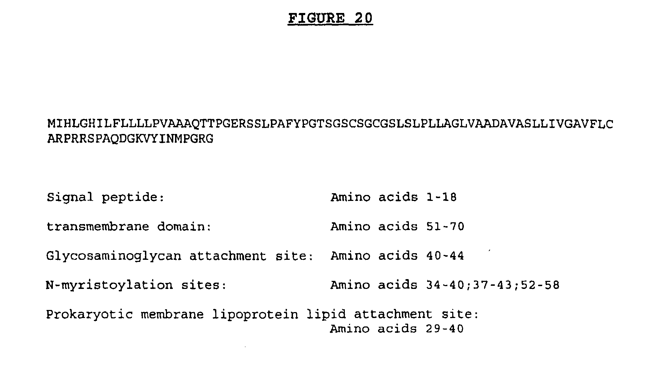

The entire coding sequence of DNA60292-1506 is included in Figure 19 (SEQ ID NO:19). Clone DNA60292-1506 contains a single open reading frame with an apparent translational initiation site at nucleotide positions 56-58 and ending at the stop codon at nucleotide positions 332-334 (Figure 19). The predicted polypeptide precursor is 92 amino acids long (Figure 20; SEQ ID NO:20). The full-length PRO1157 protein shown in Figure 20 has an estimated molecular weight of about 9.360 daltons and a pI of about 9.17. Analysis of the full-length PRO1157 sequence shown in Figure 20 (SEQ ID NO:20) evidences the presence of a variety of important polypeptide domains, wherein the locations given for those important polypeptide domains are approximate as described above. Analysis of the full-length PRO1157 sequence shown in Figure 20 evidences the presence of the following: a signal peptide from about amino acid 1 to about amino acid 18; a putative transmembrane domain from about amino acid 51 to about amino acid 70; a glycosaminoglycan attachment site from about amino acid 40 to about amino acid 44; N-myristoylation sites from about amino acid 34 to about amino acid 40, from about amino acid 37 to about amino acid 43 and from about amino acid 52 to about amino acid 58; and a prokaryotic membrane lipoprotein lipid attachment site from about amino acid 29 to about amino acid 40. Clone DNA60292-1506 has been deposited with ATCC on December 15, 1998 and is assigned ATCC deposit no. 203540.

An analysis of the Dayhoff database (version 35.45 SwissProt 35), using a WU-BLAST2 sequence alignment analysis of the full-length sequence shown in Figure 20 (SEQ ID NO:20), evidenced homology between the PRO 1157 amino acid sequence and the following Dayhoff sequences: PTPN_HUMAN. B69251, 151419, AF019562_1, AF019563_1, C211_HUMAN, 137577, A39171, GAT5_MOUSE, ACR3_MOUSE, 5H6_RAT, P_W31512, and 558082.

### EXAMPLE 14

### Isolation of cDNA Clones Encoding Human PRO 1199

A public expressed sequence tag (EST) DNA database (GenBank) was searched with the full-length murine m-FIZZ1 DNA (DNA53517), and an EST [designated AA311223 and renamed as DNA53028] was identified which showed homology to the m-FIZZ1 DNA.

Oligonucleotides probes based upon the above described EST sequence were then synthesized: 1) to identify by PCR a cDNA library that contained the sequence of interest, and 2) for use as probes to isolate a clone of the full-length coding sequence for PRO1199. Forward and reverse PCR primers generally range from 20 to 30 nucleotides and are often designed to give a PCR product of about 100-1000 bp in length. The probe sequences are typically 40-55 bp in length. In order to screen several libraries for a full-length clone, DNA from the libraries was screened by PCR amplification, as per Ausubel *et al.,* Current Protocols in Molecular Biology, *supra,* with the PCR primer pair. A positive library was then used to isolate clones encoding the gene of interest using the probe oligonucleotide and one of the primer pairs.

The oligonucleotide probes employed were as follows:
forward primer (h-FIZZ3.f):
5'-GGATTTGGTTAGCTGAGCCCACCGAGA-3' (SEQ ID NO:78)
reverse primer (h-FIZZ3.r):
5'-GCACTGCGCGCGACCTCAGGGCTGCA-3' (SEQ ID NO:79)
probe (h-FIZZ3.p):
5'-CTTATTGCCCTAAATATTAGGGAGCCGGCGACCTCCTGGATCCTCTCATT-3' (SEQ ID NO:80)

In order to screen several libraries for a source of a full-length clone, DNA from the libraries was screened by PCR amplification with the PCR primer pair identified above. A positive library was then used to isolate clones encoding the PRO1199 gene using the probe oligonucleotide and one of the PCR primers.

RNA for construction of cDNA libraries was then isolated from human bone marrow tissue. The cDNA libraries used to isolate the cDNA clones encoding human PRO1199 were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site*: see,* Holmes et al., Science, 253:1278-1280 (1991)) in the unique XhoI and NotI.

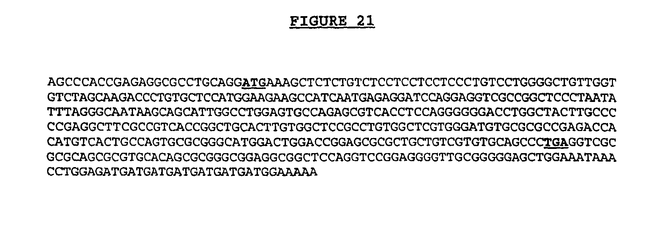

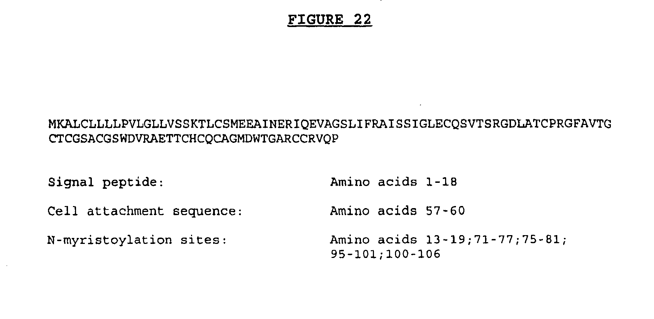

A full length clone DNA65351-1366-1 was identified that contained a single open reading frame with an apparent translational initiation site at nucleotide positions 25-27 and a stop signal at nucleotide positions 349-351 (Figure 21; SEQ ID NO:21). The predicted polypeptide precursor is 108 amino acids long, and has a calculated molecular weight of approximately 11,419 daltons and an estimated pl of approximately 7.05. Analysis of the full-length PRO1199 sequence shown in Figure 22 (SEQ ID NO:22) evidences the presence of a variety of important polypeptide domains as shown in Figure 22, wherein the locations given for those important polypeptide domains are approximate as described above. Analysis of the full-length PRO 1199 polypeptide shown in Figure 22 evidences the presence of the following: a signal peptide from about amino acid 1 to about amino acid 18; a cell attachment sequence motif (RGD) from about amino acid 57 to about amino acid 60; and N-myristoylation sites from about amino acid 13 to about amino acid 19, from about amino acid 71 to about amino acid 77, from about amino acid 75 to about amino acid 81, from about amino acid 95 to about amino acid 101, and from about amino acid 100 to about amino acid 106. Clone DNA65351-1366-1 has been deposited with ATCC on May 12, 1998 and is assigned ATCC deposit no. 209856.

### EXAMPLE 15

### Isolation of cDNA Clones Encoding Human PRO1265

DNA60764-1533 was identified by applying the proprietary signal sequence finding algorithm described in Example 3 above. Use of the above described signal sequence algorithm allowed identification of an EST cluster sequence from the LIFESEQ^{®} database, designated Incyte EST cluster no. 86995. This EST cluster sequence was then compared to a variety of expressed sequence tag (EST) databases which included public EST databases (*e.g*., GenBank) and a proprietary EST DNA database (LIFESEQ^{®}. Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases, 90) or greater that did not encode known proteins were clustered and assembled into a consensus DNA sequence with the program "phrap" (Phil Green, University of Washington, Seattle, Washington). The consensus sequence obtained therefrom is herein designated as DNA55717.

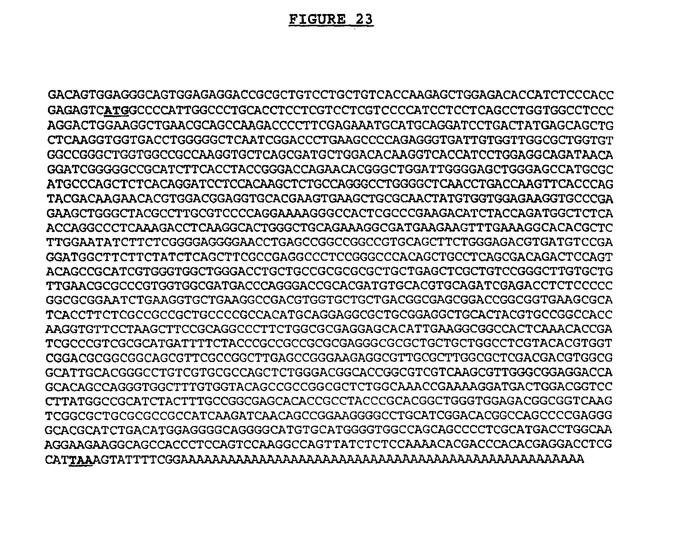

In light of the sequence homology between the DNA55717 consensus sequence and Incyte EST no. 20965, Incyte EST no. 20965 was purchased and the cDNA insert was obtained and sequenced. The sequence of this cDNA insert is shown in Figure 23 (SEQ ID NO:23) and is herein designated as DNA60764-1533.

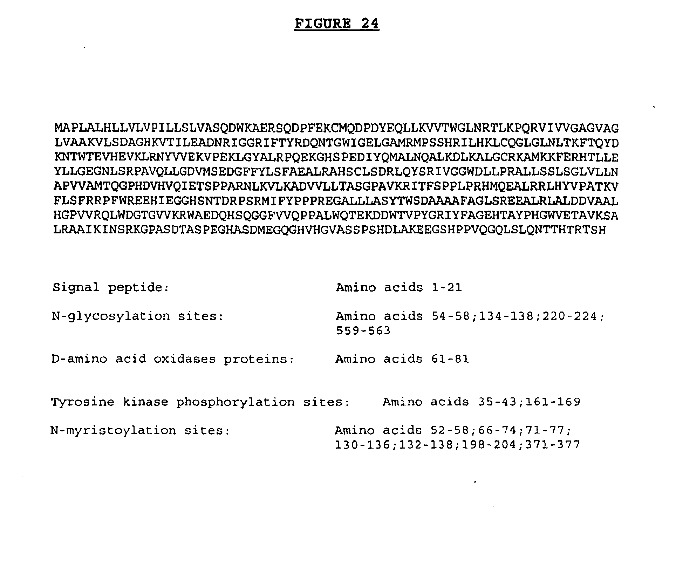

The entire coding sequence of DNA60764-1533 is included in Figure 23 (SEQ ID NO:23). Clone DNA60764-1533 contains a single open reading frame with an apparent translational initiation site at nucleotide positions 79-81 and ending at the stop codon at nucleotide positions 1780-1782 (Figure 23). The predicted polypeptide precursor is 567 amino acids long (Figure 24; SEQ ID NO:24). The full-length PRO1265 protein shown in Figure 24 has an estimated molecular weight of about 62,881 daltons and a pl of about 8.97. Analysis of the full-length PRO1265 sequence shown in Figure 24 (SEQ ID NO:24) evidences the presence of a variety of important polypeptide domains, wherein the locations given for those important polypeptide domains are approximate as described above. Analysis of the full-length PRO1265 sequence shown in Figure 24 evidences the presence of the following: a signal peptide from about amino acid to about amino acid 21; N-glycosylation sites from about amino acid 54 to about amino acid 58. from about amino acid 134 to about amino acid 138, from about amino acid 220 to about amino acid 224, and from about amino acid 559 to about amino acid 563; tyrosine kinase phosphorylation sites from about amino acid 35 to about amino acid 43. and from about amino acid 161 to about amino acid 169; N-myristoylation sites from about amino acid 52 to about amino acid 58, from about amino acid 66 to about amino acid 74, from about amino acid 71 to about amino acid 77, from about amino acid 130 to about amino acid 136, from about amino acid 132 to about amino acid 138, from about amino acid 198 to about amino acid 204, and from about amino acid 371 to about amino acid 377; and a D-amino acid oxidase protein site from about amino acid 61 to about amino acid 81. Clone DNA60764-1533 has been deposited with ATCC on November 10, 1998 and is assigned ATCC deposit no. 203452.

An analysis of the Dayhoff database (version 35.45 SwissProt 35), using a WU-BLAST2 sequence alignment analysis of the full-length sequence shown in Figure 24 (SEQ ID NO:24), evidenced significant sequence identity between the PRO1265 amino acid sequence and Dayhoff sequence no. MMU70429_1. Sequence homology was also found to exist between the full-length sequence shown in Figure 24 (SEQ ID NO:24.) and the following Dayhoff sequences: BC542A_1, E69899, S76290, MTV014_14, AOFB_HUMAN, ZMJ002204_1, S45812_1, DBRNAPD_1, and CRT1_SOYBN.

### EXAMPLE 16

### Isolation of cDNA Clones Encoding Human PRO1286

DNA64903-1553 was identified by applying the proprietary signal sequence finding algorithm described in Example 3 above. Use of the above described signal sequence algorithm allowed identification of an EST cluster sequence from the LIFESEQ^{®} database, designated Incyte EST cluster no. 86809. This EST cluster sequence was then compared to a variety of expressed sequence tag (EST) databases which included public EST databases (*e.g*., GenBank) and a proprietary EST DNA database (LIFESEQ^{®}, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases, 90) or greater that did not encode known proteins were clustered and assembled into a consensus DNA sequence with the program "phrap" (Phil Green, University of Washington, Seattle, Washington). ESTs in the assembly included those identified from tumors, cell lines, or diseased tissue. One or more of the ESTs was obtained from a cDNA library constructed from RNA isolated from diseased colon tissue. The consensus sequence obtained therefrom is herein designated as DNA58822.

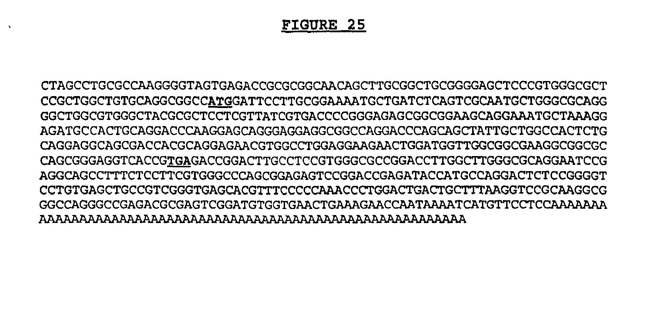

In light of the sequence homology between the DNA58822 sequence and Incyte EST clone no. 1695434. Incyte EST no. 1695434 was purchased and the cDNA insert was obtained and sequenced. The sequence of this cDNA insert is shown in Figure 25 (SEQ ID NO:25) and is herein designated as DNA64903-1553.

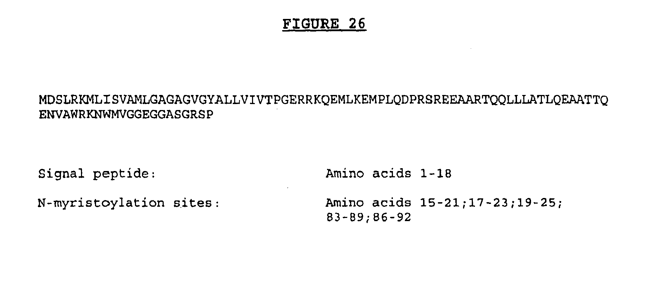

The entire coding sequence of DNA64903-1553 is included in Figure 25 (SEQ ID NO:25). Clone DNA64903-1553 contains a single open reading frame with an apparent translational initiation site at nucleotide positions 93-95 and ending at the stop codon at nucleotide positions 372-374 (Figure 25). The predicted polypeptide precursor is 93 amino acids long (Figure 26; SEQ ID NO:26). The full-length PRO1286 protein shown in Figure 26 has an estimated molecular weight of about 10,111 1 daltons and a pl of about 9.70. Analysis of the full-length PRO1286 sequence shown in Figure 26 (SEQ ID NO:26) evidences the presence of a variety of important polypeptide domains, wherein the locations given for those important polypeptide domains are approximate as described above. Analysis of the full-length PRO1286 sequence shown in Figure 26 evidences the presence of the following: a signal peptide from about amino acid 1 to about amino acid 18; and N-myristoylation sites from about amino acid 15 to about amino acid 21, from about amino acid 17 to about amino acid 23, from about amino acid 19 to about amino acid 25, from about amino acid 83 to about amino acid 89, and from about amino acid 86 to about amino acid 92. Clone DNA64903-1553 has been deposited with ATCC on September 15, 1998 and is assigned ATCC deposit no. 203223.

An analysis of the Dayhoff database (version 35.45 SwissProt 35), using a WU-BLAST2 sequence alignment analysis of the full-length sequence shown in Figure 26 (SEQ ID NO:26), revealed some homology between the PRO1286 amino acid sequence and the following Dayhoff sequences: SR5C_ARATH, CELC17H12_11, MCPD_ENTAE, JQ2283, INVO_LEMCA, P_R07309, ADEVBCAGN_4, AF020947_1, CELT23H2_1, and MDH_STRAR.

### EXAMPLE 17

### Isolation of cDNA Clones Encoding Human PRO1313

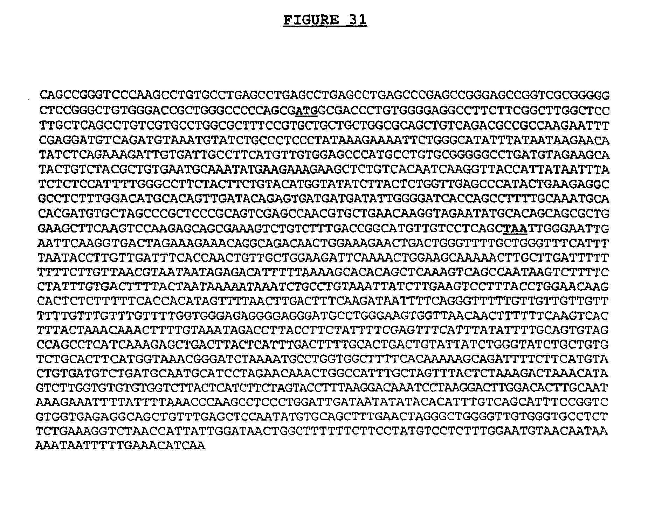

A consensus DNA sequence was assembled relative to other EST sequences using phrap as described in Example 1 above. This consensus sequence is designated herein as DNA64876. Based on the DNA64876 consensus sequence and upon a search for sequence homology with a proprietary Genentech EST sequence designated as DNA57711, a Merck/Washington University EST sequence (designated R80613) was found to have significant homology with DNA64876 and DNA57711. Therefore, the Merck/Washington University EST clone no. R80613 3 was purchased and the insert thereof obtained and sequenced, thereby giving rise to the DNA64966-1575 sequence shown in Figure 31 (SEQ ID NO:31), and the derived protein sequence for PRO1313.

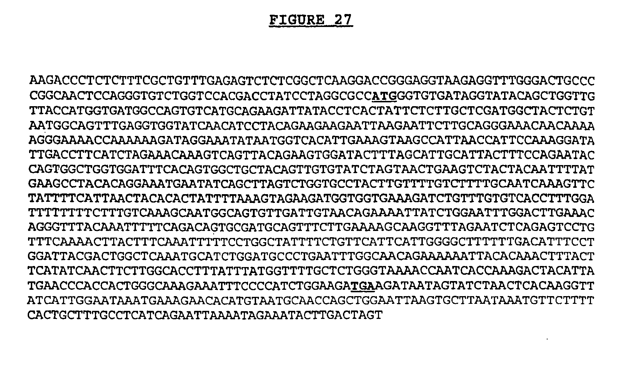

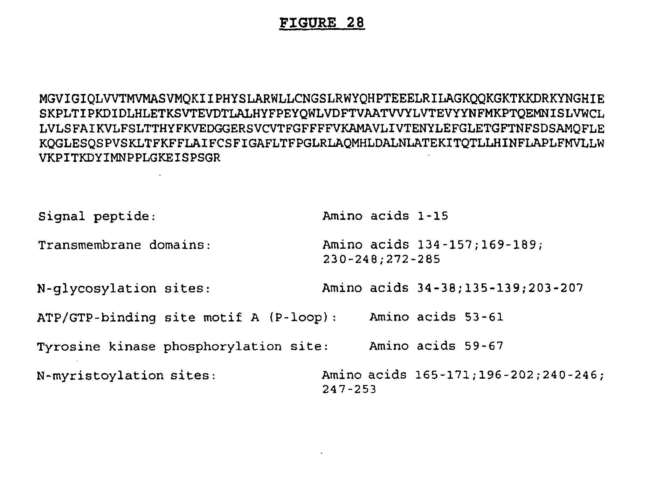

The entire coding sequence of DNA64966-1575 is included in Figure 27 (SEQ ID NO:27). Clone DNA64966-1575 contains a single open reading frame with an apparent translational initiation site at nucleotide positions 115-117, and an apparent stop codon at nucleotide positions 1036-1038. The predicted polypeptide precursor is 307 amino acids long, and has an estimated molecular weight of about 35,098 daltons and a pI of about 8.11. Analysis of the full-length PRO1313 sequence shown in Figure 28 (SEQ ID NO:28) evidences the presence of a variety of important polypeptide domains, wherein the locations given for those important polypeptide domains are approximate as described above. Analysis of the full-length PRO1313 polypeptide shown in Figure 28 evidences the presence of the following: a signal peptide from about amino acid 1 to about amino acid 15; transmembrane domains from about amino acid 134 to about amino acid 157, from about amino acid 169 to about amino acid 189, from about amino acid 230 to about amino acid 248, and from about amino acid 272 to about amino acid 285; N-glycosylation sites from about amino acid 34 to about amino acid 38, from about amino acid 135 to about amino acid 139, and from about amino acid 203 to about amino acid 207; a tyrosine kinase phosphorylation site from about amino acid 59 to about amino acid 67; N-myristoylation sites from about amino acid 165 to about amino acid 171, from about amino acid 196 to about amino acid 202. from about amino acid 240 to about amino acid 246, and from about amino acid 247 to about amino acid 253; and an ATP/GTP-binding site motif A (P-loop) from about amino acid 53 to about amino acid 61. Clone DNA64966-1575 has been deposited with the ATCC on January 12, 1999 and is assigned ATCC deposit no. 203575.

An analysis of the Dayhoff database (version 35.45 SwissProt 35), using a WU-BLAST2 sequence alignment analysis of the full-length sequence shown in Figure 28 (SEQ ID NO:28), evidenced significant homology between the PRO1313 amino acid sequence and the following Dayhoff sequences: CELT27A1_3, CEF09C6_7, U93688_9, H64896, YDCX_ECOLI, and RNU06101_1.

### EXAMPLE 18

### Isolation of cDNA Clones Encoding Human PRO1338

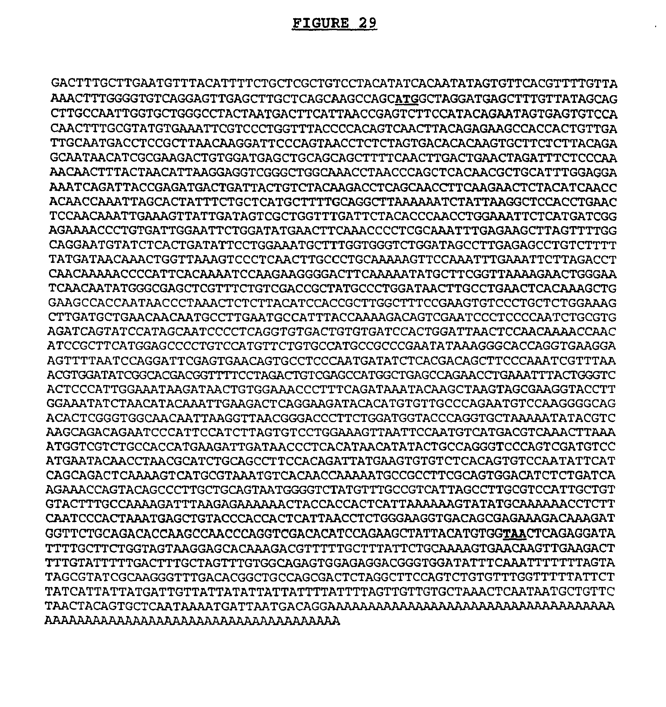

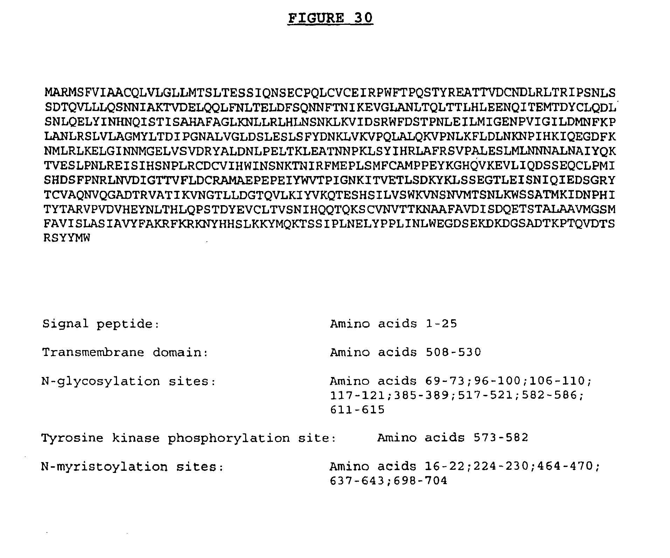

The use of yeast screens resulted in EST sequences which were then compared to various public and private EST databases in a manner similar to that described above under ECD homology (Example 1) and which resulted in the identification of Incyte EST2615184, an EST derived fromcholecystitis gall bladder tissue. Analysis of the corresponding full-length sequence ultimately resulted in the isolation of DNA66667 (SEQ ID NO:29, Figure 29) and the derived PRO1338 native sequence protein (SEQ ID NO:30, Figure 30).

DNA66667 (SEQ ID NO:29) as shown in Figure 29 contains a single open reading frame with a translation initiation site at about nucleotide residues 115-117 and ending at the stop codon (TAA) at nucleotide positions 2263-2265, as indicated by bolded underline. The predicted PRO1338 polypeptide precursor (SEQ ID NO:30) is 716 amino acids in length (Figure 30), and has a calculated molecular weight of 80,716 daltons and a pI of 6.06.

Analysis of the PRO1338 polypeptide (SEQ ID NO:30) of Figure 30 reveals a signal sequence at about amino acid residues 1 to 25; a transmembrane domain at about amino acid residues 508 to 530; N-glycosylation sites at about amino acid residues 69-73, 96-100, 106-110, 117-121, 385-389, 517-521, 582-586 and 611-615; a tyrosine kinase phosphorylation site at about residues 573-582; and N-myristoylation sites at about amino acid residues 16-22, 224-230, 464-470, 637-643 and 698-704.

A cDNA containing DNA66667 has been deposited with the ATCC under the designation DNA66667 on September 22, 1998 and has been assigned ATCC deposit number 203267.

### EXAMPLE 19

### Isolation of cDNA Clones Encoding Human PRO1375

A Merck/Wash. U. database was searched and a Merck EST was identified. This sequence was then put in a program which aligns it with other sequences from the Swiss-Prot public database, public EST databases (*e.g.*, GenBank, Merck/Wash. U.), and a proprietary EST database (LIFESEQ^{®}, Incyte Pharmaceuticals, Palo Alto, CA). The search was performed using the computer program BLAST or BLAST2 (Altschul et al., Methods in Enzymology, 266:460-480 (1996)) as a comparison of the extracellular domain (ECD) protein sequences to a 6 frame translation of the EST sequences. Those comparisons resulting in a BLAST score of 70 (or in some cases, 90) or greater that did not encode known proteins were clustered and assembled into consensus DNA sequences with the program "phrap" (Phil Green, University of Washington, Seattle, Washington).

A consensus DNA sequence was assembled relative to other EST sequences using phrap. This consensus sequence is designated herein "DNA67003".

Based on theDNA67003 consensus sequence, a nucleic acid was identified in a human pancreas library. DNA sequencing of the clone gave the full-length DNA67004-1614 sequence and the derived protein sequence for PRO1375.

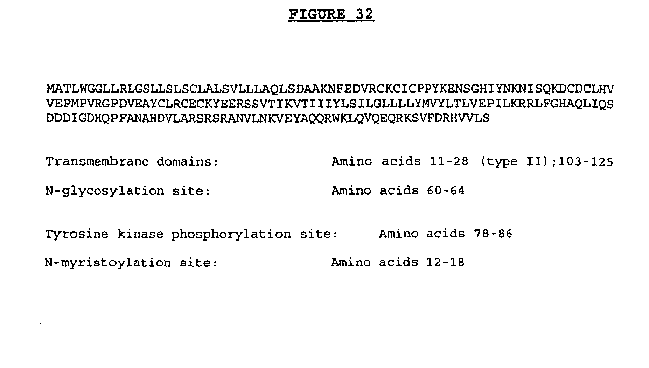

The entire coding sequence of PRO 1375 is shown in Figure 31 (SEQ ID NO:31). Clone DNA67004-1614 contains a single open reading frame with an apparent translational initiation site at nucleotide positions 104-106, and an apparent stop codon at nucleotide positions 698-700. The predicted polypeptide precursor is 198 amino acids long and is shown in Figure 32 (SEQ ID NO:32). The transmembrane domains are at about amino acids 11-28 (type II) and 103-125; an N-glycosylation site is at about amino acids 60-64; a tyrosine kinase phosphorylation site is at about amino acids 78-86; and an N-myristoylation site is at about amino acids 12-18. Clone DNA67004-1614 has been deposited with ATCC on August 11, 1998 and is assigned ATCC deposit no. 203115. The full-length PRO1375 protein shown in Figure 32 has an estimated molecular weight of about 22,531 daltons and a pI of about 8.47.

An analysis of the Dayhoff database (version 35.45 SwissProt 35), using a WU-BLAST2 sequence alignment analysis of the full-length sequence shown in Figure 32, revealed sequence identity between the PRO1375 amino acid sequence and the following Dayhoff sequences: AF026198_5, CELR12C12_5, S73465, Y011_MYCPN, S64538_1, P_P8150, MUVSHPO10_1, VSH_MUMPL and CVU59751_5.

### EXAMPLE 20

### Isolation of cDNA Clones Encoding Human PRO1410

DNA68874-1622 was identified by applying the proprietary signal sequence finding algorithm described in Example 3 above. Use of the above described signal sequence algorithm allowed identification of an EST cluster sequence from the LIFESEQ^{®} database, designated Incyte EST cluster sequence no. 98502. This EST cluster sequence was then compared to a variety of expressed sequence tag (EST) databases which included public EST databases (*e.g*., GenBank) and a proprietary EST DNA database (LIFESEQ®, Incyte Pharmaceuticals, Palo Alto. CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases, 90) or greater that did not encode known proteins were clustered and assembled into a consensus DNA sequence with the program "phrap" (Phil Green, University of Washington, Seattle, Washington). The consensus sequence obtained therefrom is herein designated as DNA56451.

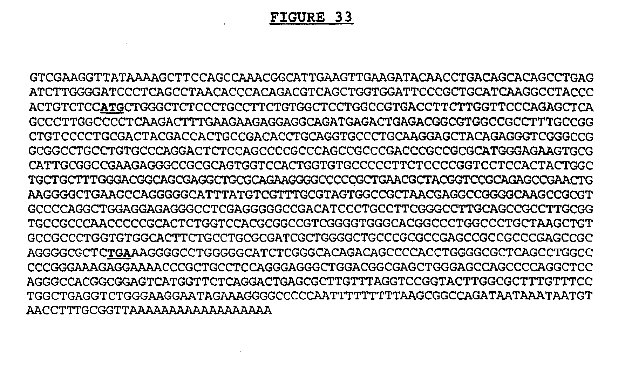

In light of the sequence homology between the DNA56451 sequence and the Incyte EST clone no. 1257046, the Incyte EST clone no. 1257046 was purchased and the cDNA insert was obtained and sequenced. The sequence of this cDNA insert is shown in Figure 33 (SEQ ID NO:33) and is herein designated as DNA68874-1622.

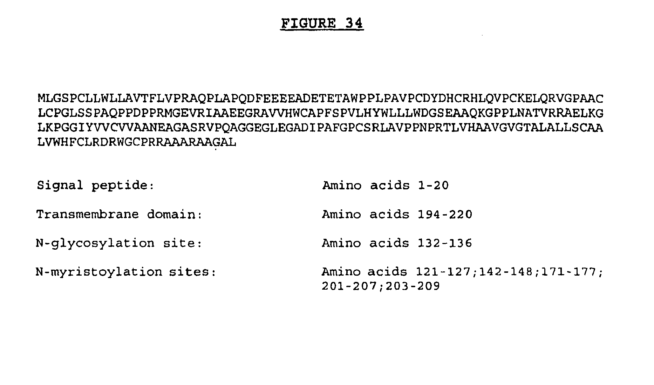

Clone DNA68874-1622 contains a single open reading frame with an apparent translational initiation site at nucleotide positions 152-154 and ending at the stop codon at nucleotide positions 866-868 (Figure 33). The predicted polypeptide precursor is 238 amino acids long (Figure 34; SEQ ID NO:34). The full-length PRO1410 protein shown in Figure 34 has an estimated molecular weight of about 25,262 daltons and a pl of about 6.44. Analysis of the full-length PRO1410 sequence shown in Figure 34 (SEQ ID NO:34) evidences the presence of a variety of important polypeptide domains, wherein the locations given for those important polypeptide domains are approximate as described above. Analysis of the full-length PRO1410 sequence shown in Figure 34 evidences the presence of the following: a signal peptide from about amino acid 1 to about amino acid 20; a transmembrane domain from about amino acid 194 to about amino acid 220; a potential N-glycosylation site from about amino acid 132 to about amino acid 136; and N-myristoylation sites from about amino acid 121 to about amino acid 127, from about amino acid 142 to about amino acid 148, from about amino acid 171 to about amino acid 177, from about amino acid 201 to about amino acid 207, and from about amino acid 203 to about amino acid 209. Clone DNA68874-1622 has been deposited with ATCC on September 22, 1998 and is assigned ATCC deposit no. 203277.

An analysis of the Dayhoff database (version 35.45 SwissProt 35), using a WU-BLAST2 sequence alignment analysis of the full-length sequence shown in Figure 34 (SEQ ID NO:34), evidenced significant homology between the PRO1410 amino acid sequence and the following Dayhoff sequences: 148652, P_R76466, HSMHC3W36A_2, EPB4_HUMAN, P_R14256, EPA8_MOUSE, P_R77285, P_W13569, AF000560_1, and ASF1_HELAN.

### EXAMPLE 21

### Isolation of cDNA Clones Encoding Human PRO1488

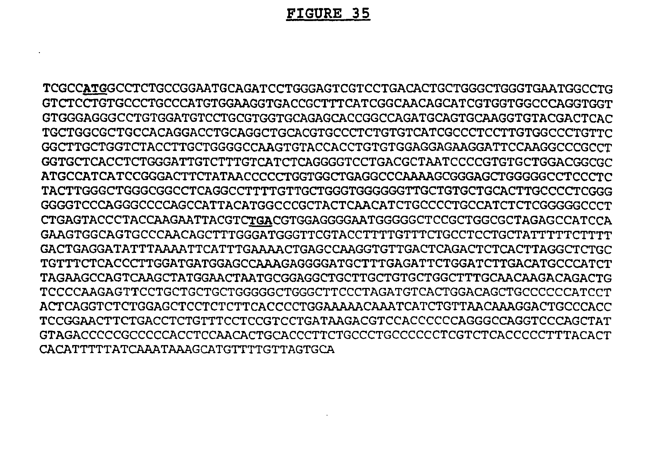

An expressed sequence tag (EST) DNA database (LIFESEQ^{®}, Incyte Pharmaceuticals, Palo Alto, CA) was searched and EST No. 3639112H1 was identified as having homology to CPE-R. EST No. 3639112H1 is designated herein as "DNA69562". EST clone 3639112H1, which was derived from a lung tissue library of a 20-week old fetus who died from Patau's syndrome, was purchased and the cDNA insert was obtained and sequenced in its entirety. The entire nucleotide sequence of PRO1488 is shown in Figure 35 (SEQ ID NO:35), and is designated herein as DNA73736-1657. DNA73736-1657 contains a single open reading frame with an apparent translational initiation site at nucleotide positions 6-8 and a stop codon at nucleotide positions 666-668 (Figure 35; SEQ ID NO:35). The predicted polypeptide precursor is 220 amino acids long.

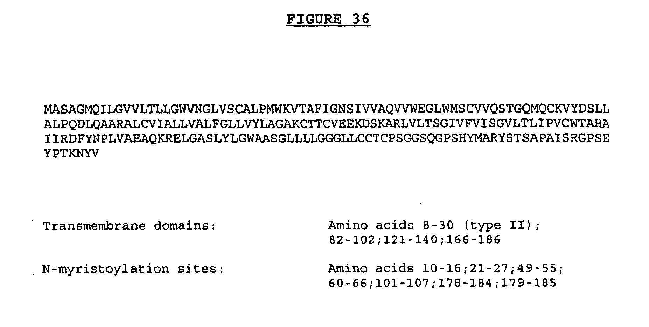

The full-length PRO1488 protein shown in Figure 36 has an estimated molecular weight of about 23,292 daltons and a pl of about 8.43. Four transmembrane domains have been identified as being located at about amino acid positions 8-30, 82-102, 121-140, and 166-186. N-myristoylation sites are at about amino acid positions 10-16. 21-27, 49-55, 60-66, 101-107, 178-184, and 179-185.

An analysis of the Dayhoff database (version 35.45 SwissProt 35), using a WU-BLAST2 sequence alignment analysis of the full-length sequence shown in Figure 36 (SEQ ID NO:36), revealed significant homology between the PRO 1488 amino acid sequence and Dayhoff sequence AB000712_1. Homology was also found between the PRO1488 amino acid sequence and the following additional Dayhoff sequences: AB000714_1, AF007189_1,AF000959_1,P_W63697,MMU82758_1,AF072127_1,AF072128_1,HSU89916_1,AF068863_1, CEAF000418_1, and AF077739_1.

Clone DNA73736-1657 was deposited with the ATCC on November 17, 1998, and is assigned ATCC deposit no. 203466.

### EXAMPLE 22

### Isolation of cDNA Clones Encoding Human PRO3438

DNA82364-2538 was identified by applying the proprietary signal sequence finding algorithm described in Example 3 above. Use of the above described signal sequence algorithm allowed identification of an EST sequence from the LIFESEQ^{®} database, designated Incyte EST187233H1. This EST sequence was then compared to a variety of expressed sequence tag (EST) databases which included public EST databases (*e*.*g*., GenBank) and a proprietary EST DNA database (LIFESEQ^{®}, Incyte Pharmaceuticals, Palo Alto, CA) to identify existing homologies. The homology search was performed using the computer program BLASTor BLAST2 (Altshul et al., Methods in Enzymology, 266:460-480 (1996)). Those comparisons resulting in a BLAST score of 70 (or in some cases, 90) or greater that did not encode known proteins were clustered and assembled into a consensus DNA sequence with the program "phrap" (Phil Green, University of Washington, Seattle, Washington). The consensus sequence obtained therefrom is herein designated as DNA73888.

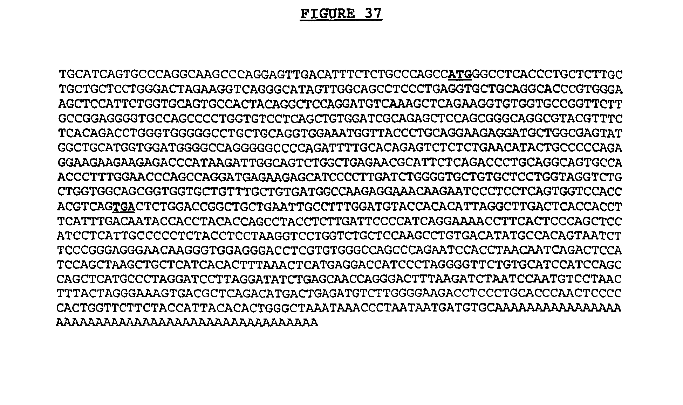

In light of the sequence homology between the DNA73888 consensus sequence and the Incyte EST187233H1, the clone including this EST was purchased and the cDNA insert was obtained and sequenced. The sequence of this cDNA insert is shown in Figure 37 (SEQ ID NO:37) and is herein designated as DNA82364-2538.

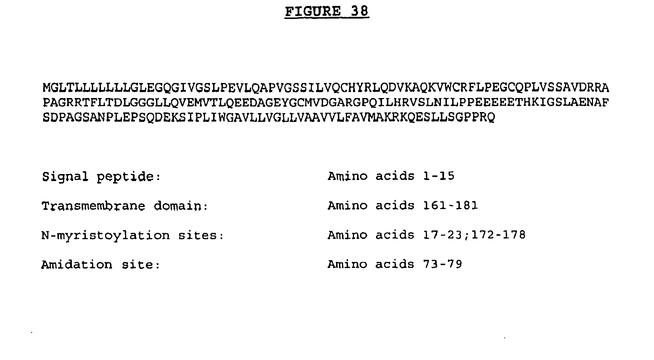

Clone DNA82364-2538 contains a single open reading frame with an apparent translational initiation site at nucleotide positions 50-52 and ending at the stop codon at nucleotide positions 647-649 (Figure 37). The predicted polypeptide precursor is 199 amino acids long (Figure 38; SEQ ID NO:38). The full-length PRO3438 protein shown in Figure 38 has an estimated molecular weight of about 21,323 daltons and a pl of about 5.05. Analysis of the full-length PRO3438 sequence shown in Figure 38 (SEQ ID NO:38) evidences the presence of a variety of important polypeptide domains, wherein the locations given for those important polypeptide domains are approximate as described above. Analysis of the full-length PRO3438 sequence shown in Figure 38 evidences the presence of the following: a signal peptide from about amino acid 1 to about amino acid 15; a transmembrane domain from about amino acid 161 to about amino acid 181; N-myristoylation sites from about amino acid 17 to about amino acid 23 and from about amino acid 172 to about amino acid 178; and an amidation site from about amino acid 73 to about amino acid 79. Clone DNA82364-2538 has been deposited with ATCC on January 20,1999 and is assigned ATCC deposit no. 203603.

An analysis of the Dayhoff database (version 35.45 SwissProt 35), using a WU-BLAST2 sequence alignment analysis of the full-length sequence shown in Figure 38 (SEQ ID NO:38), evidenced homology between the PRO3438 amino acid sequence and the following Dayhoff sequences: S48841, P_W03179, P_W03178, PIGR_HUMAN. HGS_A215. AB001489_1. HGS_B471, P_W61380, P_R15068 and MMLIL_1.

### EXAMPLE 23

### Isolation of cDNA Clones Encoding Human PRO4302

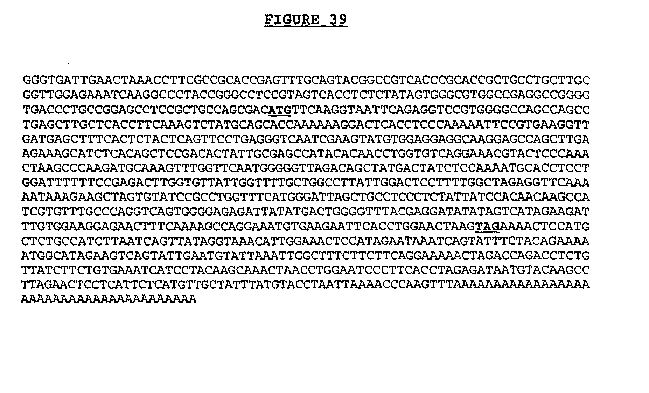

Use of the amylase screen procedure described above in Example 2 on tissue isolated from human tissue resulted in an EST sequence which was then compared against various EST databases to create a consensus sequence by a methodology as described above under the amylase yeast screen procedure and/or the ECD homology procedure. The consensus sequence obtained therefrom is herein designated DNA78875. Based upon an observed homology between the DNA78875 consensus sequence and the Incyte EST no. 2408081H1, Incyte EST no. 2408081 HI was purchased and its insert obtained and sequenced. The sequence of this cDNA insert is shown in Figure 39 (SEQ ID NO:39) and is herein designated as DNA92218-2554 and the derived PRO4302 full-length native sequence protein (SEQ ID NO:40).

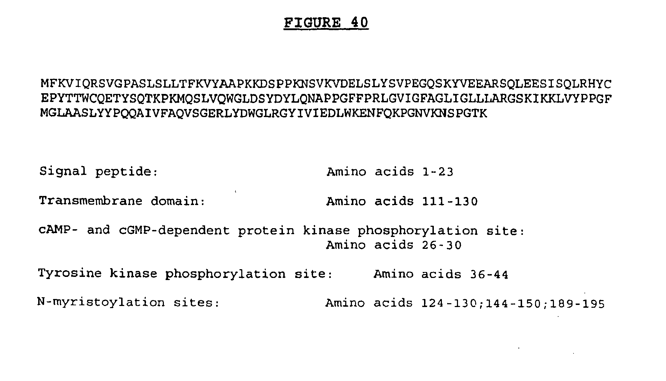

The full length clone DNA92218-2554 (SEQ ID NO:39) shown in Figure 39 has a single open reading frame with an apparent translational initiation site at nucleotide positions 174-176 and a stop signal (TAG) at nucleotide positions 768-770, as indicated by bolded underline. The predicted PRO4302 polypeptide precursor is 198 amino acids long, and has a calculated molecular weight of approximately 22,285 daltons and an estimated pI of approximately 9.35. Analysis of the full-length PRO4302 sequence shown in Figure 40 (SEQ ID NO:40) reveals a signal peptide from about amino acid residue I to about amino acid residue 23; a transmembrane domain from about amino acid residue 111 to about amino acid residue 130; a cAMP- and cGMP-dependent protein kinase phosphorylation site at amino acid residues 26-30; a tyrosine kinase phosphorylation site at amino acid residues 36-44; and N-myristoylation sites at amino acid residues 124-130, 144-150 and 189-195.

A cDNA clone containing DNA92218-2554 was deposited with the ATCC on March 9, 1999 and has been assigned deposit number 203834.

### EXAMPLE 24

### Isolation of cDNA Clones Encoding Human PRO4400

A consensus DNA sequence was assembled relative to other EST sequences using phrap as described in Example 1 above. The EST databases included public EST databases *(e.g.,* GenBank), and a proprietary EST database (LIFESEQ^{®}, Incyte Pharmaceuticals, Palo Alto, CA) and proprietary ESTs from Genentech. This consensus sequence is designated herein as DNA77634. Based on the DNA77634 consensus sequence, oligonucleotides were synthesized: 1) to identify by PCR a cDNA library that contained the sequence of interest, and 2) for use as probes to isolate a clone of the full-length coding sequence for PRO4400.

A pair of PCR primers (forward and reverse) were synthesized:
forward PCR primer:
5'-GCTGCTGCCGTCCATGCTGATG-3' (SEQ ID NO:81)
reverse PCR primer:
5'-CTCGGGGAATGTGACATCGTCGC-3' (SEQ ID NO:82)
Additionally, a synthetic oligonucleotide hybridization probe was constructed from the consensus DNA77634 sequence which had the following nucleotide sequence:
hybridization probe:
5'-GCTGCCGTCCATGCTGATGTTTGCGGTGATCGTGG-3' (SEQ ID NO:83)

RNA for construction of the cDNA libraries was isolated from a human adenocarcinoma cell line. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the Sfil site; *see,* Holmes et al., Science, 253:1278-1280 (1991)) in the unique XhoI and NotI sites.

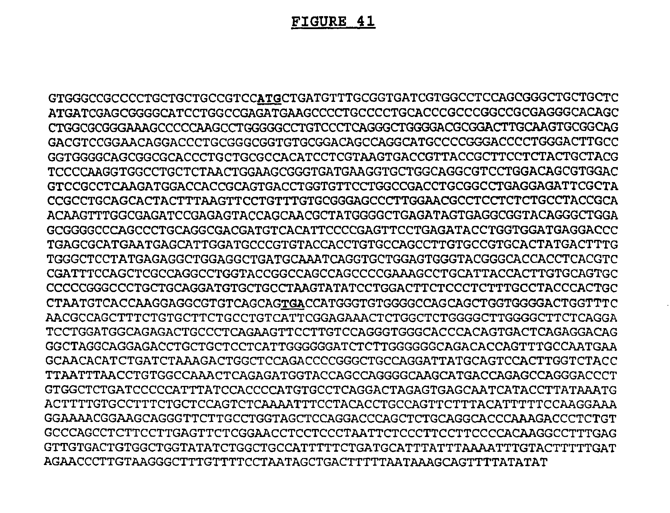

DNA sequencing of the clones isolated as described above gave the full-length DNA sequence for the PRO4400 polypeptide (designated herein as DNA87974-2609 [Figure 41, SEQ ID NO:41]) and the derived protein sequence for that PRO4400 polypeptide.

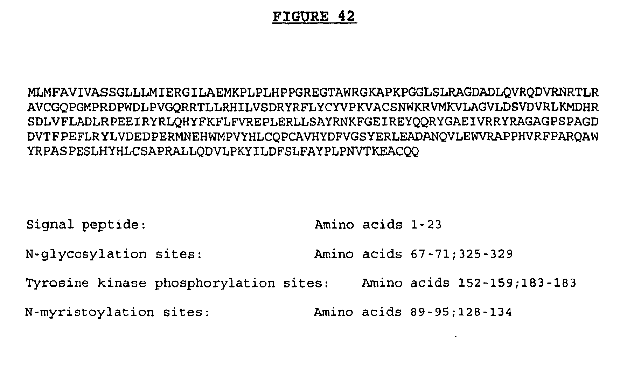

The entire coding sequence of DNA87974-2609 is included in Figure 41 (SEQ ID NO:41). Clone DNA87974-2609 contains a single open reading frame with an apparent translational initiation site at nucleotide positions 27-29, and an apparent stop codon at nucleotide positions 1026-1028. The predicted polypeptide precursor is 333 amino acids long, and has an estimated molecular weight of about 38,618 daltons and a pI of about 9.27. Analysis of the full-length PRO4400 sequence shown in Figure 42 (SEQ ID NO:42) evidences the presence of a variety of important polypeptide domains, wherein the locations given for those important polypeptide domains are approximate as described above. Analysis of the full-length PRO4400 polypeptide shown in Figure 42 evidences the presence of the following: a signal peptide from about amino acid 1 to about amino acid 23; N-gylcosylation sites from about amino acid 67 to about amino acid 71 and from about amino acid 325 to about amino acid 329; tyrosine kinase phosphorylation sites from about amino acid 152 to about amino acid 159 and at about amino acid 183; and N-myristoylation sites from about amino acid 89 to about amino acid 95, and from about amino acid 128 to about amino acid 134. Clone DNA87974-2609 has been deposited with the ATCC on April 27, 1999 and is assigned ATCC deposit no. 203963.

An analysis of the Dayhoff database (version 35.45 SwissProt 35), using a WU-BLAST2 sequence alignment analysis of the full-length sequence shown in Figure 42 (SEQ ID NO:42), evidenced significant homology between the PRO4400 amino acid sequence and the following Dayhoff sequences: AF033827_1, AF070594_1, AF022729_ 1 , CEC34F6_4, SYFB_THETH, G70405, SD_DROME. S64023, ALK1_YEAST and VG04-HSVII.

### EXAMPLE 25

### Isolation of cDNA Clones Encoding Human PRO5725

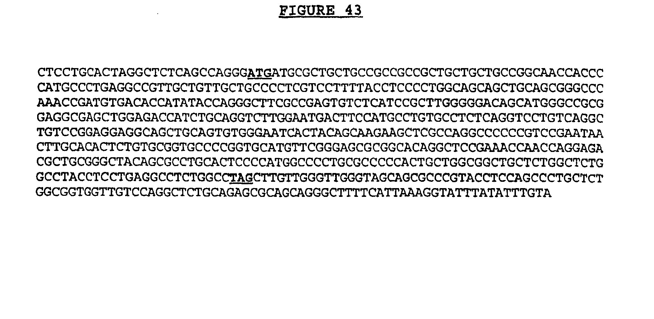

An expressed sequence tag (EST) DNA database (LIFESEQ^{®}, Incyte Pharmaceuticals, Palo Alto, CA) was searched and an EST was identified which showed homology to Neuritin. Incyte ESTclone no. 3705684 was then purchased from LIFESEQ^{®}, Incyte Pharmaceuticals, Palo Alto, CA and the cDNA insert of that clone designated herein as DNA92265-2669 was obtained and sequenced in entirety [Figure 43; SEQ ID NO:43].

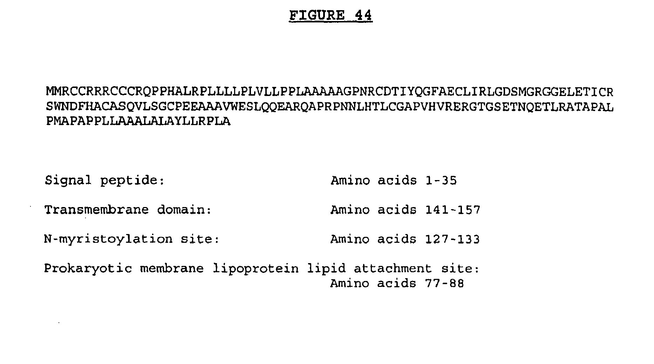

The full-length clone DNA92265-2669 (SEQ ID NO:43) contains a single open reading frame with an apparent translational initiation site at nucleotide positions 27-29 and a stop signal at nucleotide positions 522-524 (Figure 43, SEQ ID NO:43). The predicted polypeptide precursor is 165 amino acids long and has a calculated molecular weight of approximately 17,786 daltons and an estimated pl of approximately 8.43. Analysis of the full-length PRO5725 sequence shown in Figure 44 (SEQ ID NO:44) evidences the presence of a variety of important polypeptide domains as shown in Figure 44, wherein the locations given for those important polypeptide domains are approximate as described above. Analysis of the full-length PR05725 polypeptide shown in Figure 44 evidences the presence of the following: a signal sequence from about amino acid 1 to about amino acid 35; a transmembrane domain from about amino acid 141 to about amino acid 157; an N-myristoylation site from about amino acid 127 to about amino acid 133; and a prokaryotic membrane lipoprotein lipid attachment site from about amino acid 77 to about amino acid 88. Clone DNA92265-2669 has been deposited with ATCC on June 22, 1999 and is assigned ATCC deposit no. PTA-256.

An analysis of the Dayhoff database (version 35.45 SwissProt 35), using a WU-BLAST2 sequence alignment analysis of the full-length sequence shown in Figure 44 (SEQ ID NO:44), evidenced sequence identity between the PRO5725 amino acid sequence and the following Dayhoff sequences: RNU88958_1, P_W37859, P_W37858, JC6305, HGS_RE778, HGS_RE777, P_W27652, P_W44088, HGS_RE776, and HGS_RE425.

### EXAMPLE 26

### In situ Hybridization

*In situ* hybridization is a powerful and versatile technique for the detection and localization of nucleic acid sequences within cell or tissue preparations. It may be useful, for example, to identify sites of gene expression, analyze the tissue distribution of transcription, identify and localize viral infection, follow changes in specific mRNA synthesis, and aid in chromosome mapping.

*In situ* hybridization was performed following an optimized version of the protocol by Lu and Gillett, Cell Vision.1: 169-176 (1994), using PCR-generated ³³P-labeled riboprobes. Briefly,formalin-fixed,paraffin-embedded human tissues were sectioned, deparaffinized, deproteinated in proteinase K (20 g/ml) for 15 minutes at 37°C, and further processed for *in situ* hybridization as described by Lu and Gillett, *supra.* A (³³-P)UTP-labeled antisense riboprobe was generated from a PCR product and hybridized at 55 °C overnight. The slides were dipped in Kodak NTB2™ nuclear track emulsion and exposed for 4 weeks.

### ³³P-Riboprobe synthesis

6.0 *µ*l (125 mCi) of ³³P-UTP (Amersham BF 1002, SA<2000Ci/mmol) were speed-vacuum dried. To each tube containing dried ³³P-UTP, the following ingredients were added:
2.0 µl 5x transcription buffer
1.0 *µ*l DTT (100 mM)
2.0 µl NTP mix (2.5 mM: 10 µl each of 10 mM GTP, CTP & ATP + 10 µl H₂O)
1.0 µl UTP (50 µM)
1.0 µl RNAsin
1.0 µl DNA template (1*µ*g)
1.0*µ*l H₂O
1.0 µl RNA polymerase (for PCR products T3 = AS, T7 = S, usually)

The tubes were incubated at 37°C for one hour. A total of 1.0 µl RQ1 DNase was added, followed by incubation at 37°C for 15 minutes. A total of 90 µl TE (10 mM Tris pH 7.6/l mM EDTA pH 8.0) was added, and the mixture was pipetted onto DE81 paper. The remaining solution was loaded in a MICROCON-50™ ultrafiltration unit, and spun using program 10 (6 minutes). The filtration unit was inverted over a second tube and spun using program 2 (3 minutes). After the final recovery spin, a total of 100 µl TE was added, then 1 *µ*l of the final product was pipetted on DE81 paper and counted in 6 ml of BIOFLUOR II™.

The probe was run on a TBE/urea gel. A total of 1-3 µl of the probe or 5 µl of RNA Mrk III was added to 3 µl of loading buffer. After heating on a 95°C heat block for three minutes, the gel was immediately placed on ice. The wells of gel were flushed, and the sample was loaded and run at 180-250 volts for 45 minutes. The gel was wrapped in plastic wrap (SARAN™ brand) and exposed to XAR film with an intensifying screen in a -70°C freezer one hour to overnight.

### ³³P-Hybridization

### A. Pretreatment of frozen sections

The slides were removed from the freezer, placed on aluminum trays, and thawed at room temperature for 5 minutes. The trays were placed in a 55°C incubator for five minutes to reduce condensation. The slides were fixed for 10 minutes in 4% paraformaldehyde on ice in the fume hood, and washed in 0.5 x SSC for 5 minutes, at room temperature (25 ml 20 x SSC + 975 ml SQ H₂O). After deproteination in 0.5 µg/ml proteinase K for 10 minutes at 37°C (12.5 µl of 10 mg/ml stock in 250 ml prewarmed RNAse-free RNAse buffer), the sections were washed in 0.5 x SSC for 10 minutes at room temperature. The sections were dehydrated in 70%, 95%, and 100% ethanol, 2 minutes each.

### B. Pretreatment of paraffin-embedded sections

The slides were deparaffinized, placed in SQ H₂O, and rinsed twice in 2 x SSC at room temperature, for 5 minutes each time. The sections were deproteinated in 20 µg/ml proteinase K (500 µl of 10 mg/ml in 250 ml RNase-free RNase buffer; 37 °C, 15 minutes) for human embryo tissue, or 8 x proteinase K (100 *µ*l in 250 ml Rnase buffer, 37°C, 30 minutes) for formalin tissues. Subsequent rinsing in 0.5 x SSC and dehydration were performed as described above.

### C. Prehybridization

The slides were laid out in a plastic box lined with Box buffer (4 x SSC. 50% formamide)- saturated filter paper. The tissue was covered with 50 µl of hybridization buffer (3.75 g dextran sulfate + 6 ml SQ H₂O), vortexed, and heated in the microwave for 2 minutes with the cap loosened. After cooling on ice, 18.75 ml formamide, 3.75 ml 20 x SSC, and 9 ml SQ H₂O were added, and the tissue was vortexed well and incubated at 42°C for 1-4 hours.

### D. Hybridization

1.0 x 10⁶ cpm probe and 1.0 *µ*l tRNA (50 mg/ml stock) per slide were heated at 95°C for 3 minutes. The slides were cooled on ice, and 48 *µ*l hybridization buffer was added per slide. After vortexing, 50 µl ³³P mix was added to 50 µl prehybridization on the slide. The slides were incubated overnight at 55°C.

### E. Washes

Washing was done for 2x 10 minutes with 2xSSC, EDTA at room temperature (400 ml 20 x SSC + 16 ml 0.25 M EDTA, V_{f}=4L), followed by RNAseA treatment at 37°C for 30 minutes (500 µl of 10 mg/ml in 250 ml Rnase buffer = 20 µg/ml), The slides were washed 2 x 10 minutes with 2 x SSC, EDTA at room temperature. The stringency wash conditions were as follows: 2 hours at 55 °C. 0.1 x SSC, EDTA (20 ml 20 x SSC + 16 ml EDTA, V_{f}=4L).

### F. Oligonucleotides

*In situ* analysis was performed on four of the DNA sequences disclosed herein. The oligonucleotides employed for these analyses are as follows:
(1) DNA34387-1138 (PRO240) (Jagged/EGF homolog)
   Oligo B-231 W 48mer:
   5'-GGATTCTAATACGACTCACTATAGGGCCCGAGATATGCACCCAATGTC-3' (SEQ ID NO:84)
   Oligo B-231-X 47mer:
   5'-CTATGAAATTAACCCTCACTAAAGGGATCCCAGAATCCCGAAGAACA-3' (SEQ ID NO:85)
(2) DNA57708-1411 (PRO1005) (Novel secreted CA associated protein)
   678.p1:
   5'-GGA TTC TAA TAC GAC TCA CTA TAG GGC CCT CTG TCC ACT GCT TTC GTG-3' (SEQ ID NO:86)
   678.p2:
   5'-CTA TGA AAT TAA CCC TCA CTA AAG GGA GTT CTC CAC CGT GTC TCC ACA-3' (SEQ ID NO:87)
(3) DNA60764-1533 (PRO1265) (Fig-1 Homolog)
   DNA60764-pl:
   5'-GGA TTC TAA TAC GAC TCA CTA TAG GGC CGC GCT GTC CTG CTG TCA CCA-3' (SEQ ID NO:88)
   DNA60764-p2:
   5'-CTA TGA AAT TAA CCC TCA CTA AAG GGA GTT CCC CTC CCC GAG AAG ATA-3' (SEQ ID NO:89)
(4) DNA28498 (PRO183) (FHF-2)
   DNA28498-pl:
   5'-GGA TTC TAA TAC GAC TCA CTA TAG GGC CAG CAA AAG AAG CGG TGG TG-3' (SEQ ID NO:90)
   DNA28498-p2:
   5'-CTA TGA AAT TAA CCC TCA CTA AAG GGA TTC AGC ACG CCA GAG ACA CTT-3'(SEQ ID NO:91)

### G. Results

*In situ* analysis was performed on the above four DNA sequences disclosed herein. The results from these analysis are as follows:
(1) DNA34387-1138 (PRO240) (Jagged/EGF Homolog):
   *Expression pattern in human adult and fetal tissues*
      Elevated signal was observed at the following sites:
      Fetal tissues: thyroid epithelium, small intestinal epithelium, gonad, pancreatic epithelium, hepatocytes in liver and renal tubules; expression was also seen in vascular tissue in developing bones.
      Adult tissues: moderate signal in placental cytotrophoblast, renal tubular epithelium, bladder epithelium, parathyroid and epithelial tumors.
   *Expression in lung adenocarcinoma and squamous carcinoma*
      Expression was observed in all eight squamous carcinomas and in six out of eight adenocarcinomas. Expression was seen in *in-situ* and infiltrating components. Expression levels were low to moderate in the adenocarcinomas. In general, expression was higher in the squamous carcinomas and in two of these the expression was strong. No expression was seen in the tumor stroma, alveoli or normal respiratory epithelium. There was possible low level expression in lymph nodes.
(2) DNA57708-1411 (PRO1005) (Novel secreted CA associated protein):
   Extremely strong expression was seen over mucus neck cells of gastric fundal (chimp) and antral (human) mucosa. These cells are important in proliferation and mucosal regeneration in the stomach. Focal expression was also seen over fetal hepatocytes and adult hepatocytes at the edge of cirrhotic nodules. Possible expression appeared over skeletal muscle of fetal extra-ocular muscles and the lower limb. No significant expression was observed in any of the 16 primary lung carcinomas (eight squamous and eight adenocarcinomas) that were examined.
(3) DNA60764-1533 (PRO1265) (Fig-1 Homolog)
   Fifteen of the sixteen lung tumors examined were suitable for analysis (eight adeno and seven squamous carcinomas). Most of the tumors showed some expression of DNA60764. Expression was largely confined to mononuclear cells adjacent to the infiltrating tumor. In one squamous carcinoma, expression was seen by the malignant epithelium.
   Expression was also seen over cells in the fetal thymic medulla of uncertain histogenesis. Expression was seen over mononuclear cells in damaged renal interstitium and in interstitial cells in a renal cell carcinoma. Expression was also seen over cells in a germinal center, consistent with the fact that most Fig- 1 positive cells are probably inflammatory in origin.
(4) DNA28498 (PRO183) (FHF-2)
   Expression was observed over the inner aspect of the fetal retina. Strong expression was seen over spinal ganglia and over neurones in the anterior horns of the spinal cord of the human fetus. While significant expression was not observed in the human fetal brain, high expression was seen over neurones in rhesus monkey brain, including the hippocampal neurones. Expression was also observed in the spinal cord and developing hindbrain of the rat embryo.

### EXAMPLE 27

### Use of PRO as a Hybridization Probe

The following method describes use of a nucleotide sequence encoding PRO as a hybridization probe.

DNA comprising the coding sequence of full-length or mature PRO as disclosed herein or a fragment thereof is employed as a probe to screen for homologous DNAs (such as those encoding naturally-occurring variants of PRO) in human tissue cDNA libraries or human tissue genomic libraries.

Hybridization and washing of filters containing either library DNAs is performed under the following high-stringency conditions. Hybridization of radiolabeled probe derived from the gene encoding a PRO polypeptide to the filters is performed in a solution of 50% formamide, 5x SSC, 0.1 % SDS, 0.1% sodium pyrophosphate, 50 mM sodium phosphate, pH 6.8,2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence PRO can then be identified using standard techniques known in the art.

### EXAMPLE 28

### Expression of PRO in E. coli

This example illustrates preparation of an unglycosylated form of PRO by recombinant expression in *E. coli.*

The DNA sequence encoding PRO is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli; see,* Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a poly-His leader (including the first six STII codons, poly-His sequence, and enterokinase cleavage site), the PRO coding region, lambda transcriptional terminator, and an argU gene.

The ligation mixture is then used to transform a selected *E. coli* strain using the methods described in Sambrook *et al., supra.* Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO protein can then be purified using a metal chelating column under conditions that allow tight binding of the protein.

PRO may be expressed in *E. coli* in a poly-His tagged form, using the following procedure. The DNA encoding PRO is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA(tonA) Ion galE rpoHts(htpRts) clpP(lacIq). Transformants are first grown in LB containing 50 mg/ml carbenicillin at 30°C with shaking until an OD₆₀₀ of 3-5 is reached. Cultures are then diluted 50-100 fold into CRAP media (prepared by mixing 3.57 g (NH₄)₂SO₄, 0.71 g sodium citrate•2H₂O, 1.07 g KCI, 5.36 g Difco yeast extract. 5.36 g Sheffield hycase SF in 500 ml water, as well as 110 mM MPOS, pH 7.3,0.55% (w/v) glucose and 7 mM MgSO₄) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40.000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni²⁺-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5mM cysteine, 20 mM glycine and 1mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0. 1 % TFA with elution with a gradient of acetonitrile from 10 to 80%. Aliquots of fractions with A₂₈₀ absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

Many of the PRO polypeptides disclosed herein were successfully expressed as descibed above.

### EXAMPLE 29

### Expression of PRO in mammalian cells

This example illustrates preparation of a potentially glycosylated form of PRO by recombinant expression in mammalian cells.

The vector, pRK5 *(see* EP 307,247, published March 15, 1989), is employed as the expression vector. Optionally, the PRO DNA is ligated into pRK5 with selected restriction enzymes to allow insertion of the PRO DNA using ligation methods such as described in Sambrook *et al., supra.* The resulting vector is called pRK5-PRO.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 *µ*g pRK5-PRO DNA is mixed with about 1*µ*g DNA encoding the VA RNA gene [Thimmappaya et al., Cell, 31:543 (1982)] and dissolved in 500 µl of I mM Tris-HCl, 0.1 mM EDTA, 0.227 M CaCl₂. To this mixture is added, dropwise, 500 µl of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml ³⁵S-cysteine and 200 µCi/ml ³⁵S-methionine. After a 12 hour incubation, the conditioned medium is collected, concentrated on a spin filter, and loaded onto a 15% SDS gel. The processed gel may be dried and exposed to film for a selected period of time to reveal the presence of the PRO polypeptide. The cultures containing transfected cells may undergo further incubation (in serum free medium) and the medium is tested in selected bioassays.

In an alternative technique, PRO may be introduced into 293 cells transiently using the dextran sulfate method described by Somparyrac et al., Proc. Natl. Acad. Sci., 12:7575 (1981). 293 cells are grown to maximal density in a spinner flask and 700 µg pRK5-PRO DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and reintroduced into the spinner flask containing tissue culture medium. 5 µg/ml bovine insulin and 0.1 µg/ml bovine transferrin. After about four days, the conditioned media is centrifuged and filtered to remove cells and debris. The sample containing expressed PRO can then be concentrated and purified by any selected method, such as dialysis and/or column chromatography.

In another embodiment, PRO can be expressed in CHO cells. The pRK5-PRO can be transfected into CHO cells using known reagents such as CaPO₄ or DEAE-dextran. As described above, the cell cultures can be incubated, and the medium replaced with culture medium (alone) or medium containing a radiolabel such as ³⁵S-methionine. After determining the presence of a PRO polypeptide, the culture medium may be replaced with serum free medium. Preferably, the cultures are incubated for about 6 days, and then the conditioned medium is harvested. The medium containing the expressed PRO polypeptide can then be concentrated and purified by any selected method.

Epitope-tagged PRO may also be expressed in host CHO cells. The PRO may be subcloned out of the pRK5 vector. The subclone insert can undergo PCR to fuse in frame with a selected epitope tag such as a poly-His tag into a Baculovirus expression vector. The poly-His tagged PRO insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PRO can then be concentrated and purified by any selected method, such as by Ni²⁺-chelate affinity chromatography.

PRO may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (*e.g.,* extracellular domains) of the respective proteins are fused to an 1gG1 constant region sequence containing the hinge, CH2 and CH2 domains and/or as a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used in expression in CHO cells is as described in Lucas et al., Nucl. Acids Res., 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect^{®} (Qiagen), Dosper^{®} or Fugene^{®} (Boehringer Mannheim). The cells are grown as described in Lucas *et al., supra.* Approximately 3 x 10⁷ cells are frozen in an ampule for further growth and production as described below.

The ampules containing the plasmid DNA are thawed by placement into a water bath and mixed by vortexing. The contents are pipetted into a centrifuge tube containing 10 ml of media and centrifuged at 1000 rpm for 5 minutes. The supernatant is aspirated and the cells are resuspended in 10 ml of selective media (0.2 *µ*m filtered PS20 with 5% 0.2 µm diafiltered fetal bovine serum). The cells are then aliquoted into a 100 ml spinner containing 90 ml of selective media. After 1-2 days, the cells are transferred into a 250 ml spinner filled with 150 ml selective growth medium and incubated at 37°C. After another 2-3 days, 250 ml, 500 ml and 2000 ml spinners are seeded with 3 x 10⁵ cells/ml. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x 10⁶ cells/ml. On day 0, the cell number and pH is determined. On day 1, the spinner is sampled and sparging with filtered air is commenced. On day 2. the spinner is sampled, the temperature shifted to 33°C. and 30 ml of 500 g/L glucose and 0.6 ml of 10% antifoam *(e.g.,* 35% polydimethylsiloxane emulsion, Dow Corning 365 Medical Grade Emulsion) taken. Throughout the production, the pH is adjusted as necessary to keep it at around 7.2. After 10 days, or until the viability drops below 70%, the cell culture is harvested by centrifugation and filtering through a 0.22 µm filter. The filtrate is either stored at 4°C or immediately loaded onto columns for purification.

For the poly-His tagged constructs, the proteins are purified using a Ni ²⁺-NTA column (Qiagen). Before purification, imidazole is added to the conditioned media to a concentration of 5 mM. The conditioned media is pumped onto a 6 ml Ni ²⁺-NTA column equilibrated in 20 mM Hepes, pH 7.4. buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which has been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 µl of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

Many of the PRO polypeptides disclosed herein were successfully expressed as descibed above.

### EXAMPLE 30

### Expression of PRO in Yeast

The following method describes recombinant expression of PRO in yeast.

First, yeast expression vectors are constructed for intracellular production or secretion of PRO from the ADH2/GAPDH promoter. DNA encoding PRO and the promoter is inserted into suitable restriction enzyme sites in the selected plasmid to direct intracellular expression of PRO. For secretion, DNA encoding PRO can be cloned into the selected plasmid, together with DNA encoding the ADH2/GAPDH promoter, a native PRO signal peptide or other mammalian signal peptide, or, for example, a yeast alpha-factor or invertase secretory signal/leader sequence, and linker sequences (if needed) for expression of PRO.

Yeast cells, such as yeast strain AB 110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO can subsequently be isolated and purified by removing the yeast cells from the fermentation medium by centrifugation and then concentrating the medium using selected cartridge filters. The concentrate containing PRO may further be purified using selected column chromatography resins.

Many of the PRO polypeptides disclosed herein were successfully expressed as described above.

### EXAMPLE 31

### Expression of PRO in Baculovirus-Infected Insect Cells

The following method describes recombinant expression in Baculovirus-infected insect cells.

The sequence coding for PRO is fused upstream of an epitope tag contained within a baculovirus expression vector. Such epitope tags include poly-His tags and immunoglobulin tags (like Fc regions of IgG). A variety of plasmids may be employed, including plasmids derived from commercially available plasmids such as pVL1393 (Novagen). Briefly, the sequence encoding PRO or the desired portion of the coding sequence of PRO (such as the sequence encoding the extracellular domain of a transmembrane protein or the sequence encoding the mature protein if the protein is extracellular) is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold™ virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-His tagged PRO can then be purified, for example, by Ni²⁺-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 ml Hepes, pH 7.9; 12.5 mM MgCl₂; 0.1 mM EDTA; 10% glycerol; 0.1 % NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 µm filter. A Ni²⁺-NTA agarose column (commercially available from Qiagen) is prepared with a bed volume of 5 ml, washed with 25 ml of water and equilibrated with 25 ml of loading buffer. The filtered cell extract is loaded onto the column at 0.5 ml per minute. The column is washed to baseline A₂₈₀ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching A₂₈₀ baseline again, the column is developed with a 0 to 500 mM imidazole gradient in the secondary wash buffer. One ml fractions are collected and analyzed by SDS-PAGE and silver staining or Western blot with Ni²⁺-NTA-conjugated to alkaline phosphatase (Qiagen). Fractions containing the eluted His₁₀-tagged PRO are pooled and dialyzed against loading buffer.

Alternatively, purification of the IgG tagged (or Fc tagged) PRO can be performed using known chromatography techniques, including for instance, Protein A or protein G column chromatography.

Many of the PRO polypeptides disclosed herein were successfully expressed as described above.

### EXAMPLE 32

### Preparation of Antibodies that Bind PRO

This example illustrates preparation of monoclonal antibodies which can specifically bind PRO.

Techniques for producing the monoclonal antibodies are known in the art and are described, for instance, in Goding, *supra.* Immunogens that may be employed include purified PRO. fusion proteins containing PRO, and cells expressing recombinant PRO on the cell surface. Selection of the immunogen can be made by the skilled artisan without undue experimentation.

Mice, such as Balb/c, are immunized with the PRO immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research. Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO antibodies.

After a suitable antibody titer has been detected, the animals "positive" for antibodies can be injected with a final intravenous injection of PRO. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO. Determination of "positive" hybridoma cells secreting the desired monoclonal antibodies against PRO is within the skill in the art.

The positive hybridoma cells can be injected intraperitoneally into syngeneic Balb/c mice to produce ascites containing the anti-PRO monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 33

### Purification of PRO Polypeptides Using Specific Antibodies

Native or recombinant PRO polypeptides may be purified by a variety of standard techniques in the art of protein purification. For example, pro-PRO polypeptide, mature PRO polypeptide, or pre-PRO polypeptide is purified by immunoaffinity chromatography using antibodies specific for the PRO polypeptide of interest. In general, an immunoaffinity column is constructed by covalently coupling the anti-PRO polypeptide antibody to an activated chromatographic resin.

Polyclonal immunoglobulins are prepared from immune sera either by precipitation with ammonium sulfate or by purification on immobilized Protein A (Pharmacia LKB Biotechnology. Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE™ (Pharmacia LKB Biotechnology). The antibody is coupled to the resin, the resin is blocked, and the derivative resin is washed according to the manufacturer's instructions.

Such an immunoaffinity column is utilized in the purification of the PRO polypeptide by preparing a fraction from cells containing the PRO polypeptide in a soluble form. This preparation is derived by solubilization of the whole cell or of a subcellular fraction obtained via differential centrifugation by the addition of detergent or by other methods well known in the art. Alternatively, soluble PRO polypeptide containing a signal sequence may be secreted in useful quantity into the medium in which the cells are grown.

A soluble PRO polypeptide-containing preparation is passed over the immunoaffinity column, and the column is washed under conditions that allow the preferential absorbance of the PRO polypeptide (*e.g.*, high ionic strength buffers in the presence of detergent). Then, the column is eluted under conditions that disrupt antibody/PRO polypeptide binding *(e.g.,* a low pH buffer such as approximately pH 2-3, or a high concentration of a chaotrope such as urea or thiocyanate ion), and the PRO polypeptide is collected.

### EXAMPLE 34

### Drug Screening

This invention is particularly useful for screening compounds by using PRO polypeptides or a binding fragment thereof in any of a variety of drug screening techniques. The PRO polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO polypeptide or fragment. Drugs are screened against such transformed cells in competitive binding assays. Such cells, either in viable or fixed form, can be used for standard binding assays. One may measure, for example, the formation of complexes between a PRO polypeptide or a fragment and the agent being tested. Alternatively, one can examine the diminution in complex formation between the PRO polypeptide and its target cell or target receptors caused by the agent being tested.

Thus, the present invention provides methods of screening for drugs or any other agents which can affect a PRO polypeptide-associated disease or disorder. These methods comprise contacting such an agent with a PRO polypeptide or fragment thereof and assaying (i) for the presence of a complex between the agent and the PRO polypeptide or fragment, or (ii) for the presence of a complex between the PRO polypeptide or fragment and the cell, by methods well known in the art. In such competitive binding assays, the PRO polypeptide or fragment is typically labeled. After suitable incubation, the free PRO polypeptide or fragment is separated from that present in bound form, and the amount of free or uncomplexed label is a measure of the ability of the particular agent to bind to the PRO polypeptide or to interfere with the PRO polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO polypeptide, the peptide test compounds are reacted with the PRO polypeptide and washed. Bound PRO polypeptide is detected by methods well known in the art. Purified PRO polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding a PRO polypeptide specifically compete with a test compound for binding to the PRO polypeptide or fragments thereof. In this manner, the antibodies can be used to detect the presence of any peptide which shares one or more antigenic determinants with a PRO polypeptide.

### EXAMPLE 35

### Rational Drug Design

The goal of rational drug design is to produce structural analogs of a biologically active polypeptide of interest (*i.e.*, a PRO polypeptide) or of small molecules with which they interact, *e.g.,* agonists, antagonists, or inhibitors. Any of these examples can be used to fashion drugs which are more active or stable forms of the PRO polypeptide or which enhance or interfere with the function of the PRO polypeptide *in vivo* (*c.f.,* Hodgson, Bio/Technology, 9: 19-21 (1991)).

In one approach, the three-dimensional structure of the PRO polypeptide, or of a PRO polypeptide-inhibitor complex, is determined by x-ray crystallography, by computer modeling or, most typically, by a combination of the two approaches. Both the shape and charges of the PRO polypeptide must be ascertained to elucidate the structure and to determine active site(s) of the molecule. Less often, useful information regarding the structure of the PRO polypeptide may be gained by modeling based on the structure of homologous proteins. In both cases, relevant structural information is used to design analogous PRO polypeptide-like molecules or to identify efficient inhibitors. Useful examples of rational drug design may include molecules which have improved activity or stability as shown by Braxton and Wells, Biochemistry, 31:7796-7801 (1992) or which act as inhibitors, agonists, or antagonists of native peptides as shown by Athauda et al., J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO polypeptide may be made available to perform such analytical studies as X-ray crystallography. In addition, knowledge of the PRO polypeptide amino acid sequence provided herein will provide guidance to those employing computer modeling techniques in place of or in addition to x-ray crystallography.

### EXAMPLE 36

### In Vitro Antitumor Assay

The antiproliferative activityof the PRO240, PR0381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO4400, PRO5725, PRO183, PRO202, PRO542, PRO861, PRO1096 or PRO3562 polypeptide was determined in the investigational, disease-oriented *in vitro* anti-cancer drug discovery assay of the National Cancer Institute (NCI), using a sulforhodamine B (SRB) dye binding assay essentially as described by Skehan et al., J. Natl. Cancer Inst., 82:1107-1112 (1990). The 60 tumor cell lines employed in this study ("the NCl panel"), as well as conditions for their maintenance and culture *in vitro* have been described by Monks et al., J. Natl. Cancer Inst., 83:757-766 (1991). The purpose of this screen is to initially evaluate the cytotoxic and/or cytostatic activity of the test compounds against different types of tumors (Monks *et al., supra:* Boyd, Cancer: Princ. Pract. Oncol. Update, 3(10):1-12 [1989]).

Cells from approximately 60 human tumor cell lines were harvested with trypsin/EDTA (Gibco), washed once, resuspended in IMEM and their viability was determined. The cell suspensions were added by pipet (100 *µ*l volume) into separate 96-well microtiter plates. The cell density for the 6-day incubation was less than for the 2-day incubation to prevent overgrowth. Inoculates were allowed a preincubation period of 24 hours at 37°C for stabilization. Dilutions at twice the intended test concentration were added at time zero in 100 *µ*l aliquots to the microtiter plate wells (1:2 dilution). Test compounds were evaluated at five half-log dilutions (1000 to 100,000-fold). Incubations took place for two days and six days in a 5% CO₂ atmosphere and 100% humidity.

After incubation, the medium was removed and the cells were fixed in 0.1 ml of 10% trichloroacetic acid at 40°C. The plates were rinsed five times with deionized water, dried, stained for 30 minutes with 0.1 ml of 0.4% sulforhodamine B dye (Sigma) dissolved in 1% acetic acid, rinsed four times with 1% acetic acid to remove unbound dye, dried, and the stain was extracted for five minutes with 0.1 ml of 10 mM Tris base [tris(hydroxymethyl)aminomethane], pH 10.5. The absorbance (OD) of sulforhodamine B at 492 nm was measured using a computer-interfaced, 96-well microtiter plate reader.

A test sample is considered positive if it shows at least 40% growth inhibitory effect at one or more concentrations. The results are shown in the following Table 7, where the tumor cell type abbreviations are as follows:
NSCL = non-small cell lung carcinoma; CNS = central nervous system

**Table 7**

| Test compound | Days | Tumor Cell Line Type | Cell Line Designation |
|---|---|---|---|
| PR0240 | 6 | Leukemia | MOLT-4; RPMI-8226 |
| PRO240 | 6 | Colon Cancer | COLO 205; HCT-116; KM12 |
| PRO240 | 6 | Breast Cancer | MDA-MB-435 |
| PRO240 | 6 | NSCL | NCl-H322M; HOP-92 |
| PRO240 | 6 | Prostate Cancer | DU-145 |
| PRO240 | 6 | CNS-Cancer | SNB-75 |
| PRO240 | 2 | Leukemia | RPM1-8226 |
| PRO240 | 2 | NSCL | HOP92 |
| PRO240 | 2 | CNS Cancer | SF-539 |
| PRO240 | 2 | Breast Cancer | NCUADR-RES* |
| PRO240 | 2 | Leukemia | HL-60(TB) |
| PRO240 | 2 | Breast Cancer | MDA-MB-231/ATCC |
| PRO240 | N/A | Leukemia | HL-60(TB); MOLT-4 |
| PRO240 | N/A | Leukemia | RPMI-8226 |
| PRO381 | N/A | NSCL | A549/ATCC; EKVX; HOP-62* |
| PRO381 | N/A | NSCL | NCI-H226*; NCI-H23 |
| PRO381 | N/A | NSCL | NCI-H322M; NCI-H460* |
| PRO381 | N/A | NSCL | NCI-H522* |
| PRO381 | N/A | NSCL | HOP-92* |
| PRO381 | N/A | Colon Cancer | COLO 205*; HCC-2998* |
| PRO381 | N/A | Colon Cancer | HCT-116; HCT-15; HT29 |
| PRO381 | N/A | Colon Cancer | SW620; KM12 |
| PRO381 | N/A | Breast Cancer | BT-549*; HS 578T*; MCF7 |
| PRO381 | N/A | Breast Cancer | MDA-MB-435; MDA-N |
| PRO381 | N/A | Breast Cancer | T-47D; MDA-MB-231/ATCC |
| PRO381 | N/A | Ovarian Cancer | IGROVI*; OVCAR-3 |
| PRO381 | N/A | Ovarian Cancer | OVCAR-5*; OVCAR-8 |
| PRO381 | N/A | Ovarian Cancer | SK-OV-3; OVCAR-4 |
| PRO381 | N/A | Leukemia | CCRF-CEM*; HL-60(TB)* |
| PRO381 | N/A | Leukemia | K-562; MOLT-4 |
| PRO381 | N/A | Renal Cancer | 786-0*; A498; ACHN; CAKI-1 |
| PRO381 | N/A | Renal Cancer | RXF 393; SN 12C; TK-10* |
| PRO381 | N/A | Renal Cancer | UO-31* |
| PRO381 | N/A | Melanoma | LOX IMVI*; M14 |
| PRO381 | N/A | Melanoma | MALME-3M; UACC-62* |
| PRO381 | N/A | Melanoma | SK-MEL-2; SK-MEL-28 |
| PRO381 | N/A | Melanoma | SK-MEL-5; UACC-257 |
| PRO381 | N/A | Prostate Cancer | DU-145; PC-3* |
| PRO381 | N/A | CNS Cancer | SF-268; SNB-19*; U251* |
| PRO381 | N/A | CNS Cancer | SF-295; S-539; SNB-75 |
| PRO534 | N/A | NSCL | A549/ATCC: HOP-62; HOP-92 |
| PRO534 | N/A | NSCL | NCI-H23; NCI-H322M |
| PRO534 | N/A | NSCL | NCI-H460; NCI-H522 |
| PRO534 | N/A | NSCL | NCI-H266 |
| PRO534 | N/A | Colon Cancer | HCT-116; HT29; KM12 |
| PRO534 | N/A | Colon Cancer | SW620 |
| PR0534 | N/A | Breast Cancer | BT-549; HS 578T; MCF7 |
| PRO534 | N/A | Breast Cancer | MDA-MB-231/ATCC |
| PRO534 | N/A | Breast Cancer | MDA-MB-435: MDA-N |
| PRO534 | N/A | Breast Cancer | T-47D |
| PRO534 | N/A | Ovarian Cancer | IGROVI; OVCAR-3* |
| PRO534 | N/A | Ovarian Cancer | OVCAR-8 |
| PRO534 | N/A | Leukemia | HL-60(TB); K-562; MOLT-4* |
| PRO534 | N/A | Leukemia | RPMI-8226*: CCRF-CEM |
| PRO534 | N/A | Renal Cancer | 786-0; A498: ACHN; RXF 393 |
| PRO534 | N/A | Renal Cancer | SN 12C; TK-10 |
| PRO534 | N/A | Melanoma | LOX IMVI; M14; SK-MEL-28 |
| PRO534 | N/A | Melanoma | SK-MEL-2; UACC-257 |
| PRO534 | N/A | Melanoma | UACC-62 |
| PRO534 | N/A | Prostate Cancer | DU-145 |
| PRO534 | N/A | CNS Cancer | SF-268; SF-295; SNB-19 |
| PRO534 | N/A | CNS Cancer | SNB-75 |
| PRO540 | 6 | Leukemia | HL-60(TB) |
| PRO540 | 6 | Colon Cancer | KM12 |
| PRO540 | 6 | CNS Cancer | SF-295 |
| PRO540 | 6 | Melanoma | LOX IMVI |
| PRO540 | 6 | Ovarian Cancer | SK-OV-3 |
| PRO540 | 6 | Renal Cancer | 786-0; CAKI-1; UO-31; TK-10 |
| PRO540 | 2 | NSCL | HOP-62; HOP-92; NCl-H460 |
| PRO540 | 2 | Colon Cancer | HCC-2998 |
| PRO540 | 2 | CNS Cancer | SF-295; SN-75 |
| PRO540 | 2 | Ovarian Cancer | SK-OV-3 |
| PRO698 | N/A | NSCL | EKVX; HOP-62; NCI-H322M |
| PRO698 | N/A | NSCL | NCI-H522 |
| PRO698 | N/A | Colon Cancer | HCT-116 |
| PR0698 | N/A | Breast Cancer | MDA-MB-231/ATCC |
| PR0698 | N/A | Breast Cancer | MDA-MB-435; MDA-N |
| PRO698 | N/A | Ovarian Cancer | OVCAR-3; OVCAR-4 |
| PRO698 | N/A | Ovarian Cancer | OVCAR-5;OVCAR-8 |
| PRO698 | N/A | Ovarian Cancer | SK-OV-3 |
| PRO698 | N/A | Renal Cancer | ACHN; RXF 393; SN 12C |
| PRO698 | N/A | Renal Cancer | TK-10 |
| PRO698 | N/A | CNS Cancer | SF-268; SF-295; SNB-19 |
| PRO698 | N/A | CNS Cancer | SNB-75*; U251 |
| PRO982 | 6 | NSCL | HOP-62 |
| PRO982 | 6 | Leukemia | CCRF-CEM; RPMI-8226 |
| PRO982 | 6 | Melanoma | LOX IMVI |
| PRO982 | N/A | NSCL | HOP-92; NCI-H522 |
| PRO982 | N/A | Colon Cancer | COLO 205 |
| PRO982 | N/A | Breast Cancer | BT-549; MDA-MB-231/ATCC |
| PRO982 | N/A | Ovarian Cancer | IGROVI; OVCAR-5 |
| PRO982 | N/A | Leukemia | MOLT-4; RPMI-8226 |
| PRO982 | N/A | Renal Cancer | 786-0; CAKI-1; RXF 393 |
| PRO982 | N/A | Renal Cancer | TK-10 |
| PRO982 | N/A | Prostate Cancer | PC-3 |
| PRO982 | N/A | CNS Cancer | SNB-19; U251 |
| PRO1005 | N/A | NSCL | A549/ATCC |
| PRO1005 | N/A | Renal Cancer | TK-10 |
| PRO1005 | N/A | CNS Cancer | SNB-19 |
| PRO1007 | 6 | Leukemia | CCRF-CEM |
| PRO1007 | 6 | Colon Cancer | HCT-116; KM 12 |
| PRO1007 | N/A | NSCL | NCI-H522 |
| PRO1007 | N/A | Colon Cancer | KM12 |
| PRO1007 | N/A | Breast Cancer | HS 578T |
| PRO1007 | N/A | Breast Cancer | MDA-MB-231/ATCC |
| PRO1007 | N/A | Ovarian Cancer | IGROVI |
| PRO1007 | N/A | Leukemia | RPMI-8226 |
| PRO1007 | N/A | Melanoma | SK-MEL-628 |
| PRO1131 | N/A | Leukemia | MOLT-4; CCRF-CEM |
| PRO1131 | N/A | NSCL | HOP-62; NCI-H23; NCI-H522 |
| PRO1131 | N/A | Breast Cancer | MCF7; MDA-MB-231/ATCC |
| PRO1131 | N/A | Ovarian Cancer | OVCAR-3; OVCAR-4 |
| PRO1131 | N/A | Ovarian Cancer | OVCAR-8 |
| PRO1131 | N/A | Melanoma | LOX IMVI; UACC-257 |
| PRO1131 | N/A | CNS Cancer | SNB-19; U251 |
| PRO1157 | N/A | NSCL | A549/ATCC; HOP-62* |
| PRO1157 | N/A | NSCL | HOP-92*; NCI-H23 |
| PRO1157 | N/A | NSCL | NCI-H322M; NCI-H522* |
| PRO1157 | N/A | NSCL | NCI-H226 |
| PRO1157 | N/A | Colon Cancer | COLO 205; HCT-116; HCT-15 |
| PRO1157 | N/A | Colon Cancer | HT29; KM12*; SW620 |
| PRO1157 | N/A | Breast Cancer | BT-549*; HS 578T; MCF7 |
| PRO1157 | N/A | Breast Cancer | MDA-MB-231/ATCC* |
| PRO1157 | N/A | Breast Cancer | MDA-MB-435; MDA-N |
| PRO1157 | N/A | Ovarian Cancer | IGROVI; OVCAR-3* |
| PRO1157 | N/A | Ovarian Cancer | OVCAR-5*; OVCAR-8 |
| PRO1157 | N/A | Ovarian Cancer | SK-OV-3 |
| PRO1157 | N/A | Leukemia | HL-60(TB); K-562; MOLT-4 |
| PRO1157 | N/A | Leukemia | RPMI-8226; CCRF-CEM; SR |
| PRO1157 | N/A | Renal Cancer | 786-0*; A498; ACHN |
| PRO1157 | N/A | Renal Cancer | CAKI-1*; RXF 393*; TK-10* |
| PRO1157 | N/A | Renal Cancer | UO-31* |
| PRO1157 | N/A | Melanoma | LOX IMVI; M14 |
| PRO1157 | N/A | Melanoma | MALME-3M; SK-MEL-28 |
| PRO1157 | N/A | Melanoma | SK-MEL-2; SK-MEL-5 |
| PRO1157 | N/A | Melanoma | UACC-257; UACC-62 |
| PRO1157 | N/A | Prostate Cancer | DU-145; PC-3* |
| PRO1157 | N/A | CNS Cancer | SF-268; SF-295: S-539* |
| PRO1157 | N/A | CNS Cancer | SNB-19; SNB-75*; U251* |
| PRO1199 | N/A | NSCL | A549/ATCC; HOP-62 |
| PRO1199 | N/A | NSCL | HOP-92; NCI-H23 |
| PRO1199 | N/A | NSCL | NCI-H322M; NCI-H522 |
| PRO1199 | N/A | Colon Cancer | HCC-2998*; SW620 |
| PRO1199 | N/A | Breast Cancer | HS 578T; MCF7 |
| PRO1199 | N/A | Breast Cancer | MDA-MB-435; MDA-N |
| PRO1199 | N/A | Breast Cancer | T-47D |
| PRO1199 | N/A | Ovarian Cancer | IGROVI; OVCAR-3 |
| PRO1199 | N/A | Ovarian Cancer | OVCAR-4; OVCAR-5 |
| PRO1199 | N/A | Ovarian Cancer | OVCAR-8 |
| PRO1199 | N/A | Leukemia | CCRF-CEM*; RPMI-8226* |
| PRO1199 | N/A | Renal Cancer | RXF 393; SN 12C; UO-31 |
| PRO1199 | N/A | Melanoma | LOX IMVI; M14; SK-MEL-28 |
| PRO1199 | N/A | Melanoma | UACC-257 |
| PRO1199 | N/A | Prostate Cancer | PC-3 |
| PRO1199 | N/A | CNS Cancer | SNB-19; SNB-75; U251 |
| PRO1265 | N/A | NSCL | EKVX |
| PRO1265 | N/A | Colon Cancer | COLO 205; SW620 |
| PRO1265 | N/A | Breast Cancer | HS 578T; MCF7 |
| PRO1265 | N/A | Breast Cancer | MDA-MB-231/ATCC |
| PRO1265 | N/A | Breast Cancer | MDA-MB-435; MDA-N* |
| PRO1265 | N/A | Breast Cancer | BT 549 |
| PRO1265 | N/A | Ovarian Cancer | IGROVI; OVCAR-3 |
| PRO1265 | N/A | Ovarian Cancer | OVCAR-4; OVCAR-5 |
| PRO1265 | N/A | Ovarian Cancer | OVCAR-8 |
| PRO1265 | N/A | Leukemia | CCRF-CEM*; HL-60(TB) |
| PRO1265 | N/A | Leukemia | K-562; MOLT-4; RPMI-8226 |
| PRO1265 | N/A | Renal Cancer | ACHN; CAKI-1; SN 12C |
| PRO1265 | N/A | Renal Cancer | RXF-393 |
| PRO1265 | N/A | Melanoma | LOX IMVI; UACC-257 |
| PRO1265 | N/A | CNS Cancer | SF-295; SNB-19; U251 |
| PRO1286 | N/A | NSCL | A549/ATCC; EKVX; HOP-92 |
| PRO1286 | N/A | NSCL | NCI-H23; NCI-H322M |
| PRO1286 | N/A | NSCL | NCI-H522*; NCI-H226* |
| PRO1286 | N/A | Colon Cancer | HCT-116*; SW620 |
| PRO1286 | N/A | Breast Cancer | BT-549; MCF7; MDA-N |
| PRO1286 | N/A | Breast Cancer | NCI/ADR-RES*; T-47D |
| PRO1286 | N/A | Ovarian Cancer | OVCAR-4*; OVCAR-5* |
| PRO1286 | N/A | Ovarian Cancer | OVCAR-8*: SK-OV-3 |
| PRO1286 | N/A | Renal Cancer | 786-0; ACHN: CAKI-1 |
| PRO1286 | N/A | Renal Cancer | RXF 393*; SN 12C*; TK-10* |
| PRO1286 | N/A | Melanoma | LOX IMVI; SK-MEL-2 |
| PRO1286 | N/A | Melanoma | SK-MEL-5: UACC-257 |
| PRO1286 | N/A | Melanoma | MEL-14 |
| PRO1286 | N/A | Prostate Cancer | PC-3 |
| PRO1286 | N/A | CNS Cancer | SF-268; SF-295; S-539* |
| PRO1286 | N/A | CNS Cancer | SNB-19; SNB-75* |
| PRO1313 | N/A | NSCL | A549/ATCC; HOP-62; HOP-92 |
| PRO1313 | N/A | NSCL | NCI-H23; NCI-H322M |
| PRO1313 | N/A | NSCL | NCI-H460; NCI-H522 |
| PRO1313 | N/A | NSCL | NCI-H226 |
| PRO1313 | N/A | Colon Cancer | COLO 205; HCT-116; HCT-15 |
| PRO1313 | N/A | Colon Cancer | HT29; SW620 |
| PRO1313 | N/A | Breast Cancer | BT-549*; HS 578T; MCF7 |
| PRO1313 | N/A | Breast Cancer | MDA-MB-231/ATCC |
| PRO1313 | N/A | Breast Cancer | MDA-MB-435; MDA-N |
| PRO1313 | N/A | Breast Cancer | NCI/ADR-RES |
| PRO1313 | N/A | Ovarian Cancer | IGROVI*; OVCAR-3 |
| PRO1313 | N/A | Ovarian Cancer | OVCAR-4; OVCAR-5 |
| PRO1313 | N/A | Ovarian Cancer | OVCAR-8; SK-OV-3 |
| PRO1313 | N/A | Leukemia | CCRF-CEM: HL-60(TB)* |
| PRO1313 | N/A | Leukemia | K-562; MOLT-4; RPMI-8226 |
| PRO1313 | N/A | Renal Cancer | 786-0; A498: ACHN; CAKI-1 |
| PRO1313 | N/A | Renal Cancer | RXF 393; SN 12C; TK-10* |
| PRO1313 | N/A | Renal Cancer | UO-31 |
| PRO1313 | N/A | Melanoma | LOX IMVI: M 14: MALME-3M |
| PRO1313 | N/A | Melanoma | SK-MEL-28; SK-MEL-5 |
| PRO1313 | N/A | Melanoma | UACC-257: SK-MEL-2 |
| PRO1313 | N/A | Prostate Cancer | DU-145; PC-3 |
| PR01313 | N/A | CNS Cancer | SF-268; SF-295; SNB-19 |
| PR01313 | N/A | CNS Cancer | U251 * |
| PRO1338 | N/A | NSCL | A549/ATCC; EKVX*; HOP-62 |
| PRO1338 | N/A | NSCL | HOP-92*; NCI-H226* |
| PRO1338 | N/A | NSCL | NCI-H23*; NCI-H322M |
| PRO1338 | N/A | NSCL | NCI-460: NCI-H522* |
| PRO1338 | N/A | Colon Cancer | HCC-2998*; HCT-116* |
| PRO1338 | N/A | Colon Cancer | HCT-15; HT29; SW620 |
| PRO1338 | N/A | Breast Cancer | BT-549*; MCF7 |
| PRO1338 | N/A | Breast Cancer | MDA-MB-435; MDA-N |
| PRO1338 | N/A | Breast Cancer | NCI/ADR-RES* |
| PRO1338 | N/A | Ovarian Cancer | OVCAR-3; OVCAR-4* |
| PRO1338 | N/A | Ovarian Cancer | OVCAR-5*: OVCAR-8* |
| PRO1338 | N/A | Ovarian Cancer | SK-OV-3 |
| PRO1338 | N/A | Renal Cancer | 786-0: ACHN; CAKI-1 |
| PRO1338 | N/A | Renal Cancer | RXF-393*; SN 12C*; TK-10* |
| PRO1338 | N/A | Renal Cancer | UO-31 * |
| PRO1338 | N/A | Melanoma | LOX IMVI; M 14; SK-MEL-2* |
| PRO1338 | N/A | Melanoma | SK-MEL-5*; UACC-257* |
| PRO1338 | N/A | Melanoma | UACC-62 |
| PRO1338 | N/A | Prostate Cancer | DU-145; PC-3 |
| PRO1338 | N/A | CNS Cancer | SF-268; SF-295; S-539* |
| PRO1338 | N/A | CNS Cancer | SNB-19; SNB-75*; U251* |
| PRO1375 | N/A | NSCL | NCI-H23 |
| PRO1375 | N/A | Colon Cancer | SW620 |
| PRO1375 | N/A | Breast Cancer | NCI/ADR-RES |
| PRO1375 | N/A | Ovarian Cancer | OVCAR-5 |
| PRO1375 | N/A | Renal Cancer | SN 12C |
| PRO1375 | N/A | Melanoma | LOX IMVI |
| PRO1375 | N/A | CNS Cancer | SF-268 |
| PRO1410 | N/A | Renal Cancer | UO-31 |
| PRO1410 | N/A | NSCL | NCI-H522 |
| PRO1410 | N/A | Colon Cancer | HCC-2998; KM12 |
| PRO1410 | N/A | Ovarian Cancer | IGROVI |
| PRO1410 | N/A | Leukemia | SR |
| PRO1410 | N/A | Renal Cancer | A498*; TK-10 |
| PRO1410 | N/A | Melanoma | LOX IMVI |
| PRO1410 | N/A | CNS Cancer | S-539 |
| PRO1488 | N/A | NSCL | A549/ATCC*; EKVX* |
| PRO1488 | N/A | NSCL | HOP-62; HOP-92; NCI-H23 |
| PRO1488 | N/A | NSCL | NCI-H322M; NCI-H460 |
| PRO1488 | N/A | NSCL | NCI-H522; NCI-H226 |
| PRO1488 | N/A | Colon Cancer | COLO 205; HCC-2998 |
| PRO1488 | N/A | Colon Cancer | HCT-1 16*; HCT-15 |
| PRO1488 | N/A | Colon Cancer | KM12: SW620 |
| PRO1488 | N/A | Breast Cancer | HS 578T |
| PRO1488 | N/A | Breast Cancer | MDA-MB-231/ATCC |
| PRO1488 | N/A | Breast Cancer | MDA-MB-435; MDA-N |
| PRO1488 | N/A | Breast Cancer | NCI/ADR-RES; T-47D |
| PRO1488 | N/A | Breast Cancer | BT-549 |
| PRO1488 | N/A | Ovarian Cancer | IGROVI; OVCAR-3 |
| PRO1488 | N/A | Ovarian Cancer | OVCAR-4; OVCAR-5* |
| PRO1488 | N/A | Ovarian Cancer | OVCAR-8; SK-OV-3 |
| PRO1488 | N/A | Leukemia | CCRF-CEM; K-562 |
| PRO1488 | N/A | Leukemia | RPMI-8226; SR |
| PRO1488 | N/A | Renal Cancer | 786-0*; A498; ACHN; CAKI-1 |
| PRO1488 | N/A | Renal Cancer | RXF 393; SN 12C*; TK-10* |
| PRO1488 | N/A | Renal Cancer | UO-31 |
| PRO1488 | N/A | Melanoma | LOX IMVI; M14; SK-MEL-2* |
| PRO1488 | N/A | Melanoma | SK-MEL-28; SK-MEL-5 |
| PRO1488 | N/A | Melanoma | UACC-257*; UACC-62* |
| PRO1488 | N/A | Prostate Cancer | DU-145; PC-3* |
| PRO1488 | N/A | CNS Cancer | SF-268; SF-295; S-539 |
| PRO1488 | N/A | CNS Cancer | SNB-19; SNB-75 |
| PRO3438 | N/A | Colon Cancer | HCC-2998; KM12 |
| PRO3438 | N/A | Leukemia | SR |
| PRO3438 | N/A | Renal Cancer | RXF 393; TK-10 |
| PRO3438 | N/A | Prostate Cancer | PC-3 |
| PRO3438 | N/A | Ovarian Cancer | IGROVI |
| PRO4302 | N/A | NSCL | A549/ATCC*; EKVX* |
| PRO4302 | N/A | NSCL | HOP-62; HOP-92*; NCI-H23 |
| PRO4302 | N/A | NSCL | NCI-H322M; NCI-H460 |
| PRO4302 | N/A | NSCL | NCI-H522 |
| PRO4302 | N/A | Colon Cancer | COLO 205; HCC-2998 |
| PR04302 | N/A | Colon Cancer | HCT-15; KM12; SW620 |
| PR04302 | N/A | Breast Cancer | BT-549*; HS 578T |
| PRO4302 | N/A | Breast Cancer | MDA-MB-435: MDA-N |
| PRO4302 | N/A | Breast Cancer | NCI/ADR-RES;T-47D |
| PRO4302 | N/A | Ovarian Cancer | IGROVI; OVCAR-3 |
| PRO4302 | N/A | Ovarian Cancer | OVCAR-4; OVCAR-5* |
| PRO4302 | N/A | Ovarian Cancer | OVCAR-8:SK-OV-3 |
| PRO4302 | N/A | Leukemia | CCRF-CEM; HL-60(TB) |
| PRO4302 | N/A | Leukemia | K-562; SR; RPMI-8226 |
| PRO4302 | N/A | Renal Cancer | 786-0*; A498*; ACHN |
| PRO4302 | N/A | Renal Cancer | CAKI-1; RXF 393; SN 12C* |
| PRO4302 | N/A | Renal Cancer | TK-10*:UO-31* |
| PRO4302 | N/A | Melanoma | LOX IMVI; M14; SK-MEL-2* |
| PRO4302 | N/A | Melanoma | SK-MEL-28; SK-MEL-5 |
| PRO4302 | N/A | Melanoma | UACC-257*; UACC-62* |
| PRO4302 | N/A | Prostate Cancer | DU-1455; PC-3* |
| PRO4302 | N/A | CNS Cancer | SF-268; SF-295; S-539 |
| PRO4302 | N/A | CNS Cancer | SNB-19; SNB-75 |
| PRO4400 | N/A | NSCL | HOP-92; NCI-H226 |
| PRO4400 | N/A | NSCL | A549/ATCC; EKVX; HOP-62 |
| PRO4400 | N/A | NSCL | NCI-H23; NCI-H322 |
| PRO4400 | N/A | NSCL | NCI-H522 |
| PRO4400 | N/A | Colon Cancer | HCC-2998; HCT-15; HT29 |
| PRO4400 | N/A | Colon Cancer | KM12;SW620 |
| PRO4400 | N/A | Breast Cancer | HS 578T; BT-549; MCF7 |
| PRO4400 | N/A | Breast Cancer | MDA-MB-231/ATCC |
| PRO4400 | N/A | Breast Cancer | NCI/ADR-RES |
| PRO4400 | N/A | Ovarian Cancer | IGROVI*; OVCAR-3 |
| PRO4400 | N/A | Ovarian Cancer | OVCAR-4; OVCAR-5 |
| PRO4400 | N/A | Ovarian Cancer | OVCAR-8 |
| PRO4400 | N/A | Leukemia | CCRF-CEM; HL-60(TB); SR |
| PRO4400 | N/A | Leukemia | RPMI-8226; K-562; MOLT-4 |
| PRO4400 | N/A | Prostate Cancer | PC-3 |
| PRO4400 | N/A | Melanoma | MALME-3M; M14; UACC-257 |
| PRO4400 | N/A | Renal Cancer | A498; ACHN; CAK1-I |
| PRO4400 | N/A | Renal Cancer | RXF 393; SN 12C; TK-10 |
| PRO4400 | N/A | Renal Cancer | 786-0 |
| PRO4400 | N/A | CNS Cancer | SF-268; SNB-19: U251 |
| PRO5725 | N/A | NSCL | A549/ATCC; EKVX; HOP-92 |
| PRO5725 | N/A | NSCL | NCI-H23; NCI-H322M |
| PRO5725 | N/A | NSCL | NCI-H522; NCI-H226 |
| PRO5725 | N/A | NSCL | NCI-H460 |
| PRO5725 | N/A | Colon Cancer | COLO 205; HCC-2998 |
| PRO5725 | N/A | Colon Cancer | HCT-15; HT29; KM12; SW620 |
| PRO5725 | N/A | Breast Cancer | BT-549; HS 578T; MCF7 |
| PRO5725 | N/A | Breast Cancer | MDA-MB231/ATCC |
| PRO5725 | N/A | Breast Cancer | NCI/ADR-RES; T-47D |
| PRO5725 | N/A | Ovarian Cancer | OVCAR-3; OVCAR-4 |
| PRO5725 | N/A | Ovarian Cancer | OVCAR-8 |
| PRO5725 | N/A | Leukemia | CCRF-CEM; HL-60(TB)* |
| PRO5725 | N/A | Leukemia | K-562; MOLT-4; RPMI-8226 |
| PRO5725 | N/A | Leukemia | SR |
| PRO5725 | N/A | Renal Cancer | 786-0; ACHN; CAKI-1 |
| PRO5725 | N/A | Renal Cancer | RXF 393; SN 12C; TK-10 |
| PRO5725 | N/A | Renal Cancer | UO-31; A498 |
| PRO5725 | N/A | Melanoma | LOX IMVI; M 14; SK-MEL-28 |
| PRO5725 | N/A | Melanoma | UACC-257 |
| PRO5725 | N/A | Prostate Cancer | PC-3 |
| PRO5725 | N/A | CNS Cancer | SF-295; SNB-19; SNB-75 |
| PRO5725 | N/A | CNS Cancer | U251; SF-268 |
| PRO183 | 6 | Leukemia | HL-60(TB); SR; CCRF-CEM |
| PRO183 | 6 | Leukemia | RPMI-8226 |
| PRO183 | 6 | Colon Cancer | KM12; COLO 205; SW620 |
| PRO183 | 6 | Colon Cancer | HCT-15; HT-29 |
| PRO183 | 6 | Breast Cancer | MDA-N; MDA-MB-435 |
| PRO183 | 6 | NSCL | HOP-62; A549/ATCC; EKVX |
| PRO183 | 6 | NSCL | NCI-H23; NCI-H322M |
| PRO183 | 6 | Ovarian Cancer | IGROVI; OVCAR-8 |
| PRO183 | 6 | Melanoma | LOX IMVI; UACC-62 |
| PRO183 | 6 | Melanoma | UACC-257 |
| PRO183 | 6 | CNS Cancer | SF-295; S-539; U251 |
| PRO183 | 6 | Renal Cancer | SN 12C |
| PRO202 | 6 | NSCL | HOP-62; NCI-H322M |
| PRO202 | 6 | NSCL | NCI-H460; NCI-H522 |
| PR0202 | 6 | CNS Cancer | SF-268; SF-295; SNB-19 |
| PRO202 | 6 | CNS Cancer | U251 |
| PRO202 | 6 | Breast Cancer | MDA-N;MDA-MB 231/ATCC |
| PRO202 | 6 | Breast Cancer | MCF7 |
| PRO202 | 6 | Colon Cancer | KM12: HCC-2998 |
| PRO202 | 6 | Renal Cancer | SN 12C |
| PRO202 | 6 | Leukemia | CCRF-CEM; RPMI-8226 |
| PRO202 | 6 | Leukemia | MOLT-4 |
| PRO202 | 6 | Melanoma | LOX IMVI |
| PRO202 | 2 | NSCL | NCI-H322M |
| PRO202 | 2 | Colon Cancer | KM-12; HCC-2998* |
| PRO202 | 2 | CNS Cancer | SNB-19 |
| PRO202 | 2 | Leukemia | K-562; HL-60(TB) |
| PRO540 | 6 | Leukemia | CCRF-CEM; K-562; MOLT-4 |
| PRO540 | 6 | NSCL | EKVX; HOP-92; NCI-H23 |
| PRO540 | 6 | NSCL | NCI-H322M |
| PRO540 | 6 | Colon Cancer | COLO 205; HCT-116; HCT-15 |
| PRO540 | 6 | Colon Cancer | SW620 |
| PRO540 | 6 | CNS Cancer | SF-295; SNB-19; SNB-75 |
| PRO540 | 6 | CNS Cancer | U251 |
| PRO540 | 6 | Melanoma | M14 |
| PRO540 | 6 | Ovarian Cancer | IGROVI; OVCAR-4 |
| PRO540 | 6 | Ovarian Cancer | OVCAR-5 |
| PRO540 | 6 | Renal Cancer | RXF 393; SN 12C |
| PRO540 | 6 | Breast Cancer | MDA-N; BT-549 |
| PR0542 | 2 | Leukemia | SR |
| PRO542 | 2 | Breast Cancer | MDA-MB-231/ATCC |
| PRO542 | 2 | Breast Cancer | MDA-MB-435 |
| PRO542 | 2 | NSCL | EKVX; HOP-92: NCI-H226 |
| PRO542 | 2 | Colon Cancer | HCT-116; HCT-15 |
| PRO542 | 2 | CNS Cancer | SNB-75 |
| PRO542 | 2 | Ovarian Cancer | OVCAR-5 |
| PRO542 | 2 | Renal Cancer | RXF 393 |
| PRO861 | 6 | Leukemia | CCRF-CEM; HL-60(TB)* |
| PROB61 | 6 | Leukemia | MOLT-4*; SR |
| PRO861 | 6 | NSCL | HOP-92; NCI-H23; NCI-H522 |
| PRO861 | 6 | NSCL | NCI-H322M; EKVX |
| PRO861 | 6 | Colon Cancer | COLO 205; HCC-2998; HT29 |
| PRO861 | 6 | Colon Cancer | KM 12; SW620 |
| PRO861 | 6 | CNS Cancer | SF-268; U251 |
| PRO861 | 6 | Melanoma | LOX IMVI; SK-MEL-2 |
| PRO861 | 6 | Melanoma | SK-MEL-28; UACC-257 |
| PRO861 | 6 | Ovarian Cancer | IGROVI; OVCAR-3 |
| PRO861 | 6 | Ovarian Cancer | OVCAR-4; OVCAR-8 |
| PRO861 | 6 | Renal Cancer | SN 12C |
| PRO861 | 6 | Prostate Cancer | PC-3 |
| PRO861 | 6 | Breast Cancer | MCF7; MDA-MB-231/ATCC |
| PRO861 | 6 | Breast Cancer | MDA-MB-435; MDA-N |
| PRO861 | 6 | Breast Cancer | T-47D |
| PRO861 | 2 | Leukemia | CCRF-CEM; HL-60(TB); SR |
| PRO861 | 2 | NSCL | HOP-92 |
| PRO861 | 2 | Colon Cancer | COLO 205; HT29 |
| PRO861 | 2 | Melanoma | MALME-3M |
| PRO861 | 2 | Ovarian Cancer | OVCAR-5 |
| PRO861 | 2 | Breast Cancer | MCF7 |
| PRO1096 | 6 | Leukemia | CCRF-CEM; HL-60(TB)* |
| PRO1096 | 6 | Leukemia | K-562*; MOLT-4 |
| PRO1096 | 6 | Leukemia | RPMI-8226*; SR |
| PRO1096 | 6 | NSCL | A549/ATCC;EKVX; HOP-62* |
| PRO1096 | 6 | NSCL | NCI-H226*; NCI-H322M |
| PRO1096 | 6 | NSCL | NCI-H460*; NCI-H522 |
| PRO1096 | 6 | NSCL | HOP-92*; NCI-H23 |
| PRO1096 | 6 | Colon Cancer | COLO 205*; HCC-2998* |
| PRO1096 | 6 | Colon Cancer | HCT-15*; KM12; HT29 |
| PRO1096 | 6 | Colon Cancer | SW620; HCT-116* |
| PRO1096 | 6 | CNS Cancer | SF-295*; U251; S-539; SF-268 |
| PRO1096 | 6 | Melanoma | M14*; MALME-3M* |
| PRO1096 | 6 | Melanoma | SK-MEL-2; LOX IMVI |
| PRO1096 | 6 | Melanoma | SK-MEL-28; SK-MEL-5 |
| PRO1096 | 6 | Melanoma | UACC-257; UACC-62 |
| PRO1096 | 6 | Ovarian Cancer | OVCAR-3*; OVCAR-4* |
| PRO1096 | 6 | Ovarian Cancer | OVCAR-5; SK-OV-3 |
| PRO1096 | 6 | Ovarian Cancer | OVCAR-8 |
| PRO1096 | 6 | Renal Cancer | A498; ACHN*; CAKI-1 |
| PRO1096 | 6 | Renal Cancer | RXF 393*; SN 12C; TK-10* |
| PRO1096 | 6 | Renal Cancer | 786-0; UO-31 |
| PRO1096 | 6 | Prostate Cancer | DU-145; PC-3 |
| PRO1096 | 6 | Breast Cancer | MDA-MB-231/ATCC* |
| PRO1096 | 6 | Breast Cancer | MDA-MB-435; MDA-N |
| PRO1096 | 6 | Breast Cancer | BT-549; MCF7; HS 578T |
| PRO1096 | 2 | Leukemia | HL-60(TB)*; K-562* |
| PRO1096 | 2 | Leukemia | RPMI-8226*; SR; MOLT-4 |
| PRO1096 | 2 | Leukemia | CCRF-CEM |
| PRO1096 | 2 | NSCL | A549/ATCC;EKVX; HOP-92* |
| PRO1096 | 2 | NSCL | NCI-H226*; NCI-H322M* |
| PRO1096 | 2 | NSCL | NCI-H460*; NCI-H522 |
| PRO1096 | 2 | NSCL | HOP-62* |
| PRO1096 | 2 | Colon Cancer | COLO 205*; HCC-2998* |
| PRO1096 | 2 | Colon Cancer | HCf-15*;HCT-116*;KM12 |
| PRO1096 | 2 | Colon Cancer | HT29; SW620 |
| PRO1096 | 2 | CNS Cancer | SF-295*; S-539*; U251 |
| PRO1096 | 2 | Melanoma | M14*; MALME-3M* |
| PRO1096 | 2 | Melanoma | SK-MEL-28*; UACC-62 |
| PRO1096 | 2 | Melanoma | SK-MEL-2; LOX IMVI* |
| PRO1096 | 2 | Melanoma | SK-MEL-5 |
| PRO1096 | 2 | Ovarian Cancer | OYCAR-3*; OYCAR-4* |
| PRO1096 | 2 | Ovarian Cancer | OVCAR-5*; SK-OV-3* |
| PRO1096 | 2 | Renal Cancer | A498*; ACHN*; CAKI-1 |
| PRO1096 | 2 | Renal Cancer | RXF 393*; SN 12C; TK-10* |
| PRO1096 | 2 | Renal Cancer | UO-31 |
| PRO1096 | 2 | Prostate Cancer | DU-145; PC-3* |
| PRO1096 | 2 | Breast Cancer | MCF7; MDA-MB-231/ATCC* |
| PRO1096 | 2 | Breast Cancer | MDA-MB-435: MDA-N |
| PRO1096 | 2 | Breast Cancer | BT-549; HS 578T |
| PRO1096 | N/A | NSCL | A549/ATCC*; EKVX |
| PRO1096 | N/A | NSCL | HOP-62*; HOP-92*; NCI-H23 |
| PRO1096 | N/A | NSCL | NCI-H322M*; NCI-H460 |
| PRO1096 | N/A | NSCL | NCI-H522 |
| PRO1096 | N/A | Colon Cancer | COLO 205; HCT-116* |
| PRO1096 | N/A | Colon Cancer | HCT-15; HT29*; KM12* |
| PRO1096 | N/A | Colon Cancer | SW620; HCC-2998* |
| PRO1096 | N/A | Breast Cancer | HS 578T; MCF7 |
| PRO1096 | N/A | Breast Cancer | MDA-MB-231/ATCC* |
| PRO1096 | N/A | Breast Cancer | MDA-MB-435*; MDA-N* |
| PRO1096 | N/A | Breast Cancer | T-47D |
| PRO1096 | N/A | Ovarian Cancer | OVCAR-3*; OVCAR-4* |
| PRO1096 | N/A | Ovarian Cancer | OVCAR-5*; SK-OV-3 |
| PRO1096 | N/A | Leukemia | HL-60(TB); MOLT-4 |
| PRO1096 | N/A | Leukemia | RPMI-8226 |
| PRO1096 | N/A | Renal Cancer | A498; ACHN*; RXF 393* |
| PRO1096 | N/A | Renal Cancer | SN 12C*; TK-10; UO-31 |
| PRO1096 | N/A | Renal Cancer | CAKI-1 |
| PRO1096 | N/A | Melanoma | M14*; MALME-3M |
| PRO1096 | N/A | Melanoma | SK-MEL-28; SK-MEL-2 |
| PRO1096 | N/A | Melanoma | UACC-257; UACC-62 |
| PRO1096 | N/A | Melanoma | LOX IMVI |
| PRO1096 | N/A | Prostate Cancer | DU-145; PC-3* |
| PRO1096 | N/A | CNS Cancer | SF-268; SF-295; S-539 |
| PRO1096 | N/A | CNS Cancer | SNB-75*; U251 |
| * = CYTOTOXIC | | | |
| PRO3562 | N/A | Colon Cancer | HCC-2998 |
| PR03562 | N/A | NSCL | HOP-62 |
| PRO3562 | N/A | Ovarian Cancer | IGROVI; OVCAR-3 |
| PRO3562 | N/A | Ovarian Cancer | OVCAR-8 |
| PRO3562 | N/A | Leukemia | MOLT-4 |
| PRO3562 | N/A | CNS Cancer | SNB-19 |

### Deposit of Material

The following materials have been deposited with the American Type Culture Collection, 10801 University Blvd., Manassas, VA 20110-2209, USA (ATCC):

| Material | ATCC Dep. No. | Deposit Date |
|---|---|---|
| DNA34387-1138 | 209260 | September 16, 1997 |
| DNA44194-1317 | 209808 | April 28, 1998 |
| DNA48333-1321 | 209701 | March 26, 1998 |
| DNA44189-1322 | 209699 | March 26, 1998 |
| DNA48320-1433 | 209904 | May 27, 1998 |
| DNA57700-1408 | 203583 | January 12, 1999 |
| DNA57708-1411 | 203021 | June 23, 1998 |
| DNA57694-1374 | 209950 | June 9, 1998 |
| DNA59777-1480 | 203111 | August 11, 1998 |
| DNA60292-1506 | 203540 | December 15, 1998 |
| DNA65351-1366-1 | 209856 | May 12, 1998 |
| DNA60764-1533 | 203452 | November 10, 1998 |
| DNA64903-1553 | 203223 | September 15, 1998 |
| DNA64966-1575 | 203575 | January 12, 1999 |
| DNA66667 | 203267 | September 22, 1998 |
| DNA67004-1614 | 203115 | August 11, 1998 |
| DNA68874-1622 | 203277 | September 22, 1998 |
| DNA73736-1657 | 203466 | November 17, 1998 |
| DNA82364-2538 | 203603 | January 20, 1999 |
| DNA92218-2554 | 203834 | March 9, 1999 |
| DNA87974-2609 | 203963 | April 27, 1999 |
| DNA92265-2669 | PTA-256 | June 22, 1999 |

These deposits were made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposits will be made available by ATCC under the terms of the Budapest Treaty, and subject to an agreement between Genentech, Inc., and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 U.S.C. § 122 and the Commissioner's rules pursuant thereto (including 37 CFR § 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

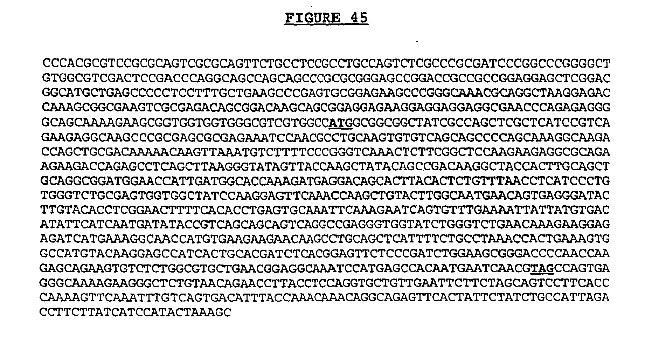

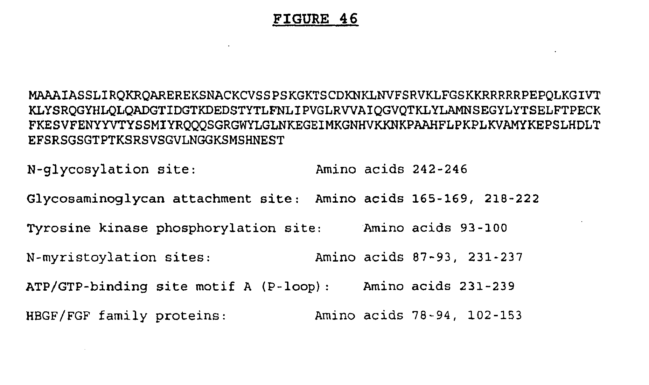

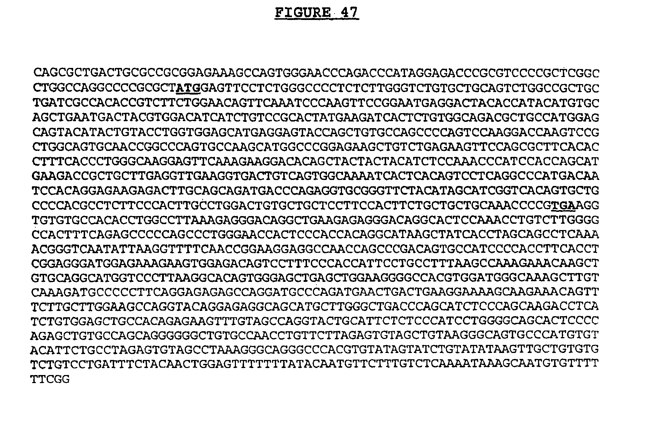

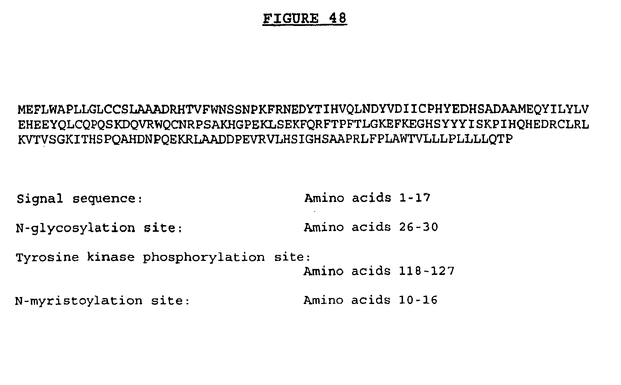

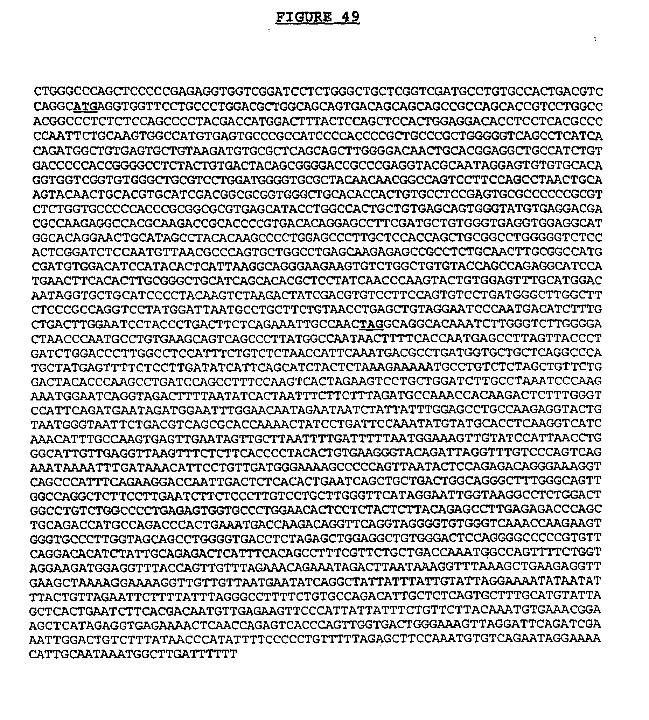

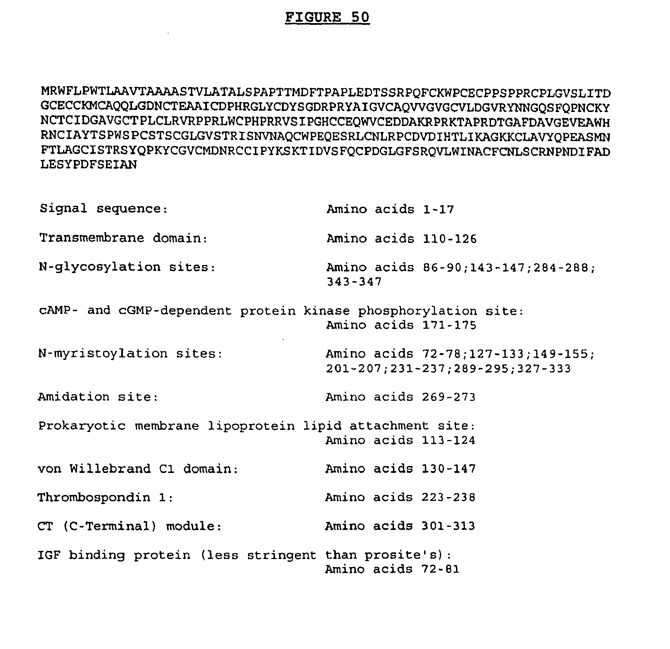

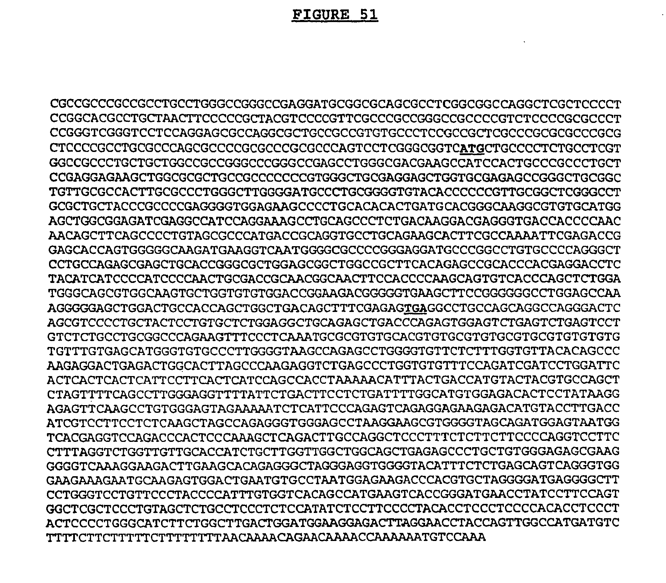

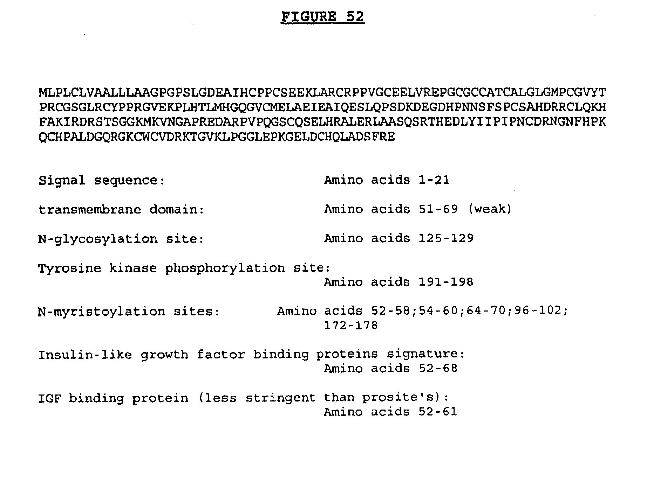

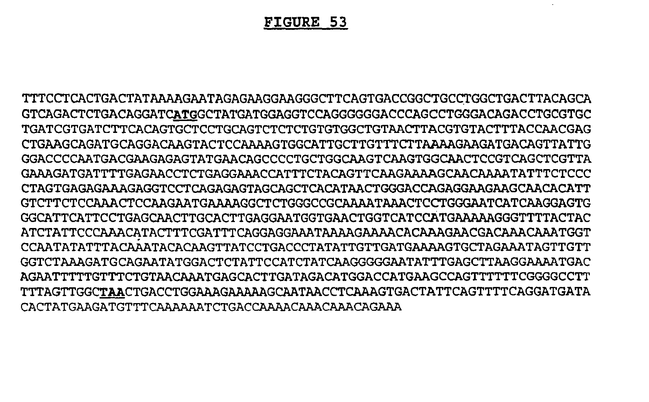

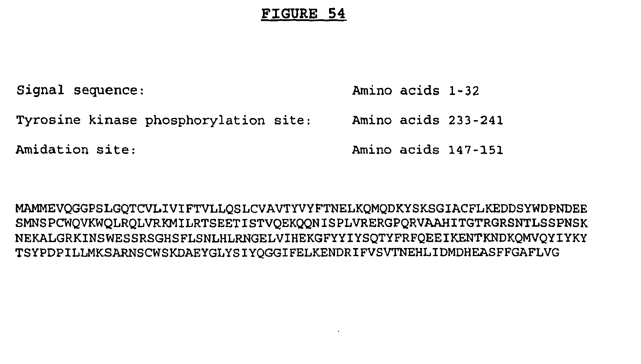

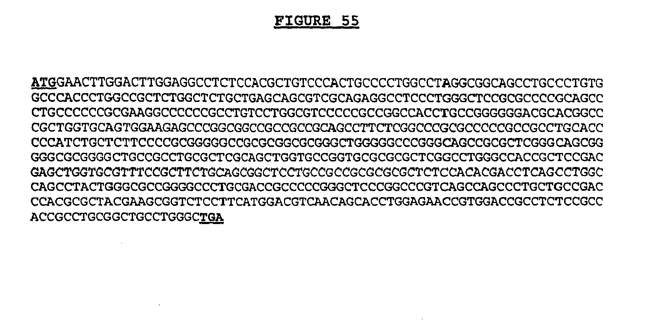

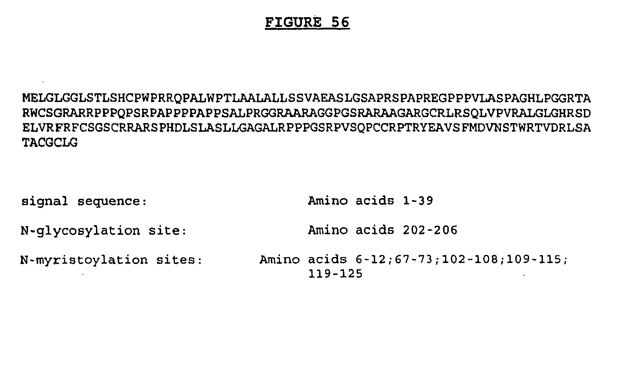

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims. The following numbered paragraphs (paras.) contain further statements of various aspects of the present invention:-
1. A composition of matter useful for the inhibition of neoplastic cell growth, said composition comprising an effective amount of a PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PR03438, PR04302, PRO4400, PRO5725, PRO183, PRO202, PRO542, PRO861, PRO1096 or PRO3562 polypeptide, or an agonist thereof, in admixture with a pharmaceutically acceptable carrier.
2. The composition of matter of Para. 1 comprising a growth inhibitory amount of a PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO4400, PRO5725, PRO183, PRO202, PRO542, PRO861, PRO1096 or PRO3562 polypeptide, or an agonist thereof.
3. The composition of matter of Para. 1 comprising a cytotoxic amount of a PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO4400, PRO5725, PRO183, PRO202, PRO542, PRO861, PRO1096 or PRO3562 polypeptide, or an agonist thereof.
4. The composition of matter of Para. 1 additionally comprising a further growth inhibitory agent, cytotoxic agent or chemotherapeutic agent.
5. A composition of matter useful for the treatment of a tumor in a mammal, said composition comprising a therapeutically effective amount of a PRO240, PRO381, PRO534, PRO540, PRO698, PRO982, PRO1005 PRO1007 PRO113 PRO115 PRO1199, PRO1265, PRO1286, PRO1313 PRO1338 PRO1375 PRO1410 PRO1488 PRO3438, PRO4302, PRO4400, PRO5725, PRO183 PRO202, PRO542, PRO861, PRO1096 or PRO3562 polypeptide, or an agonist thereof.
6. The composition of matter of Para. 5, wherein said tumor is a cancer.
7. The composition of matter of Para. 6, wherein the cancer is selected from the group consisting of breast cancer, ovarian cancer, renal cancer, colorectal cancer, uterine cancer, prostate cancer, lung cancer, bladder cancer, central nervous system cancer, melanoma and leukemia.
8. A method for inhibiting the growth of a tumor cell comprising exposing said tumor cell to an effective amount of a PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO4400, PRO5725, PRO183, PRO202, PRO542, PRO861, PRO1096 or PRO3562 polypeptide, or an agonist thereof.
9. The method of Para. 8, wherein said agonist is an anti-PR0240, anti-PR0381, anti-PRO534, anti-PR0540, anti-PR0698, anti-PR0982, anti-PRO1005, anti-PRO1007, anti-PRO1131, anti-PRO1157, anti-PRO1199, anti-PRO1265, anti-PRO1286, anti-PRO1313, anti-PRO1338, anti-PRO1375, anti-PRO1410, anti-PR01488, anti-PR03438, anti-PRO4302, anti-PR04400, anti-PR05725, anti-PRO183, anti-PR0202, anti-PR0542, anti-PRO861, anti-PRO1096 or anti-PR03562 agonist antibody.
10. The method of Para. 8, wherein said agonist is a small molecule mimicking the biological activity of a PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO4400, PRO5725, PRO183, PRO202, PRO542, PRO861, PRO1096, or PRO3562 polypeptide.
11. The method of Para. 8, wherein said step of exposing occurs *in vitro.*
12. The method of Para. 8, wherein said step of exposing occurs *in vivo.*
13. An article of manufacture comprising:
   a container; and
   a composition comprising an active agent contained within the container; wherein said active agent in the composition is a PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO4400, PRO5725, PRO183 PRO202, PRO542, PRO861 PRO109 or PRO3562 polypeptide, or an agonist thereof.
14. The article of manufacture of Para. 13, further comprising a label affixed to said container, or a package insert included in said container, referring to the use of said composition for the inhibition of neoplastic cell growth.
15. The article of manufacture of Para. 13, wherein said agonist is an anti-PR0240, anti-PRO381, anti-PRO534, anti-PR0540, anti-PR0698, anti-PR0982, anti-PRO1005, anti-PRO1007 anti-PRO1131, anti-PRO1157, anti-PRO1199, anti-PRO1265, anti-PRO1286, anti-PRO1313, anti-PRO1338, anti-PRO1375, anti-PRO1410, anti-PRO1488, anti-PRO3438, anti-PR04302, anti-PR04400, anti-PR05725, anti-PRO183, anti-PR0202, anti-PRO542, anti-PR0861, anti-PRO1096 or anti-PR03562 agonist antibody.
16. The article of manufacture of Para. 13, wherein said agonist is a small molecule mimicking the biological activity of a PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO4400, PRO5725, PRO183, PRO202, PRO542, PRO861, PRO109 or PRO3562 polypeptide.
17. The article of manufacture of Para. 13, wherein said active agent is present in an amount that is effective for the treatment of tumor in a mammal.
18. The article of manufacture of Para. 13, wherein said composition additionally comprises a further growth inhibitory agent, cytotoxic agent or chemotherapeutic agent.
19. Isolated nucleic acid having at least 80% nucleic acid sequence identity to a nucleotide sequence that encodes an amino acid sequence selected from the group consisting of the amino acid sequence shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56).
20. Isolated nucleic acid having at least 80% nucleic acid sequence identity to a nucleotide sequence selected from the group consisting of the nucleotide sequence shown in Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 41 (SEQ ID NO:41), Figure 43 (SEQ ID NO:43), Figure 45 (SEQ ID NO:45), Figure 47 (SEQ ID NO:47), Figure 49 (SEQ ID NO:49), Figure 51 (SEQ ID NO:51), Figure 53 (SEQ ID NO:53), and Figure 55 (SEQ ID NO:55).
21. Isolated nucleic acid having at least 80% nucleic acid sequence identity to a nucleotide sequence selected from the group consisting of the full-length coding sequence of the nucleotide sequence shown in Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 41 (SEQ ID NO:41), Figure 43 (SEQ ID NO:43), Figure 45 (SEQ ID NO:45), Figure 47 (SEQ ID NO:47), Figure 49 (SEQ ID NO:49), Figure 51 (SEQ ID NO:51), Figure 53 (SEQ ID NO:53), and Figure 55 (SEQ ID NO:55).
22. Isolated nucleic acid having at least 80% nucleic acid sequence identity to the full-length coding sequence of the DNA deposited under ATCC accession number 209260, 209808, 209701, 209699, 209904, 203583, 203021, 209950, 203111, 203540, 209856, 203452, 203223, 203575, 203267, 203115, 203277, 203466, 203603, 203834, 203963 or PTA-256.
23. A vector comprising the nucleic acid of any one of Paras. 19 to 22.
24. The vector of Para. 23 operably linked to control sequences recognized by a host cell transformed with the vector.
25. A host cell comprising the vector of Para. 23.
26. The host cell of Para. 25, wherein said cell is a CHO cell.
27. The host cell of Para. 25, wherein said cell is an *E*. *coli.*
28. The host cell of Para. 25, wherein said cell is a yeast cell.
29. The host cell of Para. 25, wherein said cell is a Baculovirus-infected insect cell.
30. A process for producing a PRO240, PRO381, PRO534, PRO540, PRO698, PRO982, PRO1005 PRO1007 PRO1131, PRO1157, PRO1199, PRO1265, PRO1286, PRO1313, PRO1338, PRO1375, PR0141 0, PRO1488, PRO3438, PRO4302, PRO4400, PRO5725, PRO183 PRO202, PRO542, PRO861, PRO109 or PRO3562 polypeptide comprising culturing the host cell of Para. 25 under conditions suitable for expression of said polypeptide and recovering said polypeptide from the cell culture.
31. An isolated polypeptide having at least 80% amino acid sequence identity to an amino acid sequence selected from the group consisting of the amino acid sequence shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56).
32. An isolated polypeptide scoring at least 80% positives when compared to an amino acid sequence selected from the group consisting of the amino acid sequence shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56).
33. An isolated polypeptide having at least 80% amino acid sequence identity to an amino acid sequence encoded by the full-length coding sequence of the DNA deposited under ATCC accession number 209260, 209808, 209701, 209699, 209904, 203583, 203021, 209950, 203111, 203540, 209856, 203452, 203223, 203575, 203267, 203115, 203277, 203466, 203603, 203834, 203963 or PTA-256.
34. A chimeric molecule comprising a polypeptide according to any one of Paras. 31 to 33 fused to a heterologous amino acid sequence.
35. The chimeric molecule of Para. 34, wherein said heterologous amino acid sequence is an epitope tag sequence.
36. The chimeric molecule of Para. 34, wherein said heterologous amino acid sequence is a Fc region of an immunoglobulin.
37. An antibody which specifically binds to a polypeptide according to any one of Paras. 31 to 33.
38. The antibody of Para. 37, wherein said antibody is a monoclonal antibody, a humanized antibody or a single-chain antibody.
39. Isolated nucleic acid having at least 80% nucleic acid sequence identity to:
   (a) a nucleotide sequence encoding the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56), lacking its associated signal peptide:
   (b) a nucleotide sequence encoding an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56), with its associated signal peptide; or
   (c) a nucleotide sequence encoding an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56), lacking its associated signal peptide.
40. An isolated polypeptide having at least 80% amino acid sequence identity to:
   (a) the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56), lacking its associated signal peptide;
   (b) an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56), with its associated signal peptide; or
   (c) an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56), lacking its associated signal peptide.

### NOTICE

The content of the present application is not to be construed as limited to that of the claims as filed, but includes all subject matter disclosed in the specification.

The applicant maintains the right to file amendments and/or further divisional applications from this one, directed to any subject matter that is disclosed in the present specification, irrespective of whether that subject matter is in the claims of this application as filed, and irrespective of the fate of any European patent application from which this application is divided.

## Claims

1. A composition of matter useful for the inhibition of neoplastic cell growth, said composition comprising an effective amount of a PRO1096, PRO202, PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO5725, PRO183, PRO542, PRO861 or PRO3562 polypeptide, or an agonist thereof, in admixture with a pharmaceutically acceptable carrier.

2. The composition of matter of Claim 1 comprising a growth inhibitory amount of a PRO1096, PRO202, PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO5725, PRO183, PRO542, PRO861 or PRO3562 polypeptide, or an agonist thereof.

3. The composition of matter of Claim 1 comprising a cytotoxic amount of a PRO1096, PRO202, PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO5725, PRO183, PRO542, PRO861 or PRO3562 polypeptide, or an agonist thereof.

4. The composition of matter of Claim 1 additionally comprising a further growth inhibitory agent, cytotoxic agent or chemotherapeutic agent.

5. A composition of matter useful for the treatment of a tumor in a mammal, said composition comprising a therapeutically effective amount of a PRO1096 PRO202, PRO240, PRO381, PRO534, PRO540, PRO698, PRO982, PRO1005 PRO1007 PRO1131, PRO1157, PRO1199, PRO1265 PRO1286 PRO1313, PRO1338 PRO1375 PRO1410, PRO1488, PRO3438, PRO4302, PRO5725, PRO183, PRO542, PRO861 or PRO3562 polypeptide, or an agonist thereof.

6. The composition of matter of Claim 5, wherein said tumor is a cancer.

7. The composition of matter of Claim 6, wherein the cancer is selected from the group consisting of breast cancer, ovarian cancer, renal cancer, colorectal cancer, uterine cancer, prostate cancer, lung cancer, bladder cancer, central nervous system cancer, melanoma and leukemia.

8. A method for inhibiting the growth of a tumor cell comprising exposing said tumor cell to an effective amount of a PRO1096, PRO202, PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO5725, PRO183, PRO542, PRO861 or PRO3562 polypeptide, or an agonist thereof.

9. The method of Claim 8, wherein said agonist is an anti-PRO1096, anti-PR0202, anti-PRO240, anti-PR0381, anti-PR0534, anti-PR0540, anti-PRO698, anti-PRO982, anti-PRO1005, anti-PRO1007, anti-PRO1131, anti-PRO1157, anti-PRO1199, anti-PRO1265, anti-PRO1286, anti-PRO1313, anti-PRO1338, anti-PRO1375, anti-PRO1410, anti-PRO1488, anti-PR03438, anti-PR04302, anti-PR05725, anti-PRO183, anti-PR0542, anti-PRO861 or anti-PR03562 agonist antibody.

10. The method of Claim 8, wherein said agonist is a small molecule mimicking the biological activity of a PR01096, PRO202, PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO5725, PRO183, PRO542, PRO861 or PRO3562 polypeptide.

11. The method of Claim 8, wherein said step of exposing occurs *in vitro.*

12. The method of Claim 8, wherein said step of exposing occurs *in vivo.*

13. An article of manufacture comprising:
a container; and
a composition comprising an active agent contained within the container; wherein said active agent in the composition is a PRO1096 PRO202, PRO240, PRO381, PRO534, PRO540, PRO698, PRO982, PRO1005 PRO1007, PRO1131 PRO1157 PRO1199 PRO1265 PRO1286 PRO1313 PRO1338 PRO1375 PRO1410 PRO1488 PRO3438, PRO4302, PRO5725, PRO183 PRO542, PRO86 or PRO3562 polypeptide, or an agonist thereof.

14. The article of manufacture of Claim 13, further comprising a label affixed to said container, or a package insert included in said container, referring to the use of said composition for the inhibition of neoplastic cell growth.

15. The article of manufacture of Claim 13, wherein said agonist is an anti-PRO1096, anti-PRO202, anti-PR0240, anti-PR0381, anti-PR0534, anti-PR0540, anti-PR0698, anti-PRO982, anti-PRO1005, anti-PRO1007, anti-PRO1131, anti-PRO1157, anti-PRO1199, anti-PRO1265, anti-PRO1286, anti-PRO1313, anti-PRO1338, anti-PRO1375, anti-PRO1410, anti-PRO1488, anti-PR03438, anti-PR04302, anti-PR05725, anti-PRO183, anti-PR0542, anti-PR0861 or anti-PR03562 agonist antibody.

16. The article of manufacture of Claim 13, wherein said agonist is a small molecule mimicking the biological activity of a PRO1096, PRO202, PRO240, PRO381, PRO534, PRO540, PRO698, PRO982, PRO1005, PRO1007, PRO1131, PRO1157, PRO1199, PRO1265, PRO1286, PRO1313, PRO1338, PRO1375 PRO1410, PRO1488 PRO3438, PRO4302, PRO5725, PRO183 PRO542, PRO861 or PRO3562 polypeptide.

17. The article of manufacture of Claim 13, wherein said active agent is present in an amount that is effective for the treatment of tumor in a mammal.

18. The article of manufacture of Claim 13, wherein said composition additionally comprises a further growth inhibitory agent, cytotoxic agent or chemotherapeutic agent.

19. Isolated nucleic acid having at least 80% nucleic acid sequence identity to a nucleotide sequence that encodes an amino acid sequence selected from the group consisting of the amino acid sequence shown in Figure 54 (SEQ ID NO:54), Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52) and Figure 56 (SEQ ID NO:56).

20. Isolated nucleic acid having at least 80% nucleic acid sequence identity to a nucleotide sequence selected from the group consisting of the nucleotide sequence shown in Figure 53 (SEQ ID NO:53), Figure 47 (SEQ ID NO:47), Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 43 (SEQ ID NO:43), Figure 45 (SEQ ID NO:45), Figure 49 (SEQ ID NO:49), Figure 51 (SEQ ID NO:51) and Figure 55 (SEQ ID NO:55).

21. Isolated nucleic acid having at least 80% nucleic acid sequence identity to a nucleotide sequence selected from the group consisting of the full-length coding sequence of the nucleotide sequence shown in Figure 53 (SEQ ID NO:53), Figure 47 (SEQ ID NO:47), Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 43 (SEQ ID NO:43), Figure 45 (SEQ ID NO:45), Figure 49 (SEQ ID NO:49), Figure 51 (SEQ ID NO:51) and Figure 55 (SEQ ID NO:55).

22. Isolated nucleic acid having at least 80% nucleic acid sequence identity to the full-length coding sequence of the DNA deposited under ATCC accession number 209260, 209808, 209701, 209699, 209904, 203583, 203021, 209950, 203111, 203540, 209856, 203452, 203223, 203575, 203267, 203115, 203277, 203466, 203603, 203834, or PTA-256.

23. A vector comprising the nucleic acid of any one of Claims 19 to 22.

24. The vector of Claim 23 operably linked to control sequences recognized by a host cell transformed with the vector.

25. A host cell comprising the vector of Claim 23.

26. The host cell of Claim 25, wherein said cell is a CHO cell.

27. The host cell of Claim 25, wherein said cell is an *E*. *coli.*

28. The host cell of Claim 25, wherein said cell is a yeast cell.

29. The host cell of Claim 25, wherein said cell is a Baculovirus-infected insect cell.

30. A process for producing a PRO1096,PRO202, PRO240, PRO381, PRO534, PRO540, PRO698, PRO982, PRO1005 PRO1007, PRO1131, PRO1157, PRO1199, PRO1265, PRO1286, PRO1313, PRO1338, PRO1375, PRO1410, PRO1488, PRO3438, PRO4302, PRO5725, PRO183, PRO542, PRO861 or PRO3562 polypeptide comprising culturing the host cell of Claim 25 under conditions suitable for expression of said polypeptide and recovering said polypeptide from the cell culture.

31. An isolated polypeptide having at least 80% amino acid sequence identity to an amino acid sequence selected from the group consisting of the amino acid sequence shown in Figure 54 (SEQ ID NO:54), Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46) Figure 50 (SEQ ID NO:50), Figure 52 (SEQ ID NO:52) and Figure 56 (SEQ ID NO:56).

32. An isolated polypeptide scoring at least 80% positives when compared to an amino acid sequence selected from the group consisting of the amino acid sequence shown in Figure 54 (SEQ ID NO:54), Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52) and Figure 56 (SEQ ID NO:56).

33. An isolated polypeptide having at least 80% amino acid sequence identity to an amino acid sequence encoded by the full-length coding sequence of the DNA deposited under ATCC accession number 209260, 209808, 209701, 209699, 209904, 203583, 203021, 209950, 203111, 203540, 209856, 203452, 203223, 203575, 203267, 203115, 203277, 203466, 203603, 203834, or PTA-256.

34. A chimeric molecule comprising a polypeptide according to any one of Claims 31 to 33 fused to a heterologous amino acid sequence.

35. The chimeric molecule of Claim 34, wherein said heterologous amino acid sequence is an epitope tag sequence.

36. The chimeric molecule of Claim 34, wherein said heterologous amino acid sequence is a Fc region of an immunoglobulin.

37. An antibody which specifically binds to a polypeptide according to any one of Claims 31 to 33.

38. The antibody of Claim 37, wherein said antibody is a monoclonal antibody, a humanized antibody or a single-chain antibody.

39. Isolated nucleic acid having at least 80% nucleic acid sequence identity to:
(a) a nucleotide sequence encoding the polypeptide shown in Figure 54 (SEQ ID NO:54), Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52) and Figure 56 (SEQ ID NO:56), lacking its associated signal peptide:
(b) a nucleotide sequence encoding an extracellular domain of the polypeptide shown in Figure 54 (SEQ ID NO:54), Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52) and Figure 56 (SEQ ID NO:56), with its associated signal peptide; or
(c) a nucleotide sequence encoding an extracellular domain of the polypeptide shown in Figure 54 (SEQ ID NO:54), Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52) and Figure 56 (SEQ ID NO:56), lacking its associated signal peptide.

40. An isolated polypeptide having at least 80% amino acid sequence identity to:
(a) the polypeptide shown in Figure 54 (SEQ ID NO:54), Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52) and Figure 56 (SEQ ID NO:56), lacking its associated signal peptide;
(b) an extracellular domain of the polypeptide shown in Figure 54 (SEQ ID NO:54), Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52) and Figure 56 (SEQ ID NO:56), with its associated signal peptide; or
(c) an extracellular domain of the polypeptide shown in Figure 54 (SEQ ID NO:54), Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52) and Figure 56 (SEQ ID NO:56), lacking its associated signal peptide.
